# EUROPEAN PATENT APPLICATION

(11) **EP 4 201 936 A1**
(43) Date of publication of application: **28.06.2023**
(21) Application number: 21857735.1
(22) Date of filing: 19.08.2021
(51) Int. Cl.: C07D 471/04, A61K 31/437, A61P 35/00

(54) **HETEROAROMATIC RING COMPOUND AS RET KINASE INHIBITOR, AND PREPARATION AND USE THEREOF**

(30) Priority: 20.08.2020 CN 202010843823; 09.11.2020 CN 202011236522; 21.04.2021 CN 202110431905
(71) Applicant: Jiangsu Chia Tai Fenghai Pharmaceutical Co., Ltd., Yancheng, Jiangsu 224100 (CN)
(72) Inventor: ZHU, Yongqiang, Yancheng, Jiangsu 224100 (CN); LIU, Zhaogang, Yancheng, Jiangsu 224100 (CN); FENG, Chao, Yancheng, Jiangsu 224100 (CN); CHEN, Hao, Yancheng, Jiangsu 224100 (CN); XU, Kaikai, Yancheng, Jiangsu 224100 (CN); WANG, Jia, Yancheng, Jiangsu 224100 (CN); SHI, Jingmiao, Yancheng, Jiangsu 224100 (CN)
(74) Representative: Engelhard, Markus
(86) International application number: PCT/CN2021/113514
(87) International publication number: WO 2022/037643

(57) **Abstract**

Disclosed are a heteroaromatic ring compound as a RET kinase inhibitor, and the preparation and the use thereof, wherein the compound is a compound having the structure of formula (I) or a pharmaceutically acceptable salt thereof. The compound or a salt thereof can be used for treating or preventing diseases or conditions by targeting receptors of fused and mutated forms of the RET gene, can effectively inhibit the growth of multiple tumor cells, produces an inhibitory effect on a RET gene fusion mutation and other proteases, and can be used for preparing an anti-tumor drug.

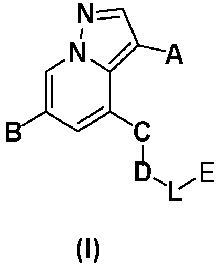

## Description

### TECHNICAL FIELD

The present invention relates to a series of derivatives of heteroaromatic ring structures and the use as RET kinase inhibitors thereof. Specifically, it relates to compound represented by formula (I) or a pharmaceutically acceptable salt thereof.

### BACKGROUND ART

In 1985, people transfected NIH3T3 cells with the high-molecular-weight DNA of human T cell lymphoma and found that RET was a new transforming gene. The gene was activated by DNA rearrangement, in which two unlinked segments of human DNA recombined to produce a new transcription unit. Afterward, RET was localized to chromosome 10q11.2 by research, where it encodes a receptor tyrosine kinase. RET is a single-pass transmembrane protein with a typical intracellular tyrosine kinase domain. Although the "classical" activation of receptor tyrosine kinases (RTKs) is due to ligand-receptor interactions, the activation of RET requires interactions between its ligands (glial cell line-derived neurotrophic factor family ligands, GFLs) and the co-receptor (GFL family receptor-α). The binding of GFL-GFRα complexes to the extracellular domain of Ret causes the phosphorylation of the intracellular tyrosine kinase domain and thereby the activation of several pathways including MAPK, PI3K, JAK-STAT, PKA and PKC.

RET is related to the development of the kidney and the gastrointestinal nervous system under normal physiological conditions; however, mutations in the RET gene lead to ligand-independent, constitutive abnormal activation of the RET kinase and thus to tumorigenesis. There are two major mechanisms for the RET kinase activation: 1. point mutations in the RET gene; and 2. RET gene rearrangement. Missense mutations in RET may occur at extracellular Cys residues, causing abnormal kinase activation. Mutations may also occur in the intracellular kinase activity domain and would promote the ligand-independent RET kinase activation. Point mutations in RET are very common in medullary thyroid carcinoma (MTC): they occur in approximately 50% of sporadic MTC and almost all familial MTC. The RET gene is rearranged into a new fusion gene by breaking itself and fusing with other genes, making the RET tyrosine kinase activation independent of the ligand's regulation, leading to further autophosphorylation. Therefore, the signal transduction function is enhanced and the kinase activation is promoted, which causes tumorigenesis. RET fusions are found in approximately 20% of papillary thyroid cancer (PTC), 1-2% of non-small cell lung cancer (NSCLC) and other cancers such as colon cancer and mammary cancer. The above results indicate that dysregulation of the RET signaling pathway is a key driver of many neoplastic diseases.

At present, in terms of activity and tolerance, various multi-kinase inhibitors have inhibitory activity against RET, but no specific inhibitory activity, and the incidence of grade 3-4 toxicity is high in patients exposed to RET TKI for a long period of time due to the inhibitory effect on the VEGFR kinase. Therefore, there is a clinical need to develop RET specific inhibitors with strong activity and high selectivity, which are expected to be new treatments for various cancers such as thyroid cancer and non-small cell lung cancer.

### SUMMARY

The present invention is intended to provide a class of heteroaromatic ring compounds as RET kinase inhibitors.

The purpose of the present invention can be achieved by providing:
A compound of formula (I) or a pharmaceutically acceptable salt, ester, stereoisomer, solvate, oxide or prodrug thereof, 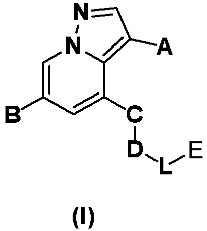 wherein,
A is selected from H, -CN, halogen, -C=ONH₂, -C=C-CN and -C≡CH;
B is selected from the following groups unsubstituted or substituted with one or more identical or different substituents: C1-C6 alkyl, C1-C6 alkylamino, C2-C6 alkynyl, C2-C6 alkenyl, HetAr¹ and HetCyc¹; the substituents are independently selected from halogen, hydroxy, -CN, =O (carbonyl), C1-C6 alkyl, deuterated C1-C6 alkyl, C1-C6 alkoxy, hydroxy C1-C6 alkyl, halo C1-C6 alkyl, cyano C1-C6 alkyl, (C1-C6 alkoxy) C1-C6 alkyl, C3-C6 cycloalkyl and (C1-C6 alkoxy SO₂) C1-C6 alkyl;
HetAr¹ is a 5- to 6-membered heteroaromatic ring having 1-3 heteroatoms independently selected from N, S and O;
HetCyc¹ is a 4- to 8-membered heterocycle having 1-3 heteroatoms selected from N and O, an 8- to 10-membered spiro ring having 1-3 heteroatoms selected from N and O or a 7- to 11-membered fused heterocycle having 1-3 heteroatoms selected from N and O;
C is a 5- to 6-membered heteroaromatic ring having 1-3 heteroatoms independently selected from N, S and O, wherein the heteroaromatic ring is unsubstituted or is optionally substituted with one or more identical or different substituents independently selected from halogen, hydroxy, -CN, nitro, C1-C3 alkyl and halo C1-C3 alkyl;
D is a C1-C6 alkyl having 1-3 heteroatoms selected from N and O, a 4- to 8-membered heterocycle having 1-3 heteroatoms selected from N and O, a 7- to 8-membered bridged ring having 1-3 heteroatoms selected from N and O, a 7- to 11-membered spiro ring having 1-3 heteroatoms selected from N and O, a 7- to 10-membered fused heterocycle having 1-3 heteroatoms selected from N and O, 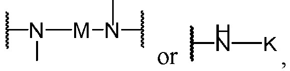 wherein M is selected from C1-C3 alkyl and C3-C8 cycloalkyl; K is a 4- to 8-membered heterocycle having 1-3 heteroatoms selected from N and O;
L is 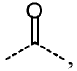 -C(=O)-C1-C3 alkyl or C1-C3 alkyl;
E is HetAr² unsubstituted or substituted with one or more identical or different substituents; the substituents are independently selected from halogen, C1-C6 alkyl, deuterated C1-C6 alkyl, C1-C6 alkoxy, deuterated C1-C6 alkoxy, hydroxy C1-C6 alkyl, C1-C6 haloalkyl, cyano C1-C6 alkyl, (C1-C6 alkoxy) C1-C6 alkyl, C3-C6 cycloalkyl and (C1-C6 alkoxy SO₂) C1-C6 alkyl;
HetAr² is a 5- to 6-membered heteroaromatic ring having 1-3 ring heteroatoms independently selected from N, S and O.

In some embodiments, in the compound of formula (I) or the pharmaceutically acceptable salt, ester, stereoisomer, solvate, oxide or prodrug thereof, A is selected from -CN, -C=C-CN and -C≡CH; in one specific embodiment, A is -CN.

In some embodiments, in the compound of formula (I) or the pharmaceutically acceptable salt, ester, stereoisomer, solvate, oxide or prodrug thereof, B is selected from the following groups unsubstituted or substituted with one or two identical or different substituents: 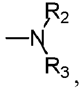 R₁-C≡CH and HetCyc¹; R₁ is selected from H, C1-C6 alkyl, deuterated C1-C6 alkyl and C1-C6 hydroxyalkyl; R₂ or R₃ is independently selected from H, C1-C6 alkyl, deuterated C1-C6 alkyl and hydroxy C1-C6 alkyl; the substituents are independently selected from halogen, hydroxy, -CN, =O (carbonyl), C1-C3 alkyl, deuterated C1-C3 alkyl, C1-C3 alkoxy, hydroxy C1-C3 alkyl, C1-C3 fluoroalkyl, cyano C1-C3 alkyl, (C1-C3 alkoxy) C1-C3 alkyl, C3-C6 cycloalkyl and (C1-C3 alkoxy SO₂) C1-C3 alkyl;

HetCyc¹ is a 4- to 8-membered heterocycle having 1-2 heteroatoms selected from N and O, a 7- to 11-membered spiro ring having 1-2 heteroatoms selected from N and O or an 8- to 10-membered fused heterocycle having 1-2 heteroatoms selected from N and O.

In some embodiments, in the compound of formula (I) or the pharmaceutically acceptable salt, ester, stereoisomer, solvate, oxide or prodrug thereof, B is selected from the following groups unsubstituted or substituted with one or two identical or different substituents: R₁-C≡CH, 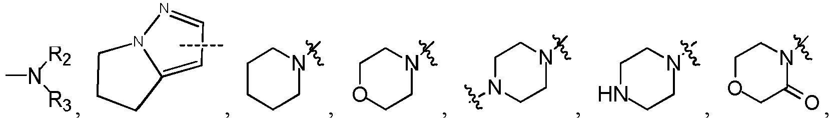 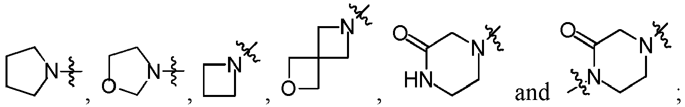 R₁ is selected from C1-C4 alkyl and hydroxy C1-C4 alkyl; R₂ or R₃ is independently selected from H, C1-C4 alkyl, deuterated C1-C4 alkyl and hydroxy C1-C4 alkyl; the substituents are independently selected from hydroxy, cyano, halogen, C1-C3 alkyl, deuterated C1-C3 alkyl, C1-C3 alkoxy and C3-C6 cycloalkyl.

In some embodiments, in the compound of formula (I) or the pharmaceutically acceptable salt, ester, stereoisomer, solvate, oxide or prodrug thereof, B is 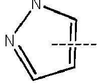 unsubstituted or substituted with one or two identical or different substituents; the substituents are independently selected from halogen, hydroxy, -CN, =O (carbonyl), C1-C3 alkyl, deuterated C1-C3 alkyl, C1-C3 alkoxy, hydroxy C1-C3 alkyl, C1-C3 fluoroalkyl, cyano C1-C3 alkyl, (C1-C3 alkoxy) C1-C3 alkyl, C3-C6 cycloalkyl and C1-C3 alkoxy SO₂) C1-C3 alkyl; C is a 5- to 6-membered heteroaromatic ring having 1-2 ring heteroatoms selected from N and S; D is 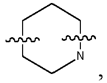 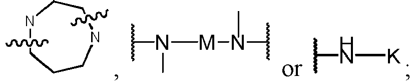 wherein M is selected from C3-C6 cycloalkyl; K is a 4- to 8-membered heterocycle having 1-3 heteroatoms selected from N and O; L is -CH₂-; E is

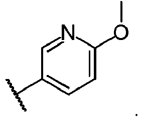

In some embodiments, in the compound of formula (I) or the pharmaceutically acceptable salt, ester, stereoisomer, solvate, oxide or prodrug thereof, C is the following group unsubstituted or substituted with one or two identical or different substituents: 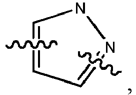 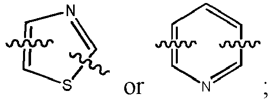 the substituents are independently selected from fluorine, chlorine and bromine.

In some embodiments, in the compound of formula (I) or the pharmaceutically acceptable salt, ester, stereoisomer, solvate, oxide or prodrug thereof, D is 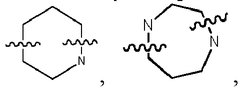 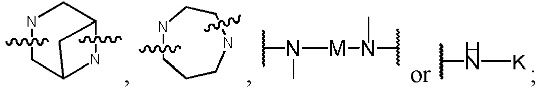 wherein M is selected from C1-C3 alkane and C3-C6 cycloalkyl; K is a 4- to 8-membered heterocycle having 1-3 heteroatoms selected from N and O; more preferably, D is -N(CH₃)CH₂CH₂N(CH₃)-,

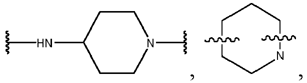

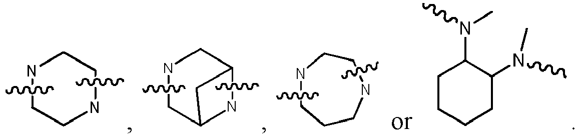

In some embodiments, in the compound of formula (I) or the pharmaceutically acceptable salt, ester, stereoisomer, solvate, oxide or prodrug thereof, L is

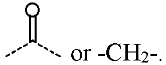

In some embodiments, in the compound of formula (I) or the pharmaceutically acceptable salt, ester, stereoisomer, solvate or prodrug thereof, E is 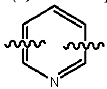 unsubstituted or substituted with one or two identical or different substituents; the substituents are independently selected from C1-C3 alkoxy and deuterated C1-C3 alkoxy.

In some embodiments, the present invention also provides a compound of formula (II) or a pharmaceutically acceptable salt, ester, stereoisomer, solvate, oxide or prodrug thereof, wherein

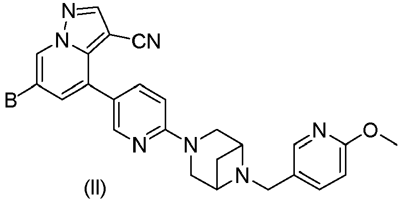

B is selected from HetCyc¹ unsubstituted or substituted with one or two identical or different substituents; HetCyc¹ is a 4- to 5-membered heterocycle having 1 N atom or 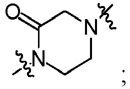 the substituents are independently selected from H, halogen, hydroxy, -CN, carbonyl, C1-C3 alkyl, deuterated C1-C3 alkyl, C1-C3 alkoxy, hydroxy C1-C3 alkyl, C1-C3 fluoroalkyl and cyano C1-C3 alkyl; in some embodiments, the substituents of B in formula (II) are independently selected from halogen, hydroxy, -CN, carbonyl, methyl, ethyl, deuterated methyl and methoxy.

In some embodiments, in the compound of formula (II) or the pharmaceutically acceptable salt, ester, stereoisomer, solvate or prodrug thereof according to the present invention, 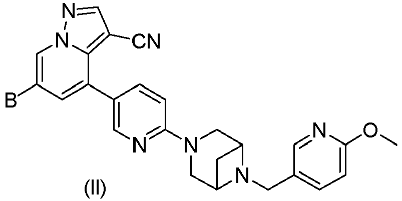 wherein, B is selected from substituted or unsubstituted HetCyc², or B is a substituted or unsubstituted 7- to 8-membered bridged ring having 1-3 heteroatoms selected from N, S and O; HetCyc² is a 4- to 6-membered heterocycle having a P atom or a 4- to 6-membered heterocycle having a P atom and a N atom; the substituents are independently selected from H, halogen, hydroxy, -CN, carbonyl, C1-C3 alkyl, deuterated C1-C3 alkyl, C1-C3 alkoxy, hydroxy C1-C3 alkyl, C1-C3 fluoroalkyl and cyano C1-C3 alkyl; in some embodiments, B is substituted or unsubstituted HetCyc², or B is a substituted or unsubstituted 7- to 8-membered bridged ring having 1-2 heteroatoms including N; HetCyc² is a 4- to 6-membered heterocycle having a P atom and a N atom; in some more specific embodiments, B is substituted or unsubstituted 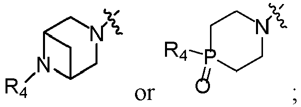 wherein R₄ is selected from H, halogen, hydroxy, -CN, carbonyl, C1-C3 alkyl, deuterated C1-C3 alkyl, C1-C3 alkoxy, hydroxy C1-C3 alkyl, C1-C3 fluoroalkyl and cyano C1-C3 alkyl; preferably, R₄ is selected from H, halogen, hydroxy, -CN, carbonyl, methyl, ethyl, deuterated methyl, methoxy or trifluoromethyl; in some embodiments, the substituents are independently selected from halogen, hydroxy, -CN, carbonyl, methyl, ethyl, deuterated methyl, methoxy and trifluoromethyl.

In some embodiments, in the compound of formula (II) or the pharmaceutically acceptable salt, ester, stereoisomer, solvate, oxide or prodrug thereof according to the present invention,

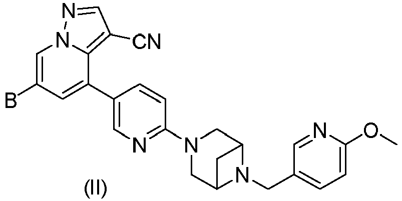

B is selected from HetCyc¹ unsubstituted or substituted with one or two identical or different substituents; HetCyc¹ is a 6-membered heterocycle having 1-2 N atoms; the substituents are independently selected from H, C1-C3 alkyl, deuterated C1-C3 alkyl and C1-C3 fluoroalkyl; in some more preferred embodiments, HetCyc¹ is 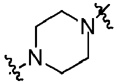 unsubstituted or substituted with substituents; the substituents of B are independently selected from halogen, methyl, ethyl, deuterated methyl and -CF₃.

The oxide described in the present invention may be formed by oxidation at any position susceptible to oxidation; in some specific embodiments, the oxide is formed by oxidation at a N atom of a 4- to 8-membered heterocycle or bridged ring having a N heteroatom; for example, in some embodiments, the oxide is formed by oxidation at a nitrogen atom of 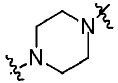 and

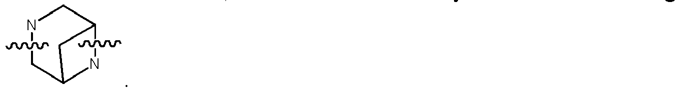

Some specific compounds of the compounds or the pharmaceutically acceptable salts thereof of the present invention are selected from:

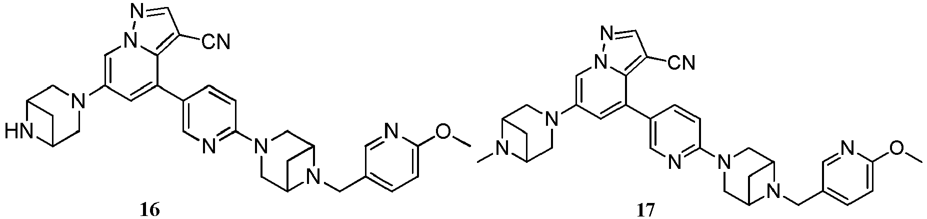

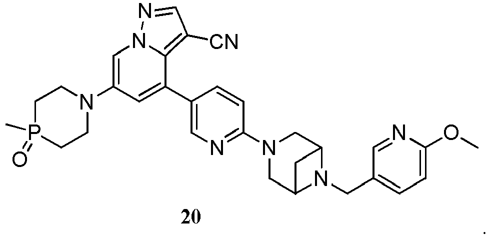

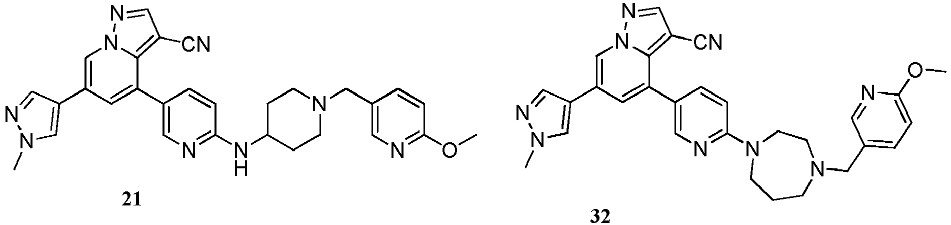

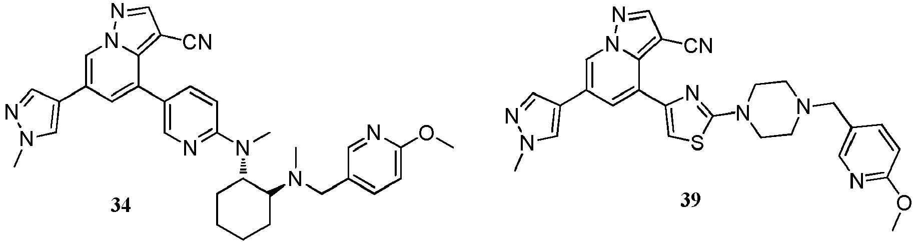

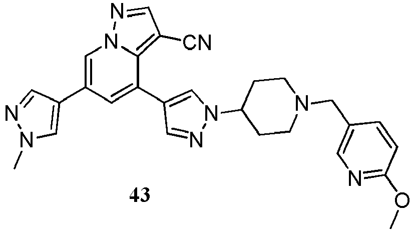

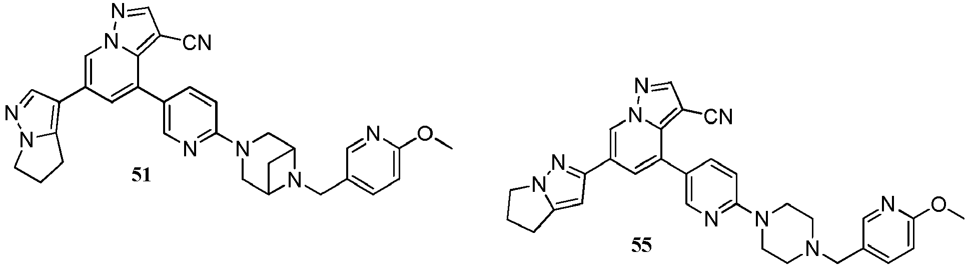

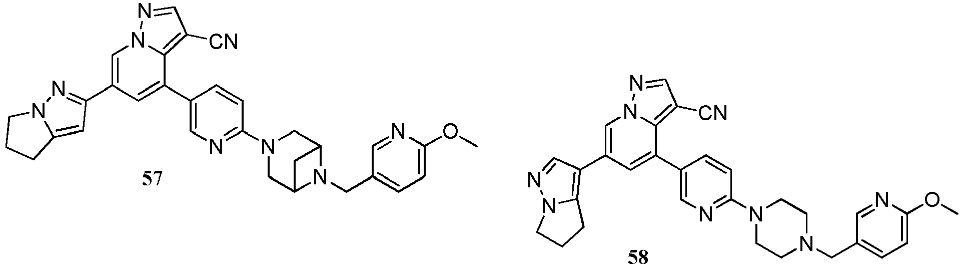

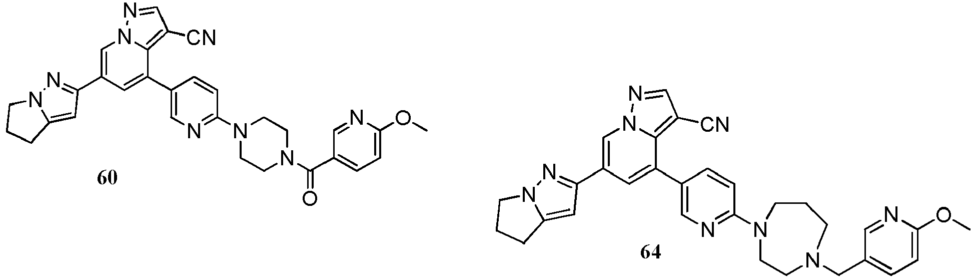

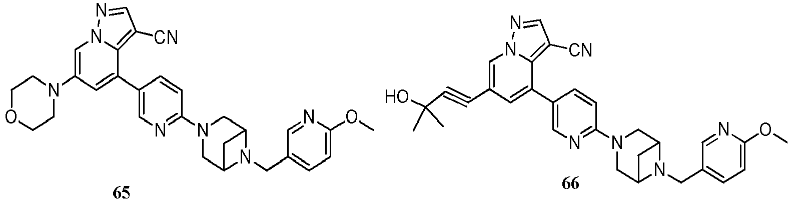

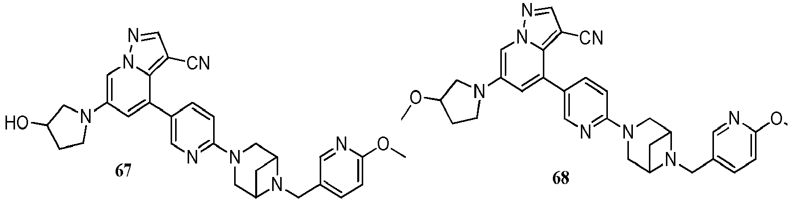

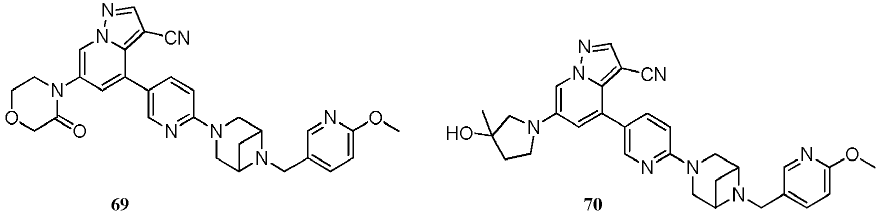

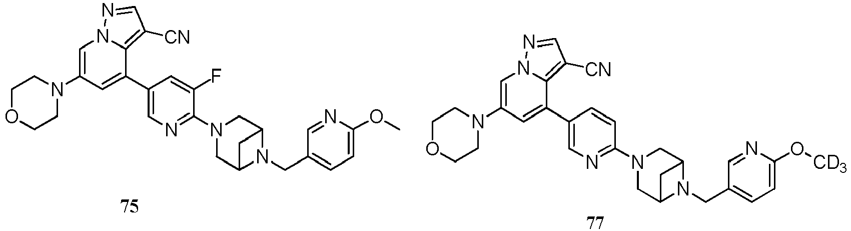

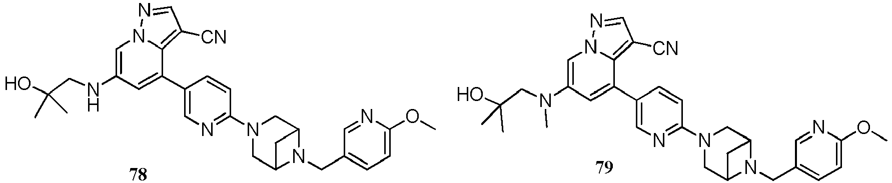

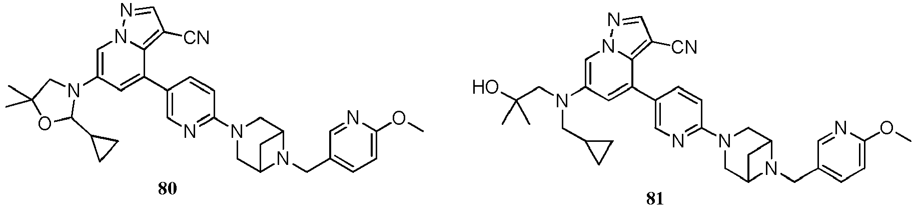

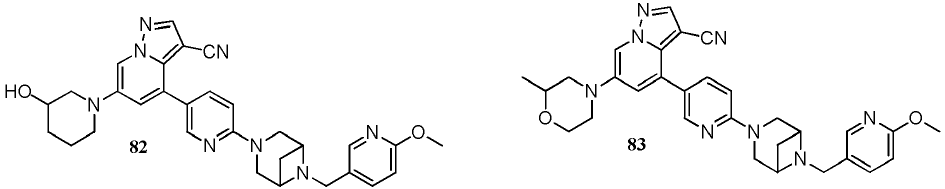

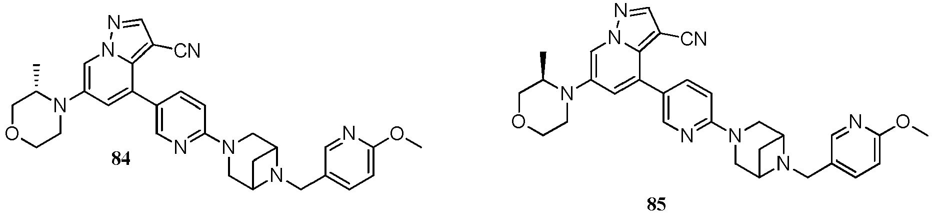

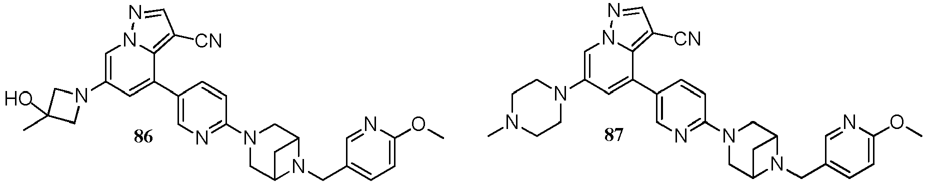

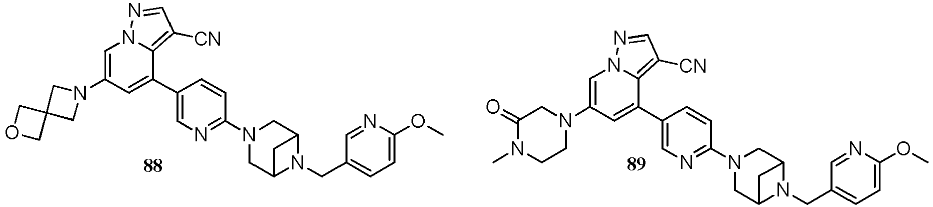

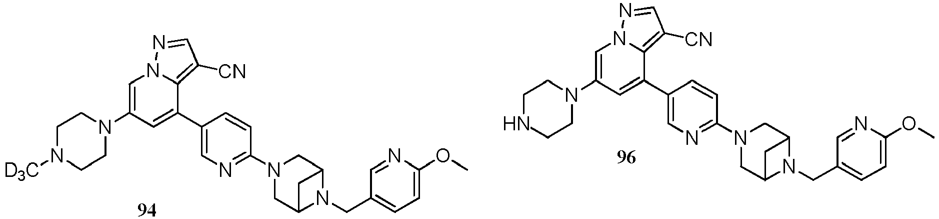

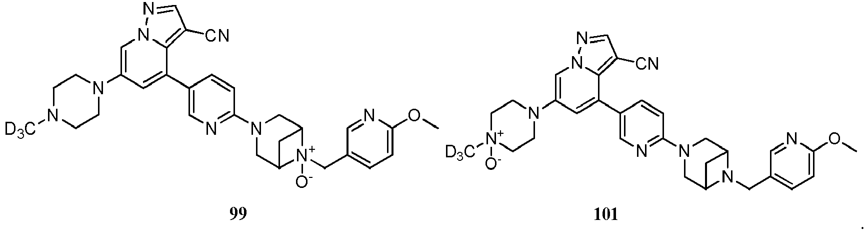

The present invention also provides a scheme for preparing a compound of general formula (I): 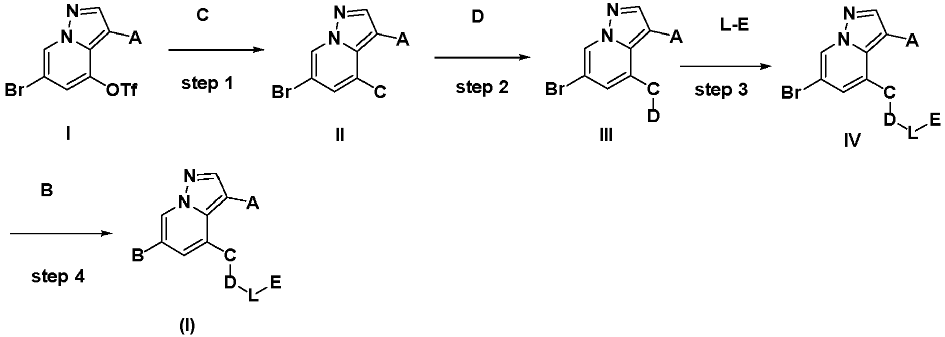 wherein A, B, C, D, L and E are as defined above.

In some embodiments, the scheme described above comprises the following specific steps:
step 1: carrying out a coupling reaction of compound **I** with a boric acid reagent C in solvent dioxane to give **II**;
step 2: carrying out a nucleophilic substitution reaction of intermediate II with an amine compound to give **III**;
step 3: carrying out a reductive amination reaction of or an acylation reaction of intermediate **III** with **L-E** in solvent 1,2-dichloroethane to give intermediate **IV;**
step 4: carrying out a C-N coupling reaction in solvents dioxane and *N,N-*dimethylformamide to give the final product (I).

The present invention also provides a method for preparing a compound of formula (II) or a pharmaceutically acceptable salt, ester, stereoisomer, solvate or prodrug thereof 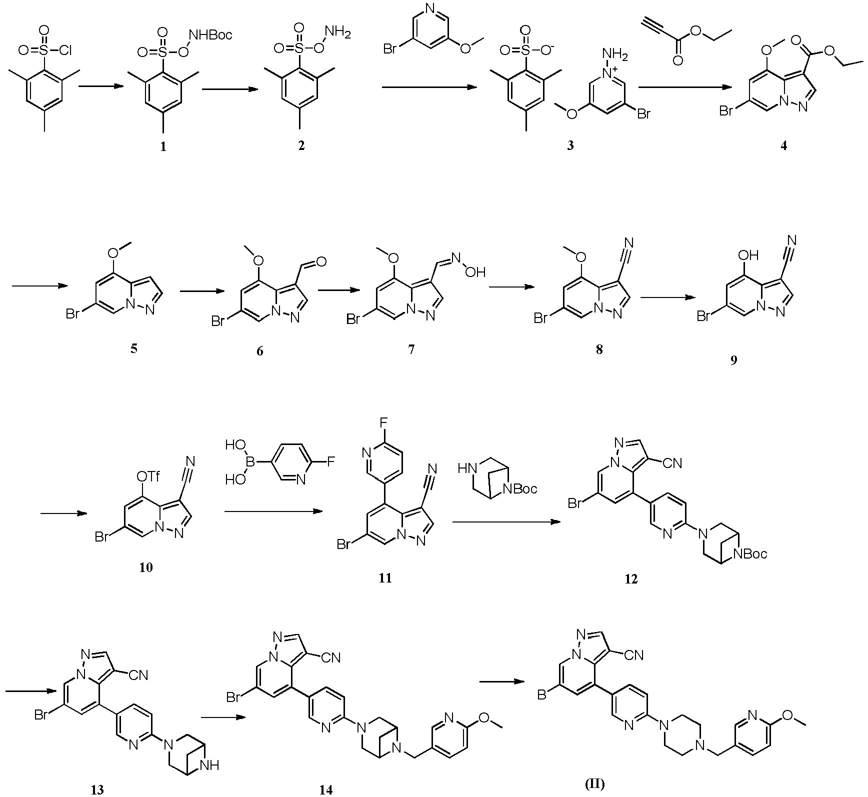 wherein B is as defined above.

The oxide of the present invention can be prepared by oxidation of compound IV before obtaining the compound of formula (I), or by oxidation after obtaining the compound of formula (I). The oxidation can be carried out using methods conventional in the art; for example, in some specific embodiments, common oxidants such as *m*-chloroperoxybenzoic acid (m-CPBA) are used to carry out the oxidation to give the oxides of the corresponding compounds.

The salts which the compound of the present invention may form are also within the scope of the present invention. Unless otherwise stated, the compound of the present invention is understood to include salts thereof. For example, the compound of formula (I) is reacted with an amount, e.g., an equivalent amount, of acid or base, and the product is isolated by salting out from a medium or by lyophilization in aqueous solution. The compound of the present invention contains basic moieties, including but not limited to amine or pyridine or imidazole rings, and may form salts with organic or inorganic acids. Non-limiting examples of the pharmaceutically acceptable salt of the compound of formula (I) include monohydrochloride, dihydrochloride, trifluoroacetate and ditrifluoroacetate salts.

The content, by weight, of the compound of the present invention, which is obtained by preparation, separation and then purification, is equal to or greater than 90%, e.g., is equal to or greater than 95%, or is equal to or greater than 99% ("very pure" compound), as listed in the text description. Herein, such "very pure" compounds of the present invention are also part of the present invention.

Also provided herein is a pharmaceutical composition comprising the compound of formula (I) or the pharmaceutically acceptable salt, ester, stereoisomer, solvate or prodrug thereof and a pharmaceutically acceptable carrier.

Also provided herein is a method for inhibiting cell proliferation *in vitro* or *in vivo,* which comprises contacting a cell with an effective amount of a compound of formula (I) as defined herein or a pharmaceutically acceptable salt or solvate or pharmaceutical composition thereof.

Also provided herein is a method for treating a RET-related disease or disorder in a patient in need of such treatment, which comprises administering to the patient a therapeutically effective amount of a compound of formula (I) as defined herein or a pharmaceutically acceptable salt or solvate or pharmaceutical composition thereof.

Also provided herein is use of the compound or the pharmaceutically acceptable salt, ester, stereoisomer, solvate or prodrug thereof described in the present invention as a RET kinase inhibitor.

Also provided herein is use of the compound or the pharmaceutically acceptable salt, ester, stereoisomer, solvate or prodrug thereof described in the present invention in the manufacture of a medicament for the treatment of a RET-related disease.

In some examples of the present invention, the RET-related disease or disorder is cancer.

Also provided herein is a method for treating cancer and/or inhibiting cancer metastasis related to a particular cancer in a patient in need of such treatment, which comprises administering to the patient a therapeutically effective amount of a compound of formula (I) as defined herein or a pharmaceutically acceptable salt or solvate or pharmaceutical composition thereof.

Also provided herein is a compound of formula (I) as defined herein or a pharmaceutically acceptable salt or solvate or pharmaceutical composition thereof for use in therapy.

Also provided herein is a compound of formula (I) as defined herein or a pharmaceutically acceptable salt or solvate or pharmaceutical composition thereof for use in the treatment of cancer and/or in the inhibition of cancer metastasis related to a particular cancer.

Also provided herein is a compound of formula (I) or a pharmaceutically acceptable salt or solvate thereof for use in the inhibition of RET kinase activity.

Also provided herein is a compound of formula (I) as defined herein or a pharmaceutically acceptable salt or solvate or pharmaceutical composition thereof for use in the treatment of a RET-related disease or disorder.

Also provided herein is use of a compound of formula (I) as defined herein or a pharmaceutically acceptable salt or solvate thereof for the manufacture of a medicament for the treatment of cancer and/or for the inhibition of cancer metastasis related to a particular cancer.

Also provided herein is use of a compound of formula (I) as defined herein or a pharmaceutically acceptable salt or solvate thereof for the manufacture of a medicament for the inhibition of RET kinase activity.

Also provided herein is use of a compound of formula (I) as defined herein or a pharmaceutically acceptable salt or solvate thereof for the manufacture of a medicament for the treatment of a RET-related disease or disorder.

Also provided herein is a method for treating cancer in a patient in need, which comprises: (a) determining whether the cancer is related to the dysregulation of the expression or activity or level of a RET gene, a RET kinase or any one of them (e.g., a RET-related cancer); and (b) administering to the patient a therapeutically effective amount of a compound of formula (I) or a pharmaceutically acceptable salt or solvate or pharmaceutical composition thereof, if it is determined that the cancer is related to the dysregulation of the expression or activity or level of the RET gene, the RET kinase or any one of them (e.g., a RET-related cancer).

Also provided herein is a pharmaceutical combination for treating cancer (e.g., a RET-related cancer, such as a RET-related cancer with one or more RET inhibitor resistance mutations) in a patient in need thereof, which comprises: (a) a compound of formula (I) or a pharmaceutically acceptable salt or solvate thereof, (b) an additional therapeutic agent, and (c) optionally at least one pharmaceutically acceptable carrier, wherein the compound of formula (I) or the pharmaceutically acceptable salt or solvate thereof and the additional therapeutic agent are prepared in separate compositions or doses for concurrent, separate or sequential use in the treatment of cancer, wherein the amount of the compound of formula (I) or the pharmaceutically acceptable salt or solvate thereof and the amount of the additional therapeutic agent together are effective in the treatment of cancer. Also provided herein is a pharmaceutical composition comprising such a combination. Also provided herein is use of such a combination for the manufacture of a medicament for the treatment of cancer. Also provided herein are a commercial package or a product comprising such a combination in a combination preparation for concurrent, separate or sequential use, and a method for treating cancer in a patient in need.

Also provided herein is a method of reversing or preventing acquired resistance to an anti-cancer medicament, which comprises administering to a patient at risk of developing or having acquired resistance to an anti-cancer medicament a therapeutically effective amount of a compound of formula (I) or a pharmaceutically acceptable salt or solvate thereof. In some embodiments, a dose of an anti-cancer medicament is administered to a patient (e.g., substantially concurrently with administration of a dose of a compound of formula (I) or a pharmaceutically acceptable salt or solvate thereof to the patient).

Also provided herein is a method for delaying and/or preventing the development of cancer resistance to an anti-cancer medicament in an individual, which comprises administering to the individual an effective amount of a compound of formula (I) or a pharmaceutically acceptable salt or solvate thereof before, during or after administering an effective amount of the anti-cancer medicament.

Also provided herein is a method for treating an individual with cancer and increased likelihood of developing resistance to an anti-cancer medicament, which comprises administering to the individual (a) an effective amount of a compound of formula (I) before, during or after administering (b) an effective amount of the anti-cancer medicament.
Also provided is a method for treating an individual with a RET-related cancer and one or more RET inhibitor resistance mutations which increase resistance of cancer to a first RET inhibitor (e.g., a substitution at amino acid position 804, such as V804M, V804L or V804E), the method comprising administering a compound of formula (I) or a pharmaceutically acceptable salt or solvate thereof before, during or after administering another anti-cancer medicament (e.g., a second-generation RET kinase inhibitor).

Also provided is a method for treating a subject with a RET-related cancer, which comprises administering a compound of formula (I) or a pharmaceutically acceptable salt or solvate thereof before, during or after administering another anti-cancer medicament (e.g., a first-generation RET kinase inhibitor).

In some embodiments of any method or use described herein, the cancer (e.g., the RET-related cancer) is hematologic cancer. In some embodiments of any method or use described herein, the cancer (e.g., the RET-related cancer) is a solid tumor. In some embodiments of any method or use described herein, the cancer (e.g., the RET-related cancer) is lung cancer (e.g., small cell lung cancer or non-small cell lung cancer), thyroid cancer (e.g., papillary thyroid cancer, medullary thyroid cancer, differentiated thyroid cancer, recurrent thyroid cancer or refractory differentiated thyroid cancer), thyroid adenoma, pheochromocytoma and paraganglioma (PPGL), lung adenocarcinoma, bronchioloalveolar carcinoma, multiple endocrine neoplasia type 2A or 2B (MEN2A or MEN2B), pheochromocytoma, parathyroid hyperplasia, breast cancer, mammary cancer, mammary carcinoma, breast neoplasm, colorectal cancer (e.g., metastatic colorectal cancer), papillary renal cell carcinoma, gastrointestinal mucosal gangliocytoma, inflammatory myofibroblastic tumors or cervical cancer.

In some embodiments, the patient is a human.

The compound of formula (I) and the pharmaceutically acceptable salt and solvate thereof are also suitable for use in the treatment of a RET-related cancer.

Also provided herein is a method for treating a patient diagnosed with or determined as having a RET-related cancer (e.g., any one of the exemplary RET-related cancers disclosed herein), which comprises administering to the patient a therapeutically effective amount of a compound of formula (I) as defined herein or a pharmaceutically acceptable salt or solvate or pharmaceutical composition thereof.

Also provided herein is use of the compound or the pharmaceutically acceptable salt, ester, stereoisomer, solvate or prodrug thereof in the manufacture of an FGFR family kinase inhibitor medicament; the FGFR family described herein includes, but is not limited to, FGFR1, FGFR1 V561M, FGFR2, FGFR2 V564F, FGFR2 N549H, FGFR2 V564I, FGFR2 K641R, FGFR3, FGFR3 V555M, FGFR3 K650E and FGFR4; in some more specific embodiments, the FGFR family includes FGFR2 V564F, FGFR2 N549H, FGFR2 V564I and FGFR3 V555M.

The terms of the present invention are defined as follows unless otherwise stated:
The term "halogen" refers to -F (sometimes referred to herein as "fluorine"), -Cl, -Br and -I.

The terms "C1-C3 alkyl", "C1-C6 alkyl", "C2-C6 alkyl" and "C3-C6 alkyl" refer to saturated, linear or branched chain monovalent hydrocarbyl groups having one to three, one to six, two to six or three to six carbon atoms, respectively. Examples include, but are not limited to, methyl, ethyl, 1-propyl, isopropyl, 1-butyl, isobutyl, sec-butyl, tert-butyl, 2-methyl-2-propyl, pentyl, neopentyl and hexyl.

The term "C1-C6 alkoxy" refers to saturated, linear or branched chain monovalent alkoxy having one to six carbon atoms, in which the bond is on an oxygen atom. Examples include methoxy, ethoxy, propoxy, isopropoxy, butoxy and tert-butoxy.

The terms "(C1-C6 alkoxy) C1-C6 alkyl-" and "(C1-C6 alkoxy) C2-C6 alkyl-" refer to saturated, linear or branched chain monovalent groups having one to six carbon atoms or two to six carbon atoms, respectively, one of which is substituted with a (C1-C6 alkoxy) group as defined herein. Examples include methoxymethyl (CH₃OCH₂-) and methoxyethyl (CH₃OCH₂CH₂-).

The terms "hydroxy C1-C6 alkyl-" and "hydroxy C2-C6 alkyl-" refer to saturated, linear or branched chain monovalent alkyl groups having one to six or two to six carbon atoms, respectively, one of which is substituted with a hydroxy group.

The terms "deuterated C1-C6 alkyl-", "halo C1-C6 alkyl" and "cyano C1-C6 alkyl" refer to saturated, linear or branched chain monovalent alkyl groups having one to six carbon atoms, one of which is substituted with deuterium, halogen or cyano, respectively.

The term "C3-C6 cycloalkyl" refers to cyclopropyl, cyclobutyl, cyclopentyl or cyclohexyl.

The term "alkenyl" refers to linear chain, branched chain or cyclic, nonaromatic hydrocarbyl containing at least one carbon-carbon double bond. Thus, "C2-C6 alkenyl" refers to alkenyl having 2 to 6 carbon atoms. Examples include, but are not limited to, ethenyl, propenyl, butenyl, 2-methylbutenyl, cyclohexenyl, and the like.

The term "alkynyl" refers to linear chain, branched chain or cyclic, nonaromatic hydrocarbyl containing at least one carbon-carbon triple bond. Thus, "C2-C6 alkynyl" refers to alkynyl having 2 to 6 carbon atoms. Examples include, but are not limited to, ethynyl, propynyl, butynyl, 3-methylbutynyl, and the like.

The term "heterocycle" refers to a monocyclic or bicyclic non-aromatic heterocycle containing, in addition to carbon atoms, 1 to 4 heteroatoms selected from the group consisting of oxygen atom, sulfur atom and nitrogen atom, and the following can be listed as specific examples: 4- to 7-membered monocyclic non-aromatic heterocycles containing, in addition to carbon atoms, 1 to 2 heteroatoms selected from the group consisting of oxygen atom, sulfur atom and nitrogen atom such as azetidine, pyrrolidine, pyrazolidine, piperidine, oxetane, tetrahydrofuran, tetrahydropyran, tetrahydrothiophene, dihydroimidazole, imidazolidine, tetrahydropyrazine, piperazine and morpholine; 6- to 8-membered bicyclic non-aromatic heterocycles containing, in addition to carbon atoms, 1-4 heteroatoms selected from the group consisting of oxygen atom, sulfur atom and nitrogen atom such as azabicyclo[3.1.0]hexane.

The term "heteroaromatic ring" represents a stable monocyclic ring containing up to 3-10 atoms in the ring or a bicyclic carbon ring containing up to 3-10 atoms in each ring, in which at least one ring is aromatic and contains 1-4 heteroatoms selected from O, N and S. Heteroaryl groups within the scope of this definition include, but are not limited to, acridinyl, carbazolyl, cinnolinyl, quinoxalinyl, pyrazolyl, indolyl, benzotriazolyl, furanyl, thienyl, benzothienyl, benzofuranyl, quinolinyl, isoquinolinyl, oxazolyl, isoxazolyl, indolyl, pyrazinyl, pyridazinyl, pyridinyl, pyrimidinyl and pyrrolyl.

The term "spiro ring" refers to a group of two rings connected by a spiro connection of a carbon atom, in which each ring has 4 to 6 ring atoms (one ring carbon atom is shared by the two rings).

The term "heterospirocycle" refers to a group of two rings connected by a spiro connection of a carbon atom containing one or more identical or different heteroatoms selected from nitrogen atom and oxygen atom, in which each ring has 4 to 6 ring atoms (one ring carbon atom is shared by the two rings).

The term "fused heterocycle" refers to a cyclic hydrocarbon in which 2-3 rings share two adjacent (ortho) atoms and in which at least one ring is an aromatic ring containing 1 to 3 heteroatoms selected from O, N and S, and to, in some embodiments, a cyclic hydrocarbon in which 2 rings share two adjacent (ortho) atoms and in which one ring is an aromatic ring containing 1 to 3 heteroatoms selected from O, N and S and the other ring is a saturated heterocycle.

The term "bridged ring" refers to polycyclic hydrocarbons in which two or more carbon atoms (bridgehead carbon atoms) are shared, which are classified into bicyclic hydrocarbons, tricyclic hydrocarbons and tetracyclic hydrocarbons and the like according to the number of constituent rings.

The use of the term "treating" or "treatment" as referred to throughout this document is conventional, e.g., is to manage or care for an individual for the purpose of resisting, alleviating, reducing or ameliorating the condition of a disease or disorder such as cancer.

The term "individual" or "patient" includes organisms, such as humans and non-human animals, which are capable of developing a cell proliferative disorder or which could benefit from administration of the compound of the present invention. Preferred humans include human patients suffering from or susceptible to a cell proliferative disorder as described herein or a related condition. The term "non-human animals" include vertebrates, e.g., mammals, such as non-human primates, sheep, cows, dogs, cats and rodents (e.g., mice), as well as non-mammals, e.g., chickens, amphibians, reptiles, and the like.

The term "cell proliferation" includes undesired or uncontrolled proliferation of cells. The compound of the present invention can be used to prevent, inhibit, block, reduce, control, etc., cell proliferation and/or cell division, and/or to cause apoptosis. The method comprises administering to an individual (including mammals, including humans) in need thereof an amount of the compound of the present invention or a pharmaceutically acceptable salt, isomer, polymorph, metabolite, hydrate or solvate thereof effective in the treatment or prevention of the disorder.

Compared with the prior art, the present invention has the following beneficial effects.

The RET kinase inhibitor compound of the present invention has enzyme and cell level biological activity superior to that of the drug selpercatinib (LOXO-292) on the market and has less cardiotoxicity. The compound of the present invention provides more options for novel anti-tumor drugs and holds promise for being applied to drugs.

### DETAILED DESCRIPTION

The following representative examples are intended to aid in the illustration of the present invention and are not intended to be, nor should they be construed as, limiting the scope of the present invention. In practice, except for those presented and described herein, the entire content of the document in the present application, including examples in accordance with the scientific literature and patents cited herein, as well as various modifications and numerous further variations resulting therefrom, will be apparent to those skilled in the art. It should also be understood that the citation of these references is helpful in setting forth the disclosure herein. The following examples contain important supplementary information, exemplification and guidance, adaptable to various variations of the present invention and the like.

### Example 1

The synthesis scheme is as follows:

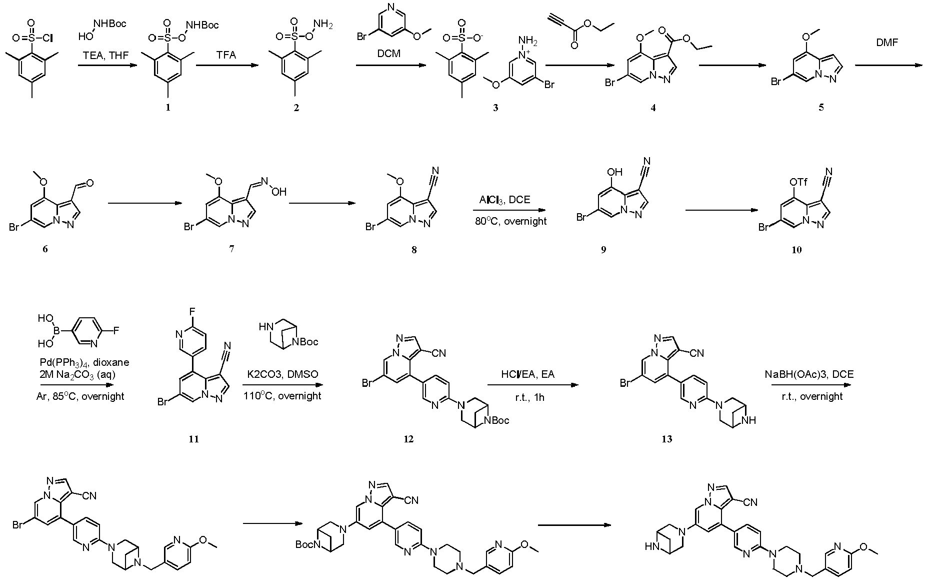

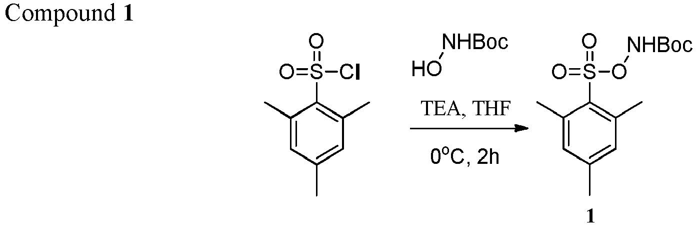

A 2 L three-necked flask, a low-temperature thermometer and an argon protective device were prepared. To the three-necked flask were added successively 2,4,6-trimethylbenzenesulfonyl chloride (80 g, 367 mmol), tert-butyl *N*-hydroxycarbamate (62.4 g, 468 mmol) and THF (1.2 L). The temperature was reduced to 0 °C, and TEA (64 mL) was slowly added dropwise at that temperature. The mixture was stirred at room temperature for another 1 h, and TLC monitoring showed no starting material remained. The solvent was distilled off under reduced pressure. EA (1.2 L) was added to dissolve the residue, and the solution was washed with H₂O (1.2 L × 3) and with 10% NaHCO₃ solution (1.2 L), dried over anhydrous Na₂SO₄ and distilled under reduced pressure to give product **1** as a pale yellow solid (110 g, 95% yield).

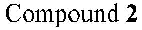

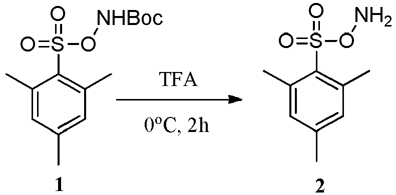

A 500 mL three-necked flask, a low-temperature thermometer and an argon protective device were prepared. To the three-necked flask was added TFA (150 mL). The temperature was reduced to 0 °C, and **1** (110 g, 348.8 mmol) was added in batches at that temperature. The mixture was stirred at that temperature for another 3 h, and TLC monitoring showed no starting material remained. The reaction mixture was slowly poured into ice water being vigorously stirred. After 10 min of stirring, a white solid precipitated. The mixture was filtered, and the filter cake was washed with ice water and dried to give product **2** as a white solid (60.1 g, 80% yield).

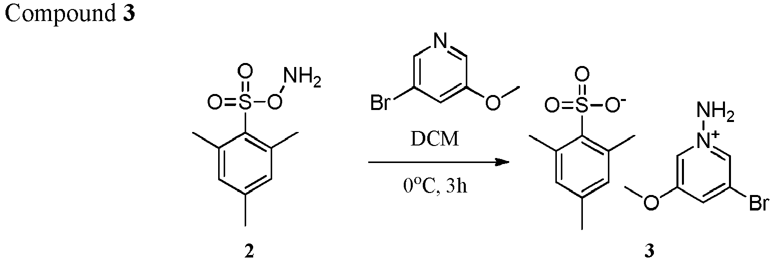

A 500 mL three-necked flask, a low-temperature thermometer and an argon protective device were prepared. To the three-necked flask were added successively **2** (60 g, 278.5 mmol), 3-bromo-5-methoxypyridine (52.37 g, 278.6 mmol) and DCM (1 L). The temperature was reduced to 0 °C, and additional 3-bromo-5-methoxypyridine (523 mg, 2.8 mmol) was added after 1 h of stirring. The mixture was stirred at 0 °C for another 2 h. PE (1 L) was added at that temperature. After 10 min of stirring, a white solid precipitated. The mixture was filtered, and the filtrate was concentrated. DCM (300 mL) was added to dissolve the residue. After the temperature was reduced to 0 °C, PE (300 mL) was added. The mixture was stirred for another 10 min, and a white solid precipitated. The mixture was filtered, and the solids obtained were combined and dried to give product **3** as a white solid (87.6 g, 78% yield).

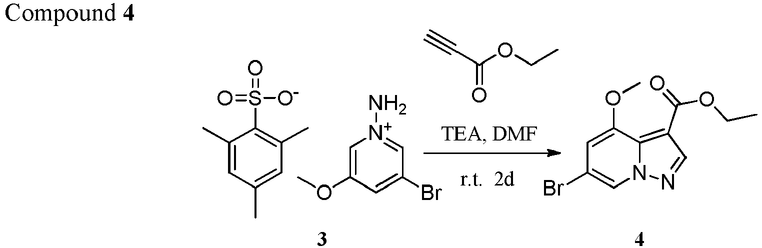

To a 500 mL single-neck flask were added successively **3** (87.6 g, 217.2 mmol), ethyl propiolate (42.6 g, 434.4 mmol) and DMF (200 mL), and TEA (60.4 mL, 434.4 mmol) was slowly added dropwise at room temperature. After the addition was completed, the mixture was stirred at room temperature for another 2 d, and TLC monitoring showed no starting material remained. The mixture was diluted and quenched by adding H₂O (600 mL) and extracted with EA (200 mL × 3). The extracts were combined, concentrated and purified by column chromatography to give product **4** as an orange solid (48.7 g, 75% yield).

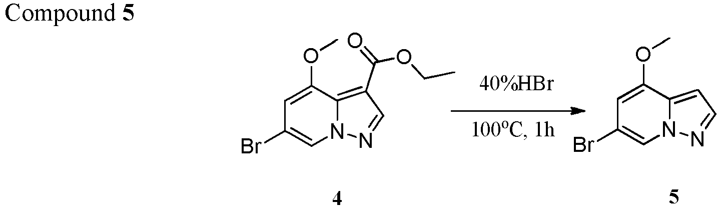

To a 1 L single-neck eggplant-shaped flask was added 40% HBr (400 mL), and **4** (48.7 g, 162.8 mmol) was added in batches with stirring. The mixture was warmed to 100 °C and stirred for another 1 h, and TLC monitoring showed no starting material remained. The mixture was cooled to room temperature and then poured into crushed ice, and the mixture was stirred. The pH was adjusted to >8 with 2 M NaOH solution, and a solid precipitated. The solid was collected by filtration and dried to give product 5 as a pink solid (34.38 g, 93% yield).

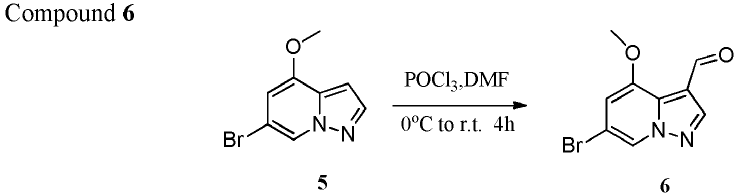

To a 500 mL single-neck flask were added successively **5** (34.38 g, 151.4 mmol) and DMF (200 mL), and POCl₃ (76 mL) was slowly added dropwise at 0 °C. After the addition was completed, the mixture was warmed to room temperature and stirred for another 4 h, and TLC monitoring showed no starting material remained. The mixture was poured into 200 mL of H₂O for dilution, and the pH was adjusted to >8 with 3 M NaOH solution. The mixture was stirred at room temperature for 20 min, and a solid precipitated. The solid was collected by filtration and dried to give product **6** as a gray solid (34.7 g, 90% yield).

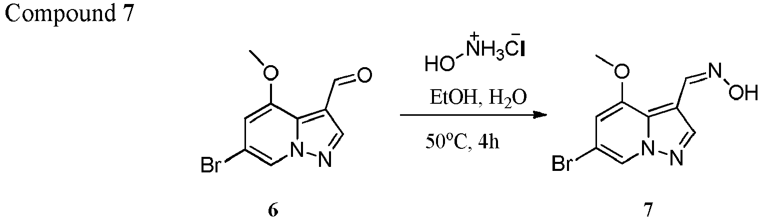

To a 500 mL single-neck flask were added successively **6** (34.38 g, 151.4 mmol), hydroxylamine hydrochloride (13.7 g, 196.82 mmol), EtOH (250 mL) and H₂O (80 mL). The mixture was stirred at 50 °C for 4 h, and TLC monitoring showed no starting material remained. EtOH was distilled off under reduced pressure. 250 mL of H₂O was added. The pH was adjusted to 9 with saturated NaHCOs solution, and a solid precipitated. The solid was collected by filtration and dried to give product 7 as a gray solid (38 g, 93% yield).

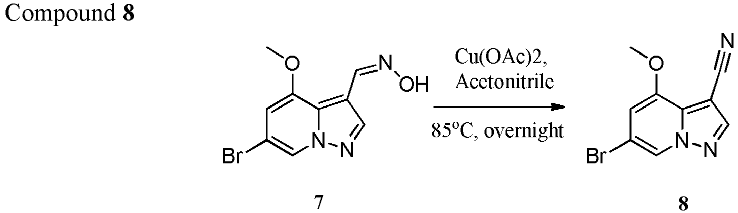

To a 500 mL single-neck flask were added successively **7** (38 g, 140.8 mmol), Cu(OAc)₂ (25.6 g, 140.8 mmol) and acetonitrile (200 mL). The mixture was reacted at 85 °C overnight, and TLC monitoring showed no starting material remained. The mixture was cooled to room temperature. The pH was adjusted to 8 with NH₃·H₂O, and a solid precipitated. The mixture was filtered, and the filter cake was triturated and washed with methyl *tert*-butyl ether. The solid was collected by filtration and dried to give product **8** as a brownish-yellow solid (19.4 g, 55% yield).

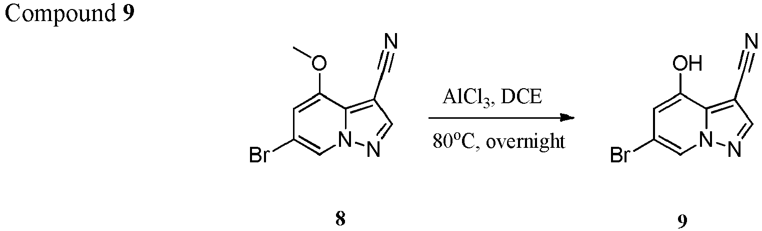

To a 45 mL sealed tube were added successively **8** (5 g, 19.8 mmol) and 1,2-dichloroethane (100 mL), and AlCl₃ (9.34 mg, 70 mmol) was added in batches at room temperature. The mixture was stirred at 80 °C overnight, and TLC monitoring showed no starting material 8 remained. The mixture was cooled to room temperature, quenched by adding Na₂SO₄·10H₂O, stirred for 1 h, filtered, washed with MeOH, concentrated to remove the solvent and purified by column chromatography to give product 9 as a gray solid (3 g, 55% yield).

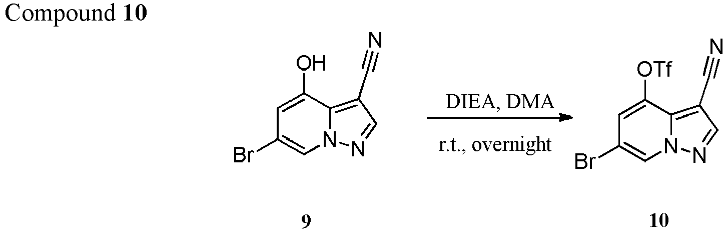

To a 50 mL single-neck flask were added successively compound 9 (3 g, 12.6 mmol), N-phenylbis(trifluoromethanesulfonyl)imide (4.5 g, 12.6 mmol), DIEA (3.26 g, 25.2 mmol) and DMA (20 mL). The mixture was stirred at room temperature overnight, and TLC monitoring showed the reaction was completed. The reaction mixture was poured into 60 mL of H₂O being stirred, and a brown solid precipitated. The solid was collected by filtration and dried to give product 10 as a brown solid (4.4 g, 95% yield).

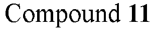

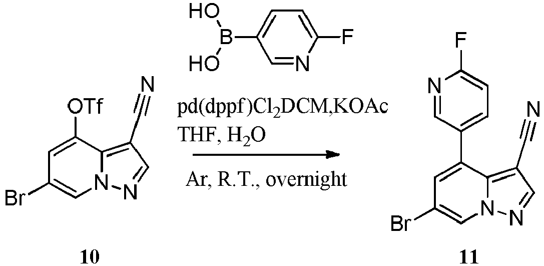

To a 12 mL sealed tube were added successively **10** (4.4 g, 11.9 mmol), Pd(dppf)Cl₂ complexed with dichloromethane (490 mg, 0.6 mmol), 2-fluoropyridine-5-boronic acid (1.68 g, 11.9 mmol), KOAc (2.92 g, 29.75 mmol) and dioxane (50 mL). The mixture was stirred at 85 °C overnight under Ar, and TLC monitoring showed the reaction was completed. The mixture was cooled to room temperature, and 100 mL of water was added. The mixture was stirred for 10 min, and a brownish-yellow solid precipitated. The solid was collected by filtration and dried to give product **11** (3 g, 80% yield).

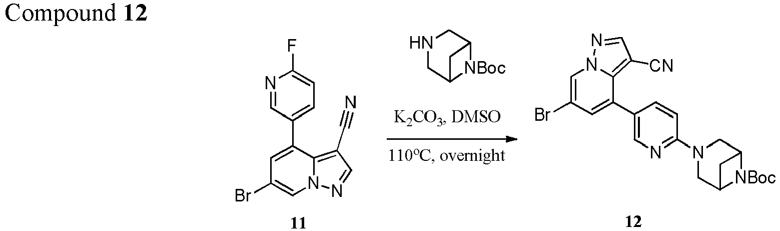

To a 45 mL sealed tube were added successively **11** (3 g, 9.46 mmol), *6-(tert-*butoxycarbonyl)-3,6-diazabicyclo[3.1.1]heptane (2.25 g, 11.3 mmol), K₂CO₃ (3.9 g, 28.35 mmol) and DMSO (20 mL). The mixture was stirred at 110 °C overnight, and TLC monitoring showed the reaction was completed. The mixture was cooled to room temperature, and 50 mL of water was added. The mixture was stirred for 10 min, and a yellow solid precipitated. The solid was collected by filtration and dried to give product **12** (3.2 g, 69% yield).

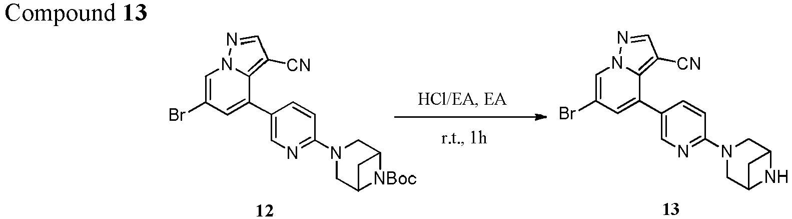

To a 25 mL single-neck flask was added a 3.5 M solution of HCl in EA (10 mL), and a solution of **12** (94.5 mg, 0.2 mmol) in EA was slowly added dropwise. After the addition was completed, the mixture was stirred at room temperature for another 1 h, concentrated to remove the solvent, neutralized by adding a solution of NH₃ in methanol, concentrated and purified by column chromatography to give product **13** as a brownish-yellow solid (68 mg, 90% yield).

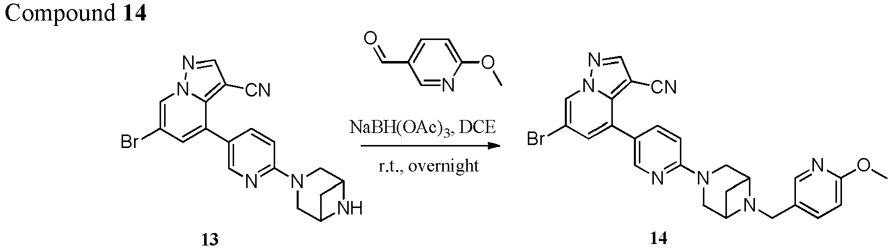

To a 50 mL single-neck flask were added successively **13** (68 mg, 0.18 mmol), 6-methoxy-3-pyridinecarboxaldehyde (30.2 mg, 0.22 mmol) and DCM (10 mL). After 10 min of stirring, NaBH(OAc)₃ (190.8 mg, 0.9 mmol) was added in batches at room temperature. The mixture was stirred at room temperature overnight, and TLC monitoring showed no starting material **13** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **14** as a yellow solid (55 mg, 61% yield).

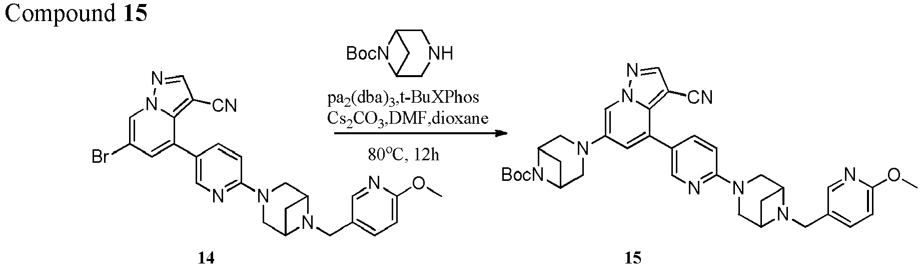

To a 25 mL sealed tube were added successively 14 (500 mg, 0.97 mmol), Pd₂(dba)₃ (106.4 mg, 0.12 mmol), *t*-BuXPhos (152.9 mg, 0.36 mmol), 6-(tert-butoxycarbonyl)-3,6-diazabicyclo[3.1.1]heptane (384.12 mg, 1.94 mmol), Cs₂CO₃ (632.1 mg, 1.94 mmol), dioxane (6 mL) and DMF (3 mL). The mixture was stirred at 80 °C overnight under Ar, and TLC monitoring showed no starting material **14** remained. The mixture was cooled to room temperature, and 10 mL of water was added. The mixture was stirred for 10 min, and a yellow solid precipitated. The solid was collected by filtration, dried and purified by column chromatography to give product **15** (450 mg, 73% yield).
¹H NMR (400 MHz, CDCl₃) δ 8.43 (d, *J* = 2.2 Hz, 1H), 8.17 (s, 1H), 8.11 (d, *J* = 2.1 Hz, 1H), 7.95 (d, *J* = 2.1 Hz, 1H), 7.83 (dd, *J* = 8.8, 2.5 Hz, 1H), 7.64 (dd, *J* = 8.5, 2.4 Hz, 1H), 7.10 (d, *J* = 2.1 Hz, 1H), 6.72 (dd, *J* = 8.6, 6.0 Hz, 2H), 4.00 (d, *J* = 5.9 Hz, 2H), 3.92 (d, *J* = 7.4 Hz, 3H), 3.85 (d, *J* = 12.0 Hz, 2H), 3.77 (t, *J* = 9.9 Hz, 2H), 3.60 (d, *J* = 13.1 Hz, 8H), 2.86 (dt, *J* = 8.8, 6.2 Hz, 1H), 2.70 (dd, *J* = 14.2, 6.2 Hz, 1H), 1.69 (dd, *J* = 20.8, 8.8 Hz, 2H),1.42(s, 9H)

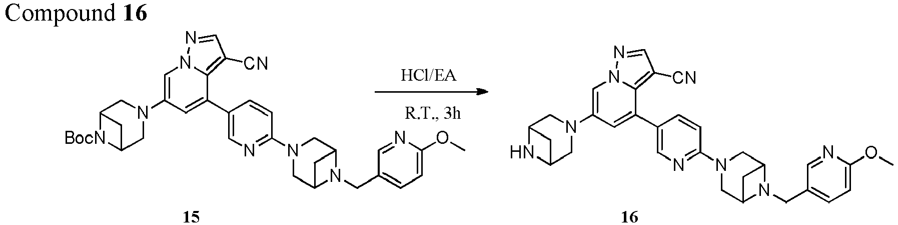

To a 25 mL single-neck eggplant-shaped flask was added a solution of hydrochloric acid in ethyl acetate (10 mL), and **15** (450 mg, 0.71 mmol) was slowly added. The mixture was stirred at room temperature for 3 h, and TLC monitoring showed no starting material remained. The mixture was concentrated, adjusted to pH 9 with ammonia water and extracted with DCM (3 mL × 3). The organic phases were combined, concentrated and purified by column chromatography to give the target product 16 as a yellow solid (350 mg, 92% yield).
¹H NMR (400 MHz, CDCl₃) δ 8.43 (d, *J* = 2.2 Hz, 1H), 8.17 (s, 1H), 8.11 (d, *J* = 2.1 Hz, 1H), 7.95 (d, *J* = 2.1 Hz, 1H), 7.83 (dd, *J* = 8.8, 2.5 Hz, 1H), 7.64 (dd, *J* = 8.5, 2.4 Hz, 1H), 7.10 (d, *J* = 2.1 Hz, 1H), 6.72 (dd, *J* = 8.6, 6.0 Hz, 2H), 4.00 (d, *J* = 5.9 Hz, 2H), 3.92 (d, *J* = 7.4 Hz, 3H), 3.85 (d, *J* = 12.0 Hz, 2H), 3.77 (t, *J* = 9.9 Hz, 2H), 3.60 (d, *J* = 13.1 Hz, 8H), 2.86 (dt, *J* = 8.8, 6.2 Hz, 1H), 2.70 (dd, *J* = 14.2, 6.2 Hz, 1H), 1.69 (dd, *J* = 20.8, 8.8 Hz, 2H).

### Example 2

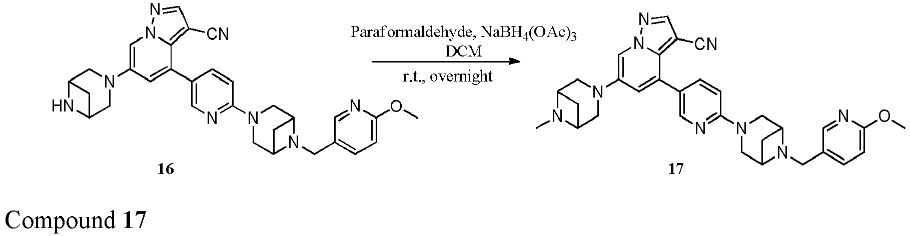

To a 25 mL single-neck eggplant-shaped flask were added 16 (200 mg, 0.37 mmol), paraformaldehyde (333 mg, 3.7 mmol) and DCM (15 mL). The mixture was stirred at room temperature for 30 min, and sodium borohydride acetate (392.2 mg, 1.85 mmol) was added. The mixture was stirred at room temperature overnight, and TLC monitoring showed no starting material remained. The pH was adjusted to 9 with ammonia water, and the mixture was extracted with DCM (3 mL × 3). The organic phases were combined, concentrated and purified by column chromatography to give the target product **17** as a yellow solid (150 mg, 74.2% yield).
¹H NMR (400 MHz, CDCl₃) δ 8.45 (d, *J* = 2.2 Hz, 1H), 8.18 (s, 1H), 8.12 (d, *J* = 2.0 Hz, 1H), 7.98 (d, *J* = 2.1 Hz, 1H), 7.84 (dd, *J* = 8.8, 2.5 Hz, 1H), 7.67 (dd, *J* = 8.5, 2.4 Hz, 1H), 7.13 (d, *J* = 2.1 Hz, 1H), 6.77 - 6.68 (m, 2H), 4.74 (s, 3H), 3.94 (s, 3H), 3.90 - 3.78 (m, 6H), 3.69 - 3.55 (m, 6H), 3.41 (d, *J* = 10.7 Hz, 2H), 2.83 - 2.67 (m, 2H), 1.70 (dt, *J* = 14.4, 7.2 Hz, 2H).

### Example 3

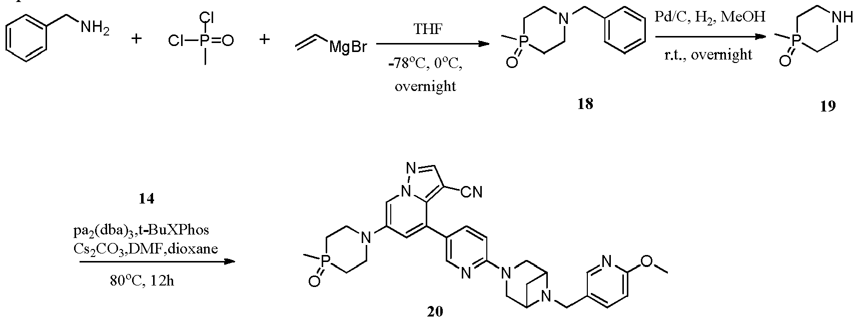

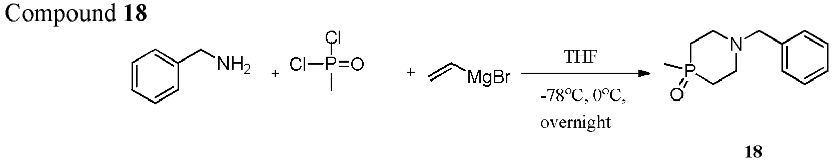

A 250 mL three-necked flask, an argon protective device and a low-temperature thermometer were prepared. To the flask were added 100 mL of THF and methylphosphonyl dichloride (5 g, 37.6 mmol). The temperature was reduced to -78 °C, and vinylmagnesium bromide (38 mL, 38 mmol) was slowly added dropwise over 30 min. The mixture was warmed to 0 °C and stirred for 1 h. A solution of benzylamine (4.8 g, 44.8 mmol) in methanol was added dropwise. The mixture was warmed to 68 °C and refluxed overnight, and TLC monitoring showed no starting material remained. The mixture was purified by column chromatography to give the target product **18** as a white solid (3.1 g, 36.9% yield).

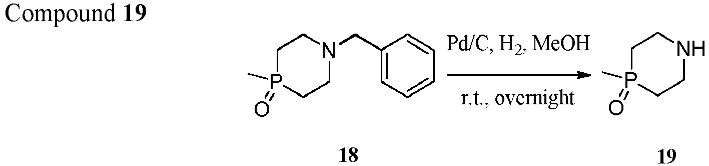

To a 100 mL single-neck flask were added successively **18** (3 g, 13.4 mmol), palladium on carbon (500 mg) and methanol (30 mL). The mixture was stirred at room temperature overnight under H2, and TLC monitoring showed no starting material remained. The mixture was filtered to remove waste palladium on carbon and concentrated to give the target product **19** (1.5 g, 83.85% yield). The product was directly used in the next step without purification.

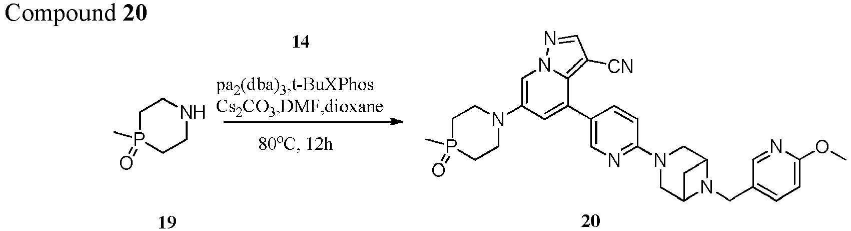

To a 25 mL sealed tube were added successively **14** (300 mg, 0.58 mmol), Pd₂(dba)₃ (64.1 mg, 0.07 mmol), *t*-BuXPhos (89.2 mg, 0.21 mmol), **19** (231.6 mg, 1.74 mmol), Cs₂CO₃ (378 mg, 1.16 mmol), dioxane (6 mL) and DMF (3 mL). The mixture was stirred at 80 °C overnight under Ar, and TLC monitoring showed no starting material **14** remained. The mixture was cooled to room temperature, and 10 mL of water was added. The mixture was stirred for 10 min, and a yellow solid precipitated. The solid was collected by filtration, dried and purified by column chromatography to give product **20** (230 mg, 70% yield).
¹H NMR (400 MHz, CDCl₃) δ 8.45 (d, *J* = 2.2 Hz, 1H), 8.18 (s, 1H), 8.12 (d, *J* = 2.0 Hz, 1H), 7.98 (d, *J* = 2.1 Hz, 1H), 7.84 (dd, *J* = 8.8, 2.5 Hz, 1H), 7.67 (dd, *J* = 8.5, 2.4 Hz, 1H), 7.13 (d, *J* = 2.1 Hz, 1H), 6.77 - 6.68 (m, 2H),δ 3.92 (s, 3H), 3.84 (m, 4H), 3.62 (m, 4H), 3.51 (m, 5H), 2.75 (m, 1H), 2.08 (m, 4H), 1.64 (m, 3H).

### Example 4

The synthesis scheme is as follows:

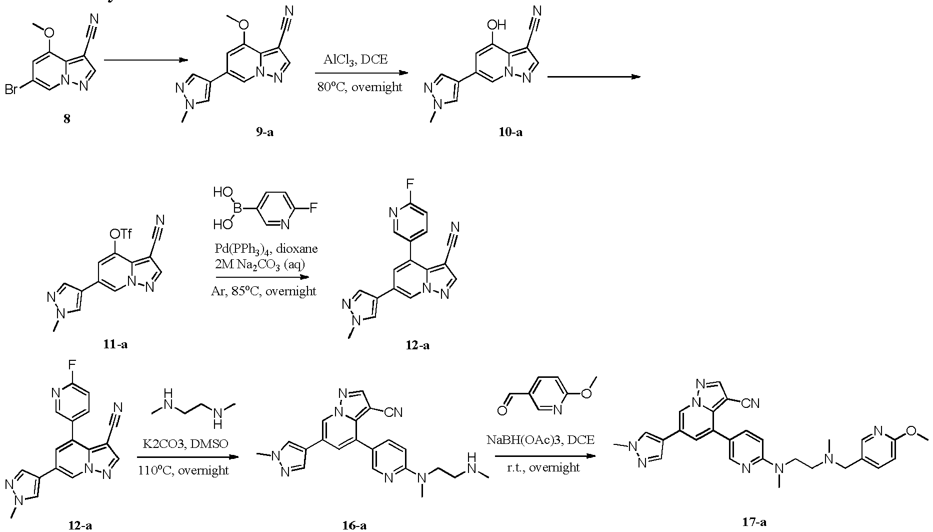

Compound **8** was synthesized as in Example 1.

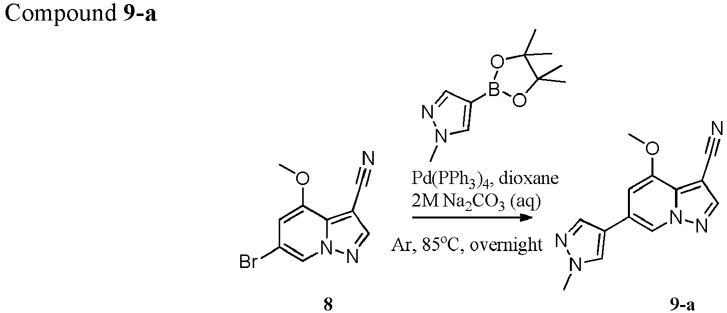

To a 25 mL sealed tube were added successively **8** (1 g, 3.97 mmol), Pd(PPh₃)₄ (229.3 mg, 0.2 mmol), 1-methyl-4-(4,4,5,5-tetramethyl-1,3,2-dioxaborolan-2-yl)-1*H*-pyrazole (991 mg, 4.76 mmol), 2 M Na₂CO₃ (1.26 g, 11.9 mmol) and 1,4-dioxane (8 mL). The mixture was stirred at 85 °C overnight under Ar, and TLC monitoring showed no starting material 8 remained. The mixture was cooled to room temperature, and 10 mL of water was added. The mixture was stirred for 10 min, and a yellow solid precipitated. The solid was collected by filtration and dried to give product **9-a** (850 mg, 85% yield).

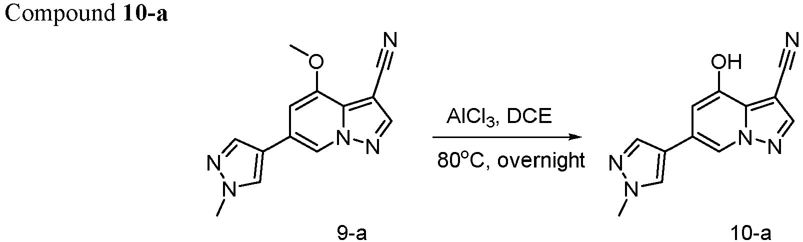

To a 45 mL sealed tube were added successively **9-a** (850 mg, 3.36 mmol) and 1,2-dichloroethane (15 mL), and AlCl₃ (1.57 g, 11.76 mmol) was added in batches at room temperature. The mixture was stirred at 80 °C overnight, and TLC monitoring showed no starting material **9-a** remained. The mixture was cooled to room temperature, quenched by adding Na₂SO₄·10H₂O, stirred for 1 h, filtered, washed with MeOH, concentrated to remove the solvent and purified by column chromatography to give product **10-a** as a gray solid (450 mg, 56% yield).

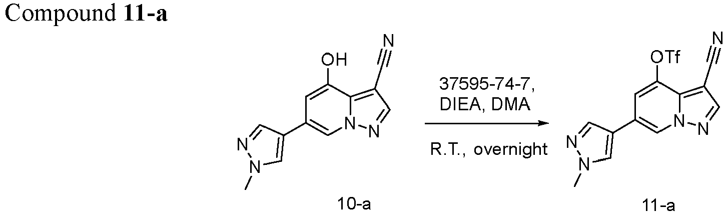

To a 50 mL single-neck flask were added successively compound **10-a** (450 mg, 1.88 mmol), *N*-phenylbis(trifluoromethanesulfonyl)imide (806 mg, 2.26 mmol), DIEA (486 mg, 3.76 mmol) and DMA (5 mL). The mixture was stirred at room temperature overnight, and TLC monitoring showed no starting material **10-a** remained. The reaction mixture was poured into 10 mL of H₂O being stirred, and a brown solid precipitated. The solid was collected by filtration and dried to give product **11-a** as a brown solid (628 mg, 90% yield).

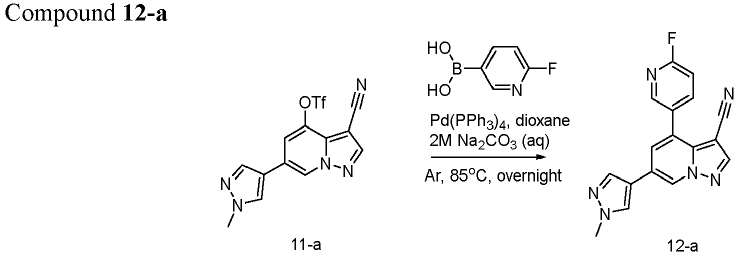

To a 12 mL sealed tube were added successively **11-a** (628 mg, 1.69 mmol), Pd(PPh₃)₄ (97.6 mg, 0.08 mmol), 2-fluoropyridine-5-boronic acid (238.3 mg, 1.69 mmol), 2 M Na₂CO₃ (358.3 mg, 3.38 mmol) and 1,4-dioxane (5 mL). The mixture was stirred at 85 °C overnight under Ar, and TLC monitoring showed no starting material **11-a** remained. The mixture was cooled to room temperature, and 10 mL of water was added. The mixture was stirred for 10 min, and a brownish-yellow solid precipitated. The solid was collected by filtration and dried to give product **12-a** (430 mg, 80% yield).

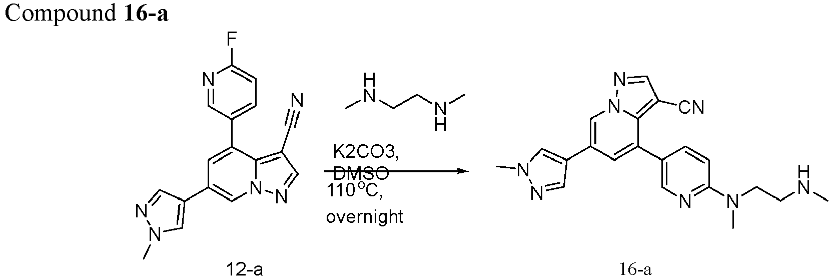

To a 12 mL sealed tube were added successively **12-a** (70 mg, 0.22 mmol), *N,N'-*dimethylethylenediamine (23.2 mg, 0.26 mmol), K₂CO₃ (61 mg, 0.44 mmol) and DMSO (1 mL). The mixture was stirred at 120 °C overnight, and TLC monitoring showed no starting material **12-a** remained. The mixture was cooled to room temperature, and 10 mL of water was added. The mixture was stirred for 10 min, and a yellow solid precipitated. The solid was collected by filtration and dried to give product **16-a** (51 mg, 60% yield).
LC-MS [M+H]⁺387.2.

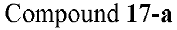

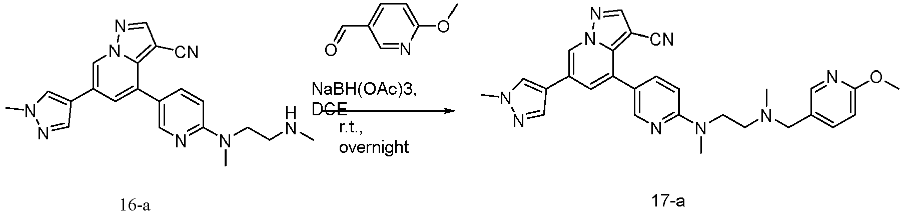

To a 50 mL single-neck flask were added successively **16-a** (51 mg, 0.13 mmol), 6-methoxy-3-pyridinecarboxaldehyde (26.7 mg, 0.19 mmol) and DCM (15 mL). After 10 min of stirring, NaBH(OAc)₃ (137.8 mg, 0.65 mmol) was added in batches at room temperature. The mixture was stirred at room temperature overnight, and TLC monitoring showed no starting material **16-a** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **17-a** as a yellow solid (30 mg, 46% yield).
1H NMR (400 MHz, CDCl₃) δ 8.62 (s, 1H), 8.31 (s, 1H), 8.25 (s, 1H), 8.02 (s, 1H), 7.80 (s, 1H), 7.70 (s, 1H), 7.68 (s, 1H), 7.54 (d, J = 6.6 Hz, 1H), 7.39 (s, 1H), 6.64 (dd, J = 36.1, 8.7 Hz, 2H), 3.99 (s, 3H), 3.89 (s, 3H), 3.79 (t, J = 6.7 Hz, 2H), 3.53 (s, 2H), 3.11 (s, 3H), 2.67 (t, J = 6.6 Hz, 2H), 2.30 (s, 3H). LC-MS [M+H]⁺507.6.

### Example 5

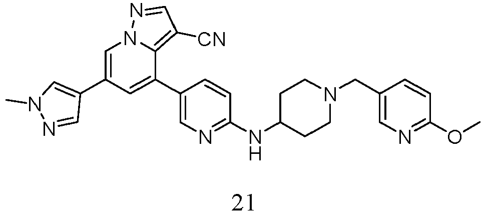

The synthesis scheme is as follows:

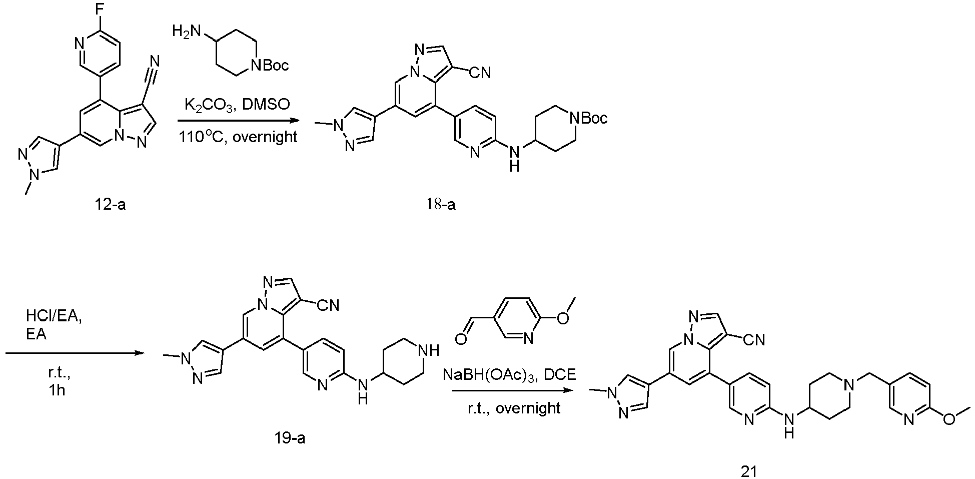

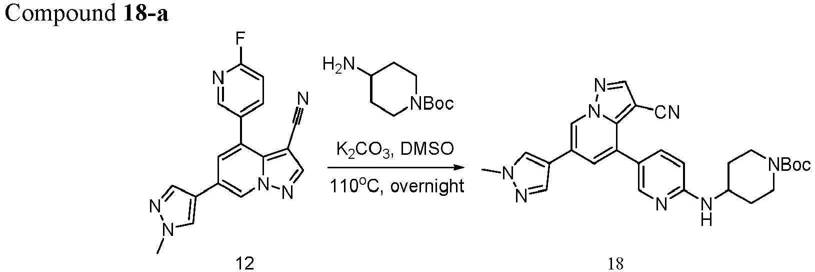

To a 12 mL sealed tube were added successively **12-a** (80 mg, 0.25 mmol), 1-Boc-4-aminopiperidine (60.5 mg, 0.3 mmol), K₂CO₃ (69 mg, 0.5 mmol) and DMSO (1 mL). The mixture was stirred at 120 °C overnight, and TLC monitoring showed no starting material **12-a** remained. The mixture was cooled to room temperature, and 10 mL of water was added. The mixture was stirred for 10 min, and a yellow solid precipitated. The solid was collected by filtration and dried to give product **18-a** (70 mg, 56% yield).
LC-MS [M+H]⁺499.25.

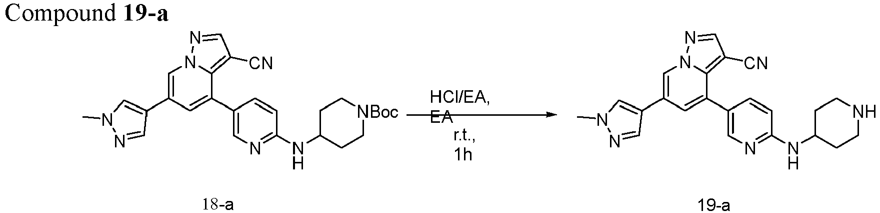

To a 50 mL single-neck flask was added a 3.5 M solution of HCl in EA (10 mL), and a solution of **18-a** (70 mg, 0.14 mmol) in EA was slowly added dropwise. After the addition was completed, the mixture was stirred at room temperature for another 3 h, concentrated to remove the solvent, neutralized by adding dropwise a solution of NH₃ in methanol and concentrated to give product **19-a** as a yellow solid (55 mg, 98% yield).
LC-MS [M+H]⁺399.25.

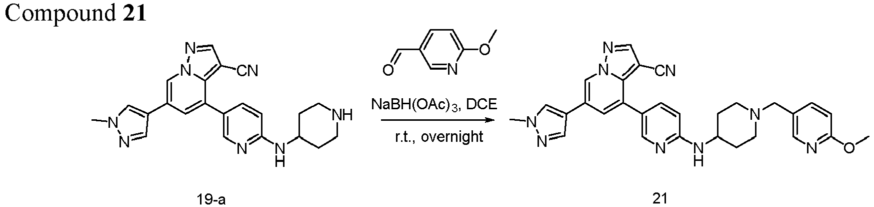

To a 50 mL single-neck flask were added successively **19-a** (55 mg, 0.138 mmol), 6-methoxy-3-pyridinecarboxaldehyde (22.7 mg, 0.166 mmol) and DCM (15 mL). After 10 min of stirring, NaBH(OAc)₃ (146.28 mg, 0.69 mmol) was added in batches at room temperature. The mixture was stirred at room temperature overnight, and TLC monitoring showed no starting material **19-a** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **21** as a yellow solid (30 mg, 41.8% yield).
1H NMR (400 MHz, CDCl₃) δ 8.63 (s, 1H), 8.29 - 8.20 (m, 2H), 8.06 (s, 1H), 7.79 (s, 1H), 7.64 (ddd, J = 19.4, 7.1, 2.8 Hz, 3H), 7.38 (s, 1H), 6.74 (d, J = 8.4 Hz, 1H), 6.52 (d, J = 8.6 Hz, 1H), 4.78 (d, J = 7.7 Hz, 1H), 4.00 (s, 3H), 3.94 (s, 3H), 3.77 (s, 1H), 3.54 (s, 2H), 2.90 (s, 2H), 2.29 (d, J = 10.2 Hz, 2H), 2.10 (d, J = 15.2 Hz, 2H), 1.61 (d, J = 10.2 Hz, 2H). LC-MS [M+H]+ 519.6.

### Example 6

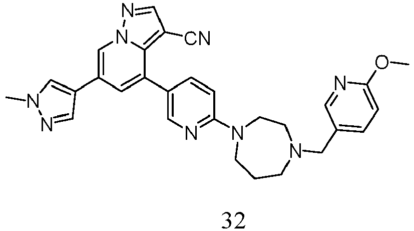

The synthesis scheme is as follows:

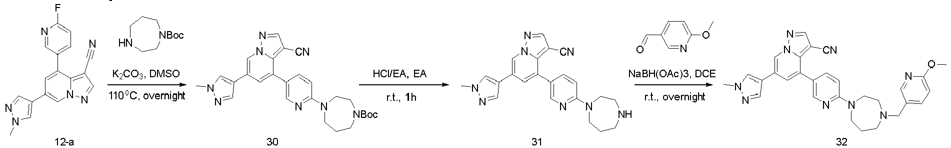

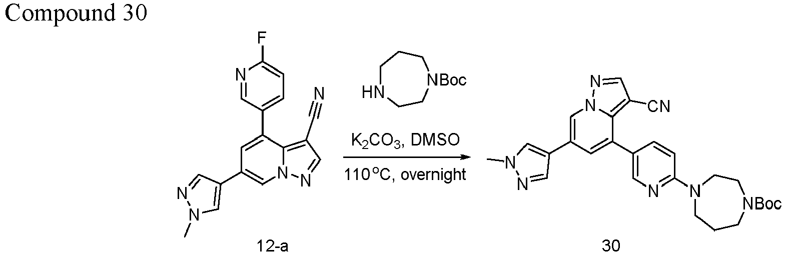

To a 12 mL sealed tube were added successively **12-a** (70 mg, 0.22 mmol), *tert*-butyl 1,4-diazepan-1-carboxylate (52.1 mg, 0.26 mmol), K₂CO₃ (60.8 mg, 0.44 mmol) and DMSO (3 mL). The mixture was stirred at 120 °C overnight, and TLC monitoring showed no starting material **12-a** remained. The mixture was cooled to room temperature, and 5 mL of water was added. The mixture was stirred for 10 min, and a yellow solid precipitated. The solid was collected by filtration and dried to give product 30 (55 mg, 50% yield).
LC-MS [M+H]⁺499.25.

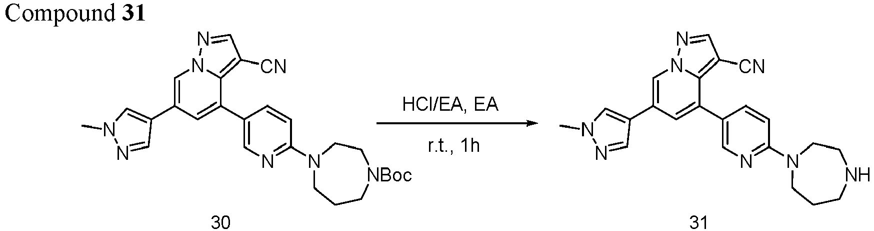

To a 50 mL single-neck flask was added a 3.5 M solution of HCl in EA (5 mL), and a solution of **30** (55 mg, 0.11 mmol) in EA was slowly added dropwise. After the addition was completed, the mixture was stirred at room temperature for another 3 h, concentrated to remove the solvent, neutralized by adding dropwise a solution of NH₃ in methanol and concentrated to give product 31 as a yellow solid (44.6 mg, 100% yield).
LC-MS [M+H]⁺399.25.

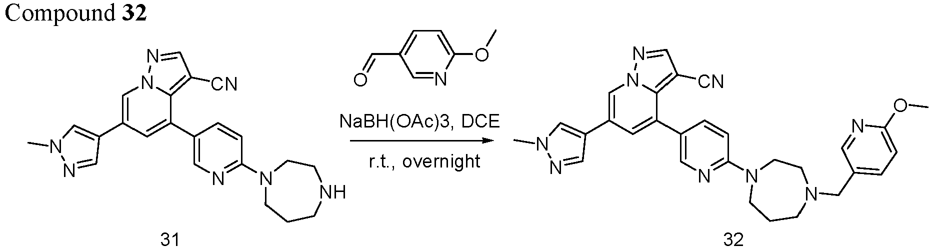

To a 50 mL single-neck flask were added successively **31** (44.6 mg, 0.11 mmol), 6-methoxy-3-pyridinecarboxaldehyde (18.1 mg, 0.13 mmol) and DCM (5 mL). After 10 min of stirring, NaBH(OAc)₃ (93.3 mg, 0.44 mmol) was added in batches at room temperature. The mixture was stirred at room temperature overnight, and TLC monitoring showed no starting material **31** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **32** as a yellow solid (32 mg, 55% yield).
1H NMR (400 MHz, CDCl₃) δ 8.62 (d, J = 1.4 Hz, 1H), 8.34 (d, J = 2.4 Hz, 1H), 8.25 (s, 1H), 8.05 (d, J = 1.8 Hz, 1H), 7.79 (s, 1H), 7.71 (dd, J = 8.9, 2.5 Hz, 1H), 7.68 (s, 1H), 7.63 - 7.57 (m, 1H), 7.39 (d, J = 1.4 Hz, 1H), 6.72 (d, J = 8.5 Hz, 1H), 6.63 (d, J = 8.9 Hz, 1H), 3.99 (s, 3H), 3.93 (s, 3H), 3.85 (m, 2H), 3.73 (t, J = 6.1 Hz, 2H), 3.58 (s, 2H), 2.79 (s, 2H), 2.64 (d, J = 5.1 Hz, 2H), 1.98 (s, 2H), 1.68 (s, 2H). LC-MS [M+H]⁺519.6.

### Example 7

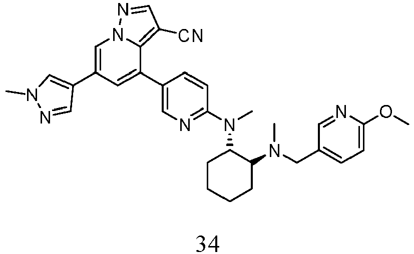

The synthesis scheme is as follows:

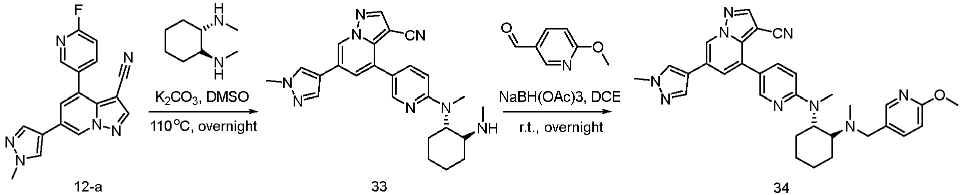

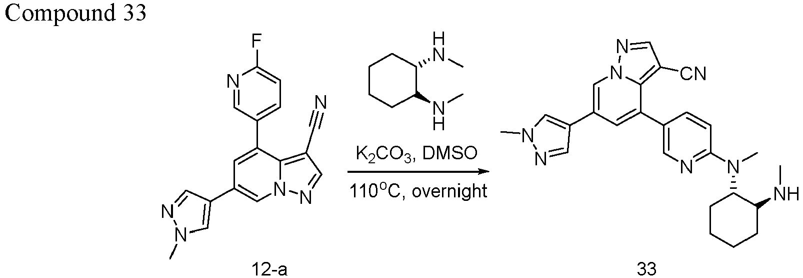

To a 12 mL sealed tube were added successively **12-a** (70 mg, 0.22 mmol), (1*S*,2*S*)-(+)-*N,N*'-dimethyl-1,2-cyclohexanediamine (31.3 mg, 0.22 mmol), K₂CO₃ (60.8 mg, 0.44 mmol) and DMSO (3 mL). The mixture was stirred at 120 °C overnight, and TLC monitoring showed no starting material **12-a** remained. The mixture was cooled to room temperature, and 5 mL of water was added. The mixture was stirred for 10 min, and a yellow solid precipitated. The solid was purified by column chromatography to give product **33** (43.6 mg, 45% yield).
LC-MS [M+H]⁺441.24.

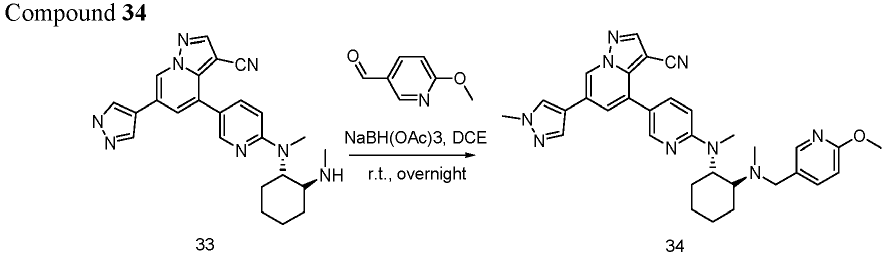

To a 50 mL single-neck flask were added successively **33** (43.6 mg, 0.1 mmol), 6-methoxy-3-pyridinecarboxaldehyde (16.7 mg, 0.12 mmol) and DCM (5 mL). After 10 min of stirring, NaBH(OAc)₃ (84.8 mg, 0.4 mmol) was added in batches at room temperature. The mixture was stirred at room temperature overnight, and TLC monitoring showed no starting material **33** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **34** as a yellow solid (30 mg, 53% yield).
1H NMR (400 MHz, CDCl₃) δ 8.59 (s, 1H), 8.24 (s, 1H), 8.21 (s, 1H), 7.84 (s, 1H), 7.78 (s, 1H), 7.73 (d, J = 9.2 Hz, 1H), 7.67 (s, 1H), 7.25 - 7.21 (m, 1H), 7.15 (s, 1H), 6.64 (d, J = 8.6 Hz, 1H), 6.58 (d, J = 8.2 Hz, 1H), 3.99 (s, 3H), 3.83 (s, 3H), 3.73 (d, J = 12.9 Hz, 1H), 3.25 (d, J = 13.0 Hz, 1H), 2.89 (s, 3H), 2.70 (s, 1H), 2.10 (s, 3H), 2.04 (s, 1H), 1.92 - 1.76 (m, 3H), 1.44 (dd, J = 38.9, 14.7 Hz, 3H). LC-MS [M+H]⁺561.7.

### Example 8

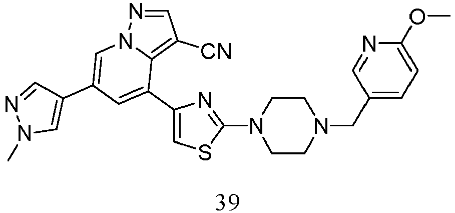

The synthesis scheme is as follows:

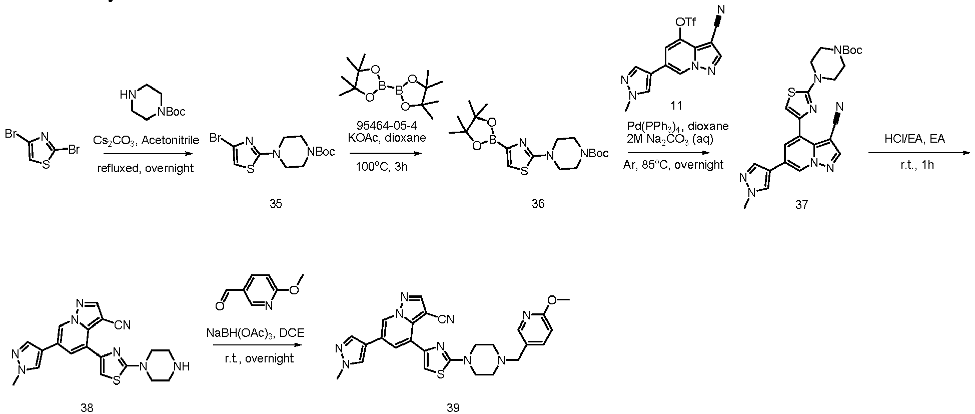

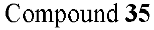

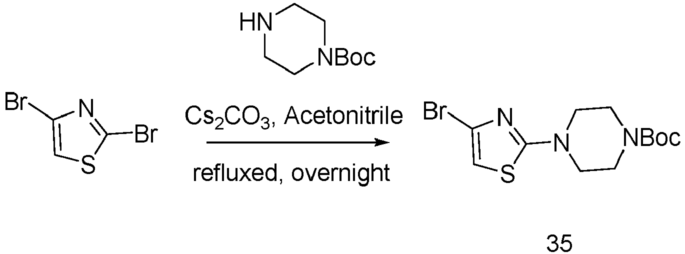

To a 50 mL single-neck flask were added successively the compounds 2,4-dibromothiazole (1.5 g, 6.17 mmol), 1-A-BOC-piperazine (1.7 g, 9.26 mmol), Cs₂CO₃ (4.0 g, 12.3 mmol) and acetonitrile (20 mL). The mixture was heated at reflux overnight, and TLC monitoring showed no starting material remained. The mixture was diluted by adding H2O (50 mL) and extracted with EA (20 mL × 3). The organic phases were combined, concentrated and purified by column chromatography to give product **35** as a white solid (1.6 g, 74.5% yield).
LC-MS [M+H]⁺348.03.

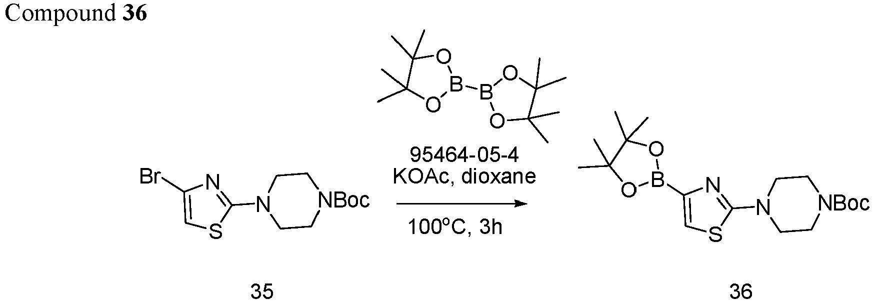

To a 45 mL sealed tube were added successively **35** (1.2 g, 3.45 mmol), bis(pinacolato)diboron (918.7 mg, 3.62 mmol), Pd(dppf)Cl₂·DCM (140.9 mg, 0.17 mmol), KOAc (1.02 g, 10.35 mmol) and 1,4-dioxane (15 mL). The mixture was stirred at 100 °C for 3 h under Ar, and TLC monitoring showed no starting material 35 remained. The mixture was cooled to room temperature, and 10 mL of water was added. The mixture was stirred for 10 min, and a reddish-brown solid precipitated. The solid was collected by filtration and dried to give product **36** (1.26 g, 92.38% yield).
LC-MS [M+H]⁺396.21. Compound **37**

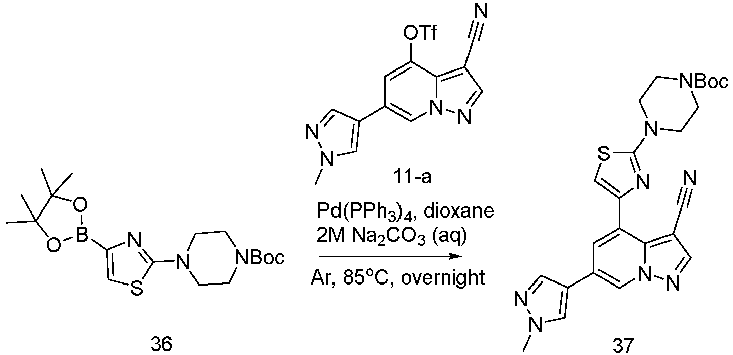

To a 45 mL sealed tube were added successively compound **36** (1.25 g, 3.18 mmol), **11-a** (1.0 g, 2.69 mmol), Pd(PPh₃)₄ (155 mg, 0.13 mmol), 4 mL of 2 M Na₂CO₃ (855.4 mg, 8.07 mmol) and 1,4-dioxane (20 mL). The mixture was stirred at 85 °C overnight under Ar, and TLC monitoring showed no starting material **11-a** remained. The mixture was cooled to room temperature, and 10 mL of water was added. The mixture was stirred for 10 min, and a solid precipitated. The solid was collected by filtration and dried to give product **37** as a gray solid (1.2 g, 91% yield).
LC-MS [M+H]⁺491.19.

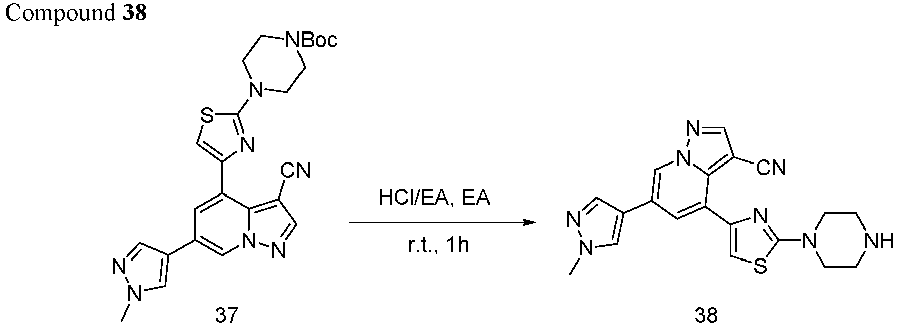

To a 25 mL single-neck flask was added a 3.5 M solution of HCl in EA (10 mL), and a solution of **37** (1.2 g, 2.45 mmol) in EA was slowly added dropwise. After the addition was completed, the mixture was stirred at room temperature for another 3 h, concentrated to remove the solvent, neutralized by adding dropwise a solution of NH₃ in methanol, concentrated and purified by column chromatography to give product **38** as a brownish-yellow solid (680 mg, 71.1% yield).
LC-MS [M+H]⁺492.19.

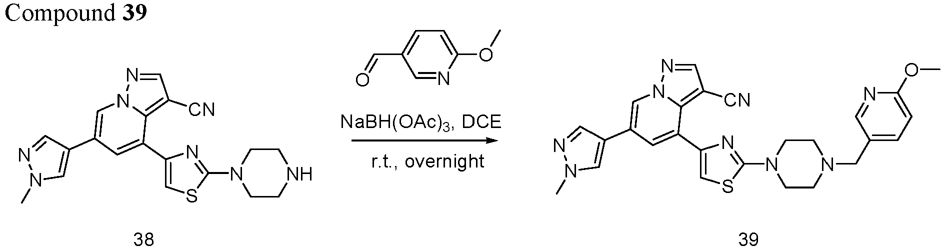

To a 50 mL single-neck flask were added successively **38** (200 mg, 0.51 mmol), 6-methoxy-3-pyridinecarboxaldehyde (70.2 mg, 0.51 mmol) and DCM (10 mL). After 10 min of stirring, NaBH(OAc)₃ (324.3 mg, 1.53 mmol) was added in batches at room temperature. The mixture was stirred at room temperature overnight, and TLC monitoring showed no starting material 38 remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **39** as a yellow solid (170 mg, 65% yield).
¹H NMR (400 MHz, CDCl₃) δ 8.61 (d, J = 1.4 Hz, 1H), 8.26 (s, 1H), 8.07 (d, J = 2.0 Hz, 1H), 7.79 (s, 1H), 7.76 (d, J = 1.4 Hz, 1H), 7.69 (s, 1H), 7.60 (dd, J = 8.5, 2.2 Hz, 1H), 6.95 (s, 1H), 6.74 (d, J = 8.5 Hz, 1H), 3.98 (s, 3H), 3.94 (s, 3H), 3.66 - 3.57 (m, 4H), 3.51 (s, 2H), 2.65 - 2.57 (m, 4H). LC-MS [M+H]+ 511.6.

### Example 9

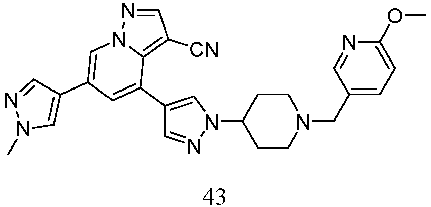

The synthesis scheme is as follows:

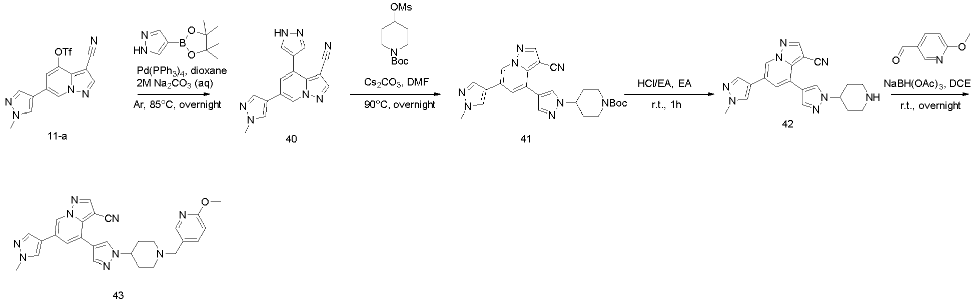

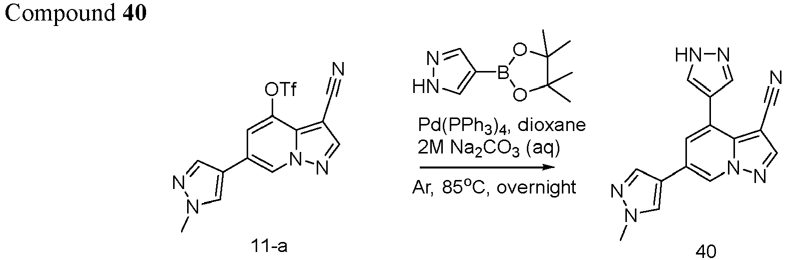

To a 45 mL sealed tube were added successively compound **11-a** (200 mg, 0.54 mmol), 4-pyrazoleboronic acid pinacol ester (104.5 mg, 0.54 mmol), Pd(PPh₃)₄ (31.2 mg, 0.07 mmol), 0.54 mL of 2 M Na₂CO₃ (114.5 mg, 1.08 mmol) and 1,4-dioxane (2 mL). The mixture was stirred at 85 °C overnight under Ar, and TLC monitoring showed no starting material **11-a** remained. The mixture was cooled to room temperature, and 5 mL of water was added. The mixture was stirred for 10 min, and a solid precipitated. The solid was collected by filtration and dried to give product **40** as a gray solid (115.7 mg, 74% yield).
LC-MS [M+H]⁺290.11.

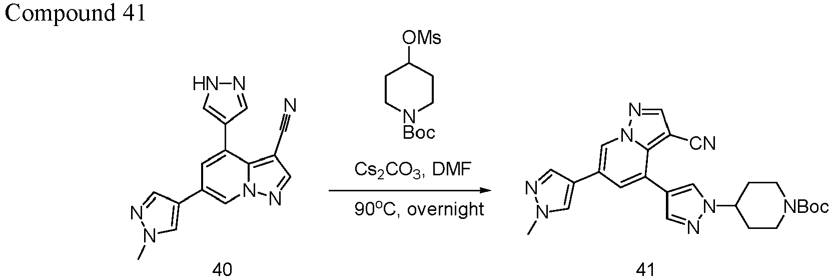

To a 50 mL single-neck flask were added successively compound **40** (115.7 mg, 0.4 mmol), 1-Boc-4-methanesulfonyloxypiperidine (167.6 mg, 0.6 mmol), Cs₂CO₃ (260.7 mg, 0.8 mmol) and DMF (2 mL). The mixture was heated at reflux overnight, and TLC monitoring showed no starting material 40 remained. The mixture was diluted by adding H2O (6 mL) and extracted with EA (2 mL × 3). The organic phases were combined, concentrated and purified by column chromatography to give product **41** as a yellow solid (94.5 mg, 50% yield).
LC-MS [M+H]⁺473.23.

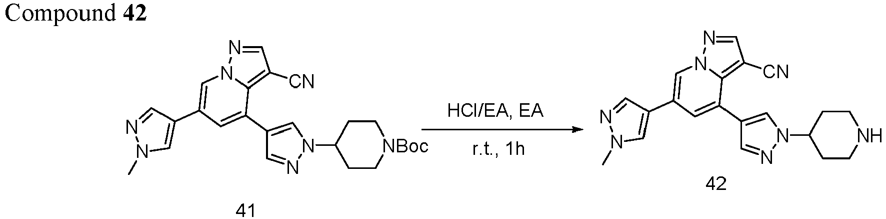

To a 25 mL single-neck flask was added a 3.5 M solution of HCl in EA (10 mL), and a solution of **41** (94.5 mg, 0.2 mmol) in EA was slowly added dropwise. After the addition was completed, the mixture was stirred at room temperature for another 3 h, concentrated to remove the solvent, neutralized by adding dropwise a solution of NH₃ in methanol, concentrated and purified by column chromatography to give product **42** as a brownish-yellow solid (68 mg, 90% yield).

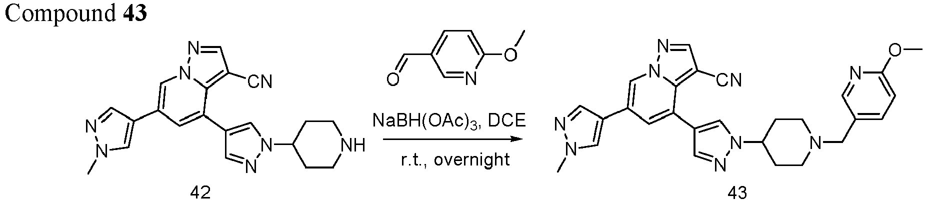

To a 50 mL single-neck flask were added successively **42** (68 mg, 0.18 mmol), 6-methoxy-3-pyridinecarboxaldehyde (30.2 mg, 0.22 mmol) and DCM (10 mL). After 10 min of stirring, NaBH(OAc)₃ (190.8 mg, 0.9 mmol) was added in batches at room temperature. The mixture was stirred at room temperature overnight, and TLC monitoring showed no starting material **42** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **43** as a yellow solid (55 mg, 61% yield).
¹H NMR (400 MHz, CDCl₃) δ 8.59 (s, 1H), 8.25 (s, 1H), 8.06 (s, 1H), 7.95 (s, 1H), 7.79 (s, 2H), 7.70 (s, 1H), 7.62 (dd, J = 8.4, 1.9 Hz, 1H), 7.47 (s, 1H), 6.73 (d, J = 8.4 Hz, 1H), 4.24 (d, J = 10.7 Hz, 1H), 3.99 (s, 3H), 3.93 (s, 3H), 3.50 (s, 2H), 2.19 (dt, J = 22.9, 10.5 Hz, 8H). LC-MS [M+H]+ 493.56.

### Example 10

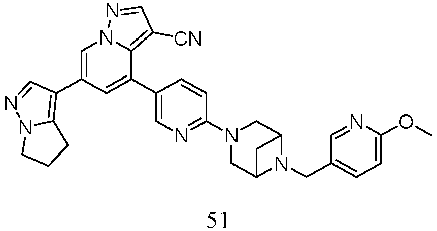

The synthesis scheme is as follows:

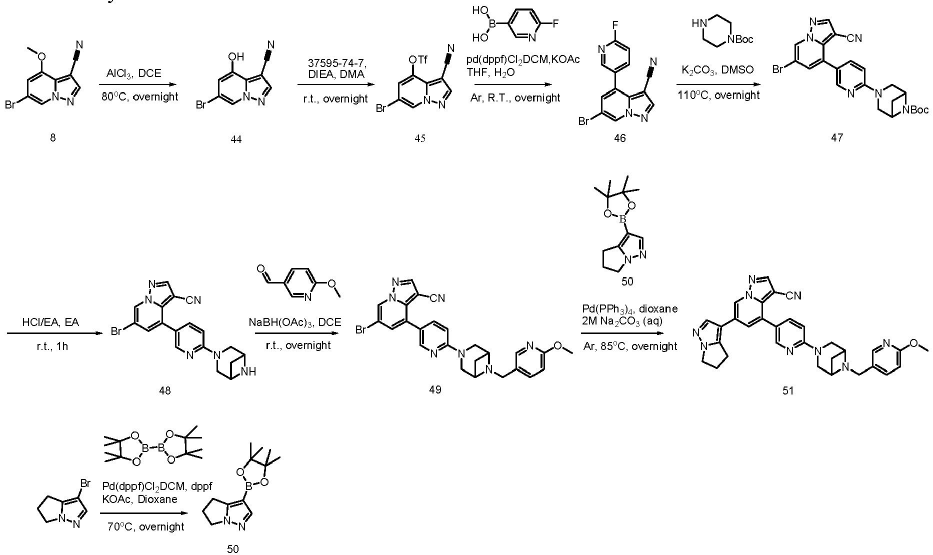

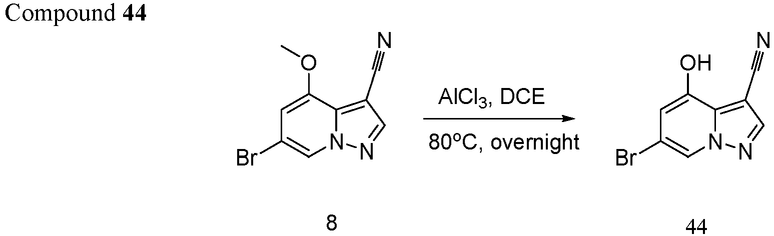

To a 45 mL sealed tube were added successively **8** (5 g, 19.8 mmol) and 1,2-dichloroethane (100 mL), and AlCl₃ (9.34 mg, 70 mmol) was added in batches at room temperature. The mixture was stirred at 80 °C overnight, and TLC monitoring showed no starting material 8 remained. The mixture was cooled to room temperature, quenched by adding Na₂SO₄·10H₂O, stirred for 1 h, filtered, washed with MeOH, concentrated to remove the solvent and purified by column chromatography to give product **44** as a gray solid (3 g, 55% yield).

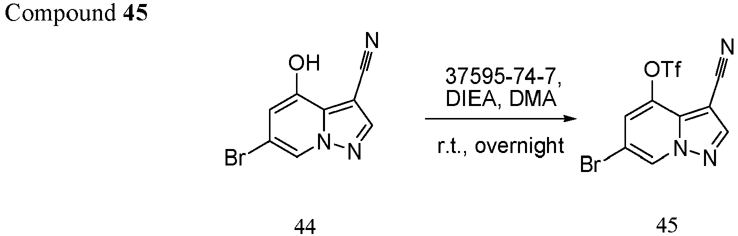

To a 50 mL single-neck flask were added successively compound **44** (3 g, 12.6 mmol), N-phenylbis(trifluoromethanesulfonyl)imide (4.5 g, 12.6 mmol), DIEA (3.26 g, 25.2 mmol) and DMA (20 mL). The mixture was stirred at room temperature overnight, and TLC monitoring showed no starting material **44** remained. The reaction mixture was poured into 60 mL of H₂O being stirred, and a brown solid precipitated. The solid was collected by filtration and dried to give product **45** as a brown solid (4.4 g, 95% yield).

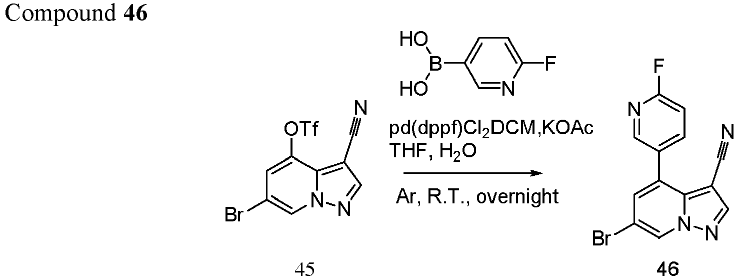

To a 12 mL sealed tube were added successively **45** (4.4 g, 11.9 mmol), Pd(dppf)Cl₂ complexed with dichloromethane (490 mg, 0.6 mmol), 2-fluoropyridine-5-boronic acid (1.68 g, 11.9 mmol), KOAc (2.92 g, 29.75 mmol) and 1,4-dioxane (50 mL). The mixture was stirred at 85 °C overnight under Ar, and TLC monitoring showed no starting material **45** remained. The mixture was cooled to room temperature, and 100 mL of water was added. The mixture was stirred for 10 min, and a brownish-yellow solid precipitated. The solid was collected by filtration and dried to give product **46** (3 g, 80% yield).

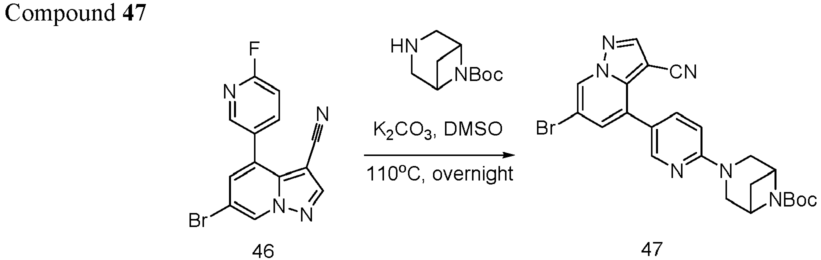

To a 45 mL sealed tube were added successively **46** (3 g, 9.46 mmol), *6-(tert-*butoxycarbonyl)-3,6-diazabicyclo[3.1.1]heptane (2.25 g, 11.3 mmol), K₂CO₃ (3.9 g, 28.35 mmol) and DMSO (20 mL). The mixture was stirred at 110 °C overnight, and TLC monitoring showed no starting material **46** remained. The mixture was cooled to room temperature, and 50 mL of water was added. The mixture was stirred for 10 min, and a yellow solid precipitated. The solid was collected by filtration and dried to give product **47** (3.2 g, 69% yield).

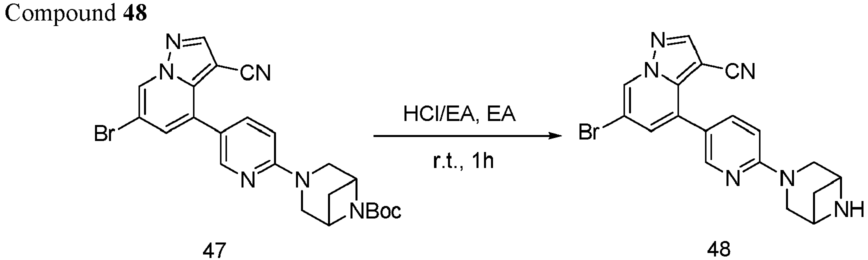

To a 25 mL single-neck flask was added a 3.5 M solution of HCl in EA (10 mL), and a solution of **47** (94.5 mg, 0.2 mmol) in EA was slowly added dropwise. After the addition was completed, the mixture was stirred at room temperature for another 1 h, concentrated to remove the solvent, neutralized by adding dropwise a solution of NH₃ in methanol, concentrated and purified by column chromatography to give product **48** as a brownish-yellow solid (68 mg, 90% yield).

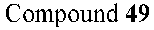

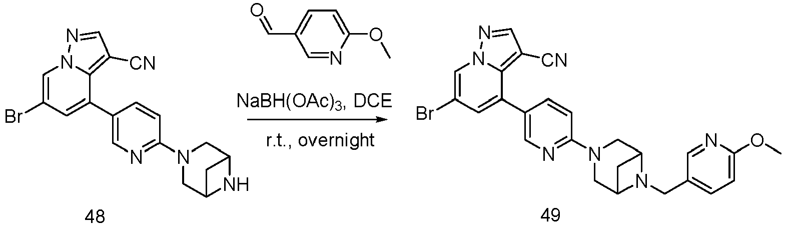

To a 50 mL single-neck flask were added successively **48** (68 mg, 0.18 mmol), 6-methoxy-3-pyridinecarboxaldehyde (30.2 mg, 0.22 mmol) and DCM (10 mL). After 10 min of stirring, NaBH(OAc)₃ (190.8 mg, 0.9 mmol) was added in batches at room temperature. The mixture was stirred at room temperature overnight, and TLC monitoring showed no starting material 48 remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **49** as a yellow solid (55 mg, 61% yield).

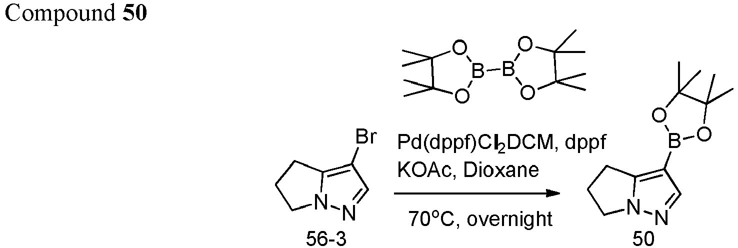

To a 25 mL sealed tube were added successively 3-bromo-5,6-dihydro-4*H*-pyrrolo[1,2-B]pyrazole (1.45 g, 7.8 mmol), bis(pinacolato)diboron (2.1 g, 8.2 mmol), Pd(dppf)Cl₂ DCM (318.2 mg, 0.4 mmol), 1,1'-bis(diphenylphosphino)ferrocene (221.8 mg, 0.4 mmol), KOAc (2.3 g, 23.4 mmol) and 1,4-dioxane (40 mL). The mixture was stirred at 70 °C overnight under Ar, and TLC monitoring showed no starting material **56-3** remained. The mixture was cooled to room temperature, and 60 mL of water was added. The mixture was stirred for 10 min and extracted with DCM (20 mL × 3). The organic phases were combined, dried over anhydrous sodium sulfate and filtered. The filtrate was concentrated to give a product as a reddish-brown liquid (1.5 g, 82% yield), which was directly used in the next step without purification.
LC-MS [M+H]⁺235.15.

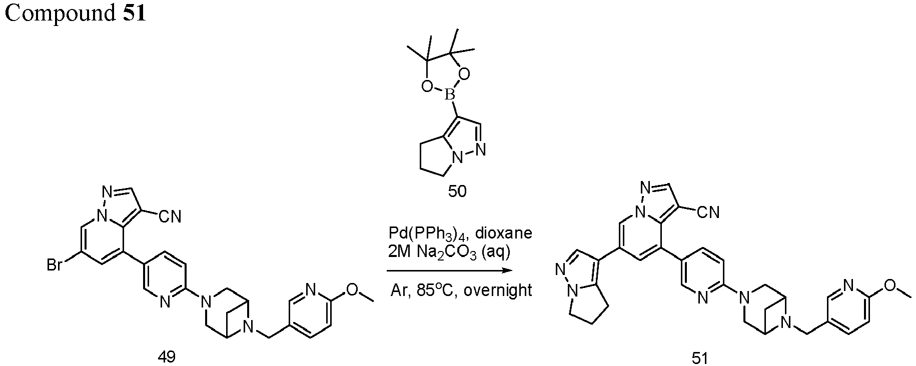

To a 25 mL sealed tube were added successively **49** (55 mg, 0.11 mmol), Pd(PPh₃)₄ (6.36 mg, 0.005 mmol), 50 (25.74 mg, 0.11 mmol), 2 M Na₂CO₃ (23.3 mg, 0.22 mmol) and 1,4-dioxane (5 mL). The mixture was stirred at 85 °C overnight under Ar, and TLC monitoring showed no starting material 49 remained. The mixture was cooled to room temperature, and 10 mL of water was added. The mixture was stirred for 10 min, and a yellow solid precipitated. The solid was collected by filtration, dried and purified by column chromatography to give product **51** (47.8 mg, 80% yield).
¹H NMR (400 MHz, CDCl₃) δ 8.56 (d, J = 1.3 Hz, 1H), 8.36 (d, J = 2.3 Hz, 1H), 8.25 (s, 1H), 8.08 (d, J = 1.9 Hz, 1H), 8.04 (d, J = 1.9 Hz, 1H), 8.02 (s, 1H), 7.82 (s, 1H), 7.75 (dd, J = 8.8, 2.5 Hz, 1H), 7.63 (dd, J = 8.4, 2.2 Hz, 1H), 7.40 (d, J = 1.4 Hz, 1H), 4.23 (t, J = 7.3 Hz, 2H), 3.92 (s, 3H), 3.84 (d, J = 11.9 Hz, 2H), 3.77 (d, J = 5.6 Hz, 2H), 3.62 (s, 2H), 3.58 (s, 2H), 2.97 (t, J = 7.3 Hz, 2H), 2.67 (dd, J = 14.3, 7.2 Hz, 3H), 1.67 (d, J = 8.6 Hz, 1H). LC-MS [M+H]+ 544.6

### Example 11

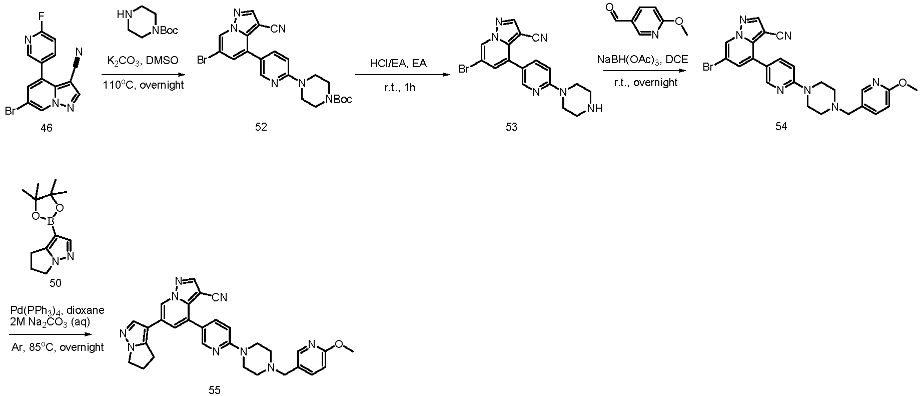

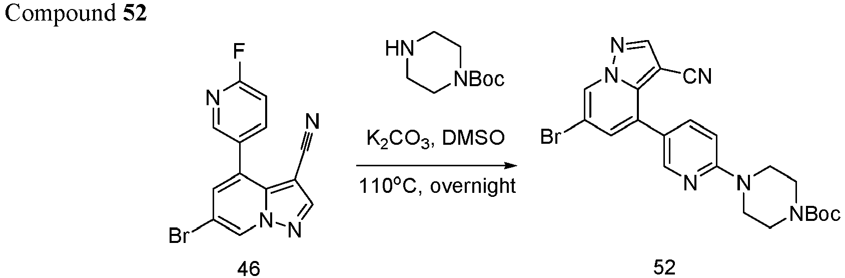

To a 45 mL sealed tube were added successively **46** (500 mg, 1.58 mmol), N BOC-piperazine (352.2 mg, 1.89 mmol), K₂CO₃ (436.1 mg, 3.16 mmol) and DMSO (20 mL). The mixture was stirred at 120 °C overnight, and TLC monitoring showed no starting material 46 remained. The mixture was cooled to room temperature, and 50 mL of water was added. The mixture was stirred for 10 min, and a yellow solid precipitated. The solid was collected by filtration and dried to give product **52** (611 mg, 80% yield).

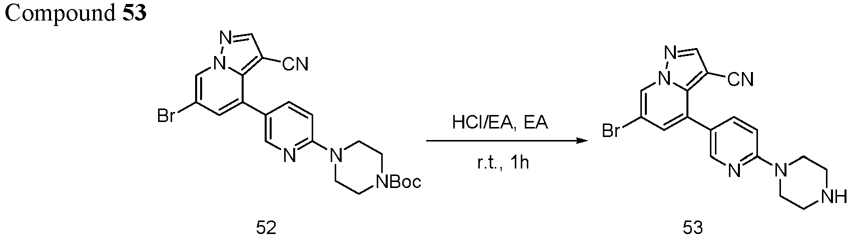

To a 25 mL single-neck flask was added a 3.5 M solution of HCl in EA (10 mL), and a solution of **52** (611 mg, 1.26 mmol) in EA was slowly added dropwise. After the addition was completed, the mixture was stirred at room temperature for another 1 h, concentrated to remove the solvent, neutralized by adding dropwise a solution of NH₃ in methanol, concentrated and purified by column chromatography to give product **53** as a brownish-yellow solid (473.2 mg, 98% yield).

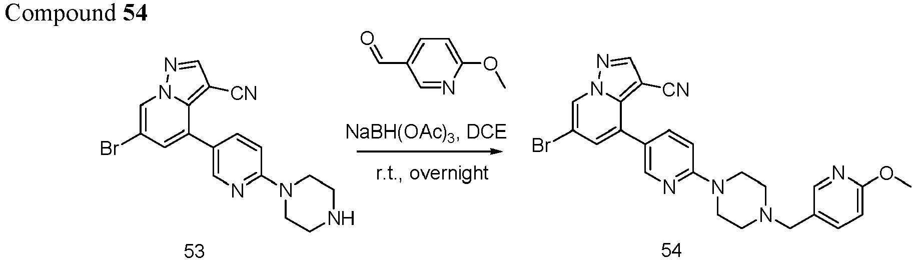

To a 50 mL single-neck flask were added successively 53 (473.2 mg, 1.23 mmol), 6-methoxy-3-pyridinecarboxaldehyde (203.2 mg, 1.48 mmol) and DCM (10 mL). After 10 min of stirring, NaBH(OAc)₃ (1.3 g, 6.15 mmol) was added in batches at room temperature. The mixture was stirred at room temperature overnight, and TLC monitoring showed no starting material 53 remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **54** as a yellow solid (55 mg, 61% yield).

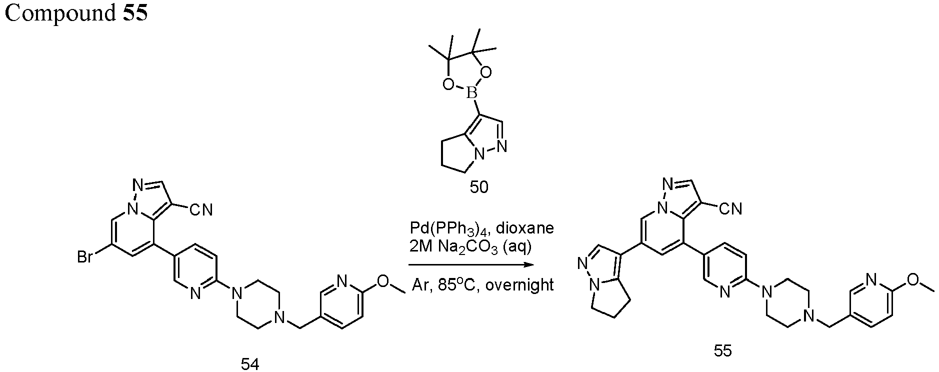

To a 25 mL sealed tube were added successively **54** (50 mg, 0.1 mmol), Pd(PPh₃)₄ (6.36 mg, 0.005 mmol), 50 (23.41 mg, 0.1 mmol), 2 M Na₂CO₃ (21.2 mg, 0.2 mmol) and 1,4-dioxane (5 mL). The mixture was stirred at 85 °C overnight under Ar, and TLC monitoring showed no starting material 54 remained. The mixture was cooled to room temperature, and 10 mL of water was added. The mixture was stirred for 10 min, and a yellow solid precipitated. The solid was collected by filtration, dried and purified by column chromatography to give product **55** (60.4 mg, 88% yield).
¹H NMR (400 MHz, CDCl₃) δ 8.56 (d, J = 1.3 Hz, 1H), 8.36 (d, J = 2.3 Hz, 1H), 8.25 (s, 1H), 8.08 (d, J = 1.9 Hz, 1H), 8.04 (d, J = 1.9 Hz, 1H), 8.02 (s, 1H), 7.82 (s, 1H), 7.75 (dd, J = 8.8, 2.5 Hz, 1H), 7.63 (dd, J = 8.4, 2.2 Hz, 1H), 7.40 (d, J = 1.4 Hz, 1H), 4.23 (t, J = 7.3 Hz, 2H), 3.95 (s, 3H), 3.93 (s, 3H), 3.70 - 3.59 (m, 4H), 3.50 (s, 2H), 3.15 - 3.08 (m, 2H), 2.79 - 2.69 (m, 2H), 2.60 - 2.51 (m, 4H). LC-MS [M+H]+ 531.6.

### Example 12

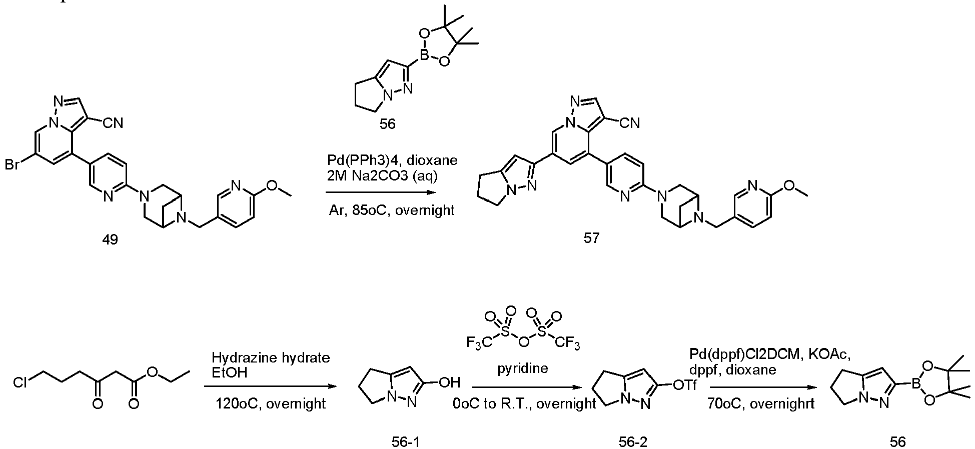

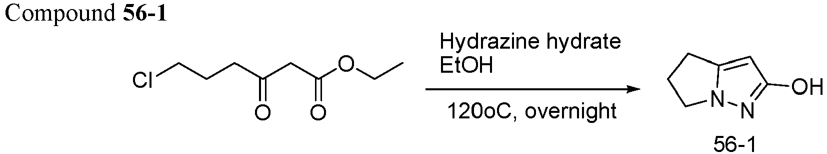

To a 100 mL sealed tube were added successively ethyl 6-chloro-3-oxohexanoate (10 g, 51.9 mmol), hydrazine hydrate (33.3 mL) and EtOH (20 mL). The mixture was stirred at 120 °C overnight, and TLC monitoring showed no starting material remained. The mixture was cooled to room temperature, concentrated and extracted with EA (20 mL × 3). The organic phases were combined, concentrated and purified by column chromatography to give a product as a yellow solid (3.4 g, 53% yield).
LC-MS [M+H]⁺125.06.

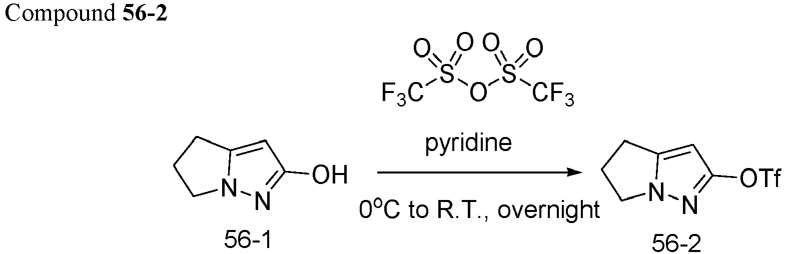

To a 100 mL three-necked flask were added successively **56-1** (3.4 g, 27.4 mmol) and pyridine (55 mL). The temperature was reduced to 0 °C, and trifluoromethanesulfonic anhydride (8.1 g, 28.8 mmol) was slowly added dropwise. After the addition was completed, the mixture was warmed to room temperature and stirred overnight, and TLC monitoring showed no starting material **56-1** remained. The reaction mixture was poured into 20 mL of 2 M HCl and extracted with EA (10 mL × 3). The organic phases were combined, washed successively with saturated brine and saturated sodium carbonate solution, dried over anhydrous sodium sulfate, concentrated and purified by column chromatography to give a product as a colorless oil (1.7 g, 24% yield).
LC-MS[M+H]⁺257.01.

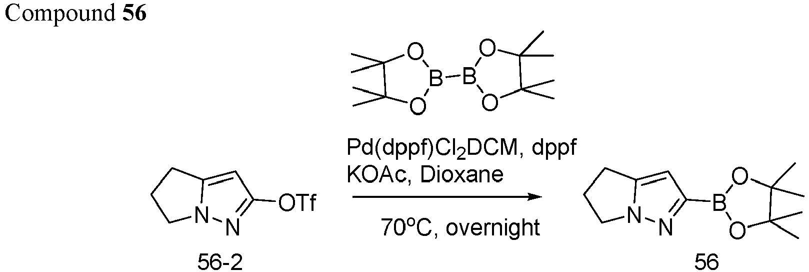

To a 25 mL sealed tube were added successively **56-2** (2 g, 7.8 mmol), bis(pinacolato)diboron (2.1 g, 8.2 mmol), Pd(dppf)Cl₂ DCM (318.2 mg, 0.4 mmol), 1,1'-bis(diphenylphosphino)ferrocene (221.8 mg, 0.4 mmol), KOAc (2.3 g, 23.4 mmol) and 1,4-dioxane (40 mL). The mixture was stirred at 70 °C overnight under Ar, and TLC monitoring showed no starting material 56-2 remained. The mixture was cooled to room temperature, and 60 mL of water was added. The mixture was stirred for 10 min and extracted with DCM (20 mL × 3). The organic phases were combined, dried over anhydrous sodium sulfate and filtered. The filtrate was concentrated to give a product as a reddish-brown liquid (1.7 g, 93% yield), which was directly used in the next step without purification.
LC-MS[M+H]⁺235.15.

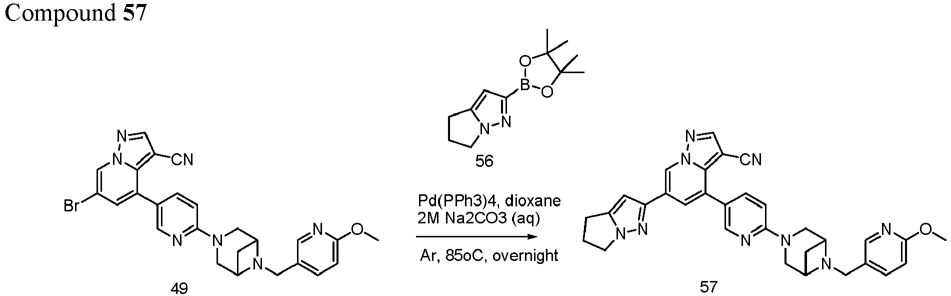

To a 25 mL sealed tube were added successively **49** (50 mg, 0.1 mmol), Pd(PPh₃)₄ (6.36 mg, 0.005 mmol), 56 (23.41 mg, 0.1 mmol), 2 M Na₂CO₃ (21.2 mg, 0.2 mmol) and 1,4-dioxane (5 mL). The mixture was stirred at 85 °C overnight under Ar, and TLC monitoring showed no starting material 49 remained. The mixture was cooled to room temperature, and 10 mL of water was added. The mixture was stirred for 10 min, and a yellow solid precipitated. The solid was collected by filtration, dried and purified by column chromatography to give product 57 (46.3 mg, 88% yield).
¹H NMR (400 MHz, CDCl₃) δ 8.90 (s, 1H), 8.47 (d, J = 2.3 Hz, 1H), 8.28 (s, 1H), 8.12 (d, J = 1.8 Hz, 1H), 7.82 (dd, J = 9.9, 3.4 Hz, 2H), 7.63 (dd, J = 8.5, 2.2 Hz, 1H), 6.70 (t, J = 8.9 Hz, 2H), 6.31 (s, 1H), 4.22 (t, J = 7.2 Hz, 2H), 3.92 (s, 3H), 3.84 (d, J = 11.9 Hz, 2H), 3.77 (d, J = 5.6 Hz, 2H), 3.62 (s, 2H), 3.58 (s, 2H), 2.97 (t, J = 7.3 Hz, 2H), 2.67 (dd, J = 14.3, 7.2 Hz, 3H), 1.67 (d, J = 8.6 Hz, 1H). LC-MS [M+H]⁺ 543.6.

### Example 13

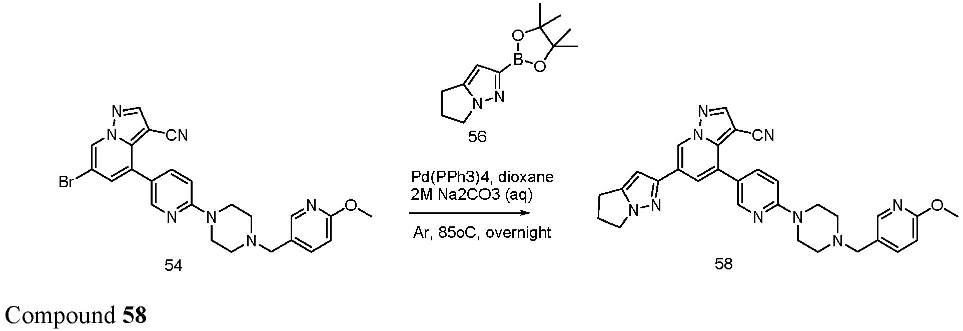

To a 25 mL sealed tube were added successively **54** (50 mg, 0.1 mmol), Pd(PPh₃)₄ (6.36 mg, 0.005 mmol), **56** (23.41 mg, 0.1 mmol), 2 M Na₂CO₃ (21.2 mg, 0.2 mmol) and 1,4-dioxane (5 mL). The mixture was stirred at 85 °C overnight under Ar, and TLC monitoring showed no starting material **54** remained. The mixture was cooled to room temperature, and 10 mL of water was added. The mixture was stirred for 10 min, and a yellow solid precipitated. The solid was collected by filtration, dried and purified by column chromatography to give product **58** (46.3 mg, 88% yield).
¹H NMR (400 MHz, CDCl₃) δ 8.88 (d, J = 1.3 Hz, 1H), 8.39 (d, J = 2.3 Hz, 1H), 8.26 (s, 1H), 8.08 (d, J = 2.0 Hz, 1H), 7.77 (d, J = 1.3 Hz, 1H), 7.74 (dd, J = 8.9, 2.5 Hz, 1H), 7.62 (dd, J = 8.5, 2.3 Hz, 1H), 6.76 (d, J = 2.7 Hz, 1H), 6.74 (d, J = 2.2 Hz, 1H), 6.29 (s, 1H), 4.20 (t, J = 7.2 Hz, 2H), 3.94 (s, 3H), 3.75 - 3.59 (m, 4H), 3.50 (s, 2H), 2.96 (t, J = 7.3 Hz, 2H), 2.73 - 2.59 (m, 2H), 2.60 - 2.48 (m, 4H). LC-MS [M+H]⁺ 531.6.

### Example 14

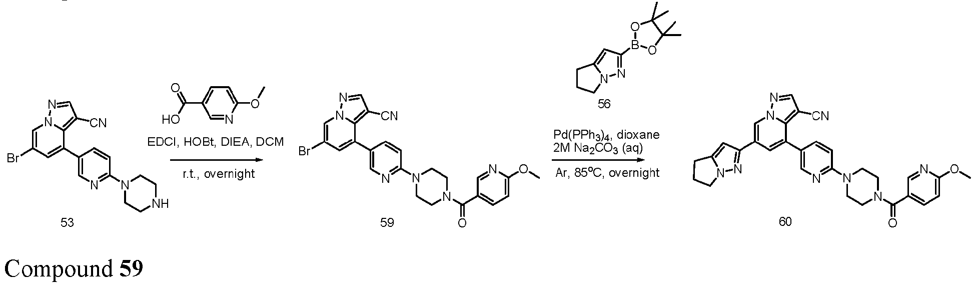

To a 25 mL single-neck flask were added successively **53** (50 mg, 0.13 mmol), 6-methoxynicotinic acid (19.9 mg, 0.13 mmol), EDCI (37.38 mg, 0.2 mmol), HOBt (35.13 mg, 0.26 mmol), DIEA (50.4 mg, 0.39 mmol) and DCM (8 mL). The mixture was stirred at room temperature overnight under Ar, and TLC monitoring showed no starting material **53** remained. 10 mL of water was added, and the mixture was stirred for 10 min. The organic phase was separated, concentrated and purified by column chromatography to give product **59** (55.25 mg, 82% yield).
LC-MS[M+H]⁺518.09.

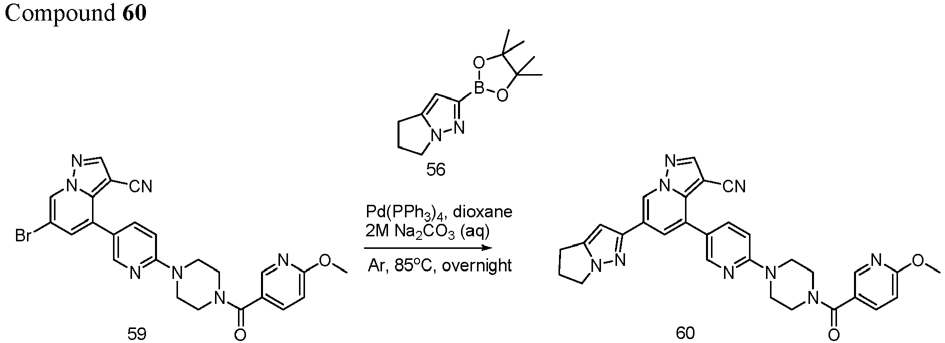

To a 25 mL sealed tube were added successively **59** (55.2 mg, 0.1 mmol), Pd(PPh₃)₄ (6.36 mg, 0.005 mmol), 56 (23.41 mg, 0.1 mmol), 2 M Na₂CO₃ (21.2 mg, 0.2 mmol) and 1,4-dioxane (5 mL). The mixture was stirred at 85 °C overnight under Ar, and TLC monitoring showed no starting material **59** remained. The mixture was cooled to room temperature, and 10 mL of water was added. The mixture was stirred for 10 min, and a yellow solid precipitated. The solid was collected by filtration, dried and purified by column chromatography to give product **60** (40 mg, 73% yield).
¹H NMR (400 MHz, CDCl₃) δ 8.90 (s, 1H), 8.42 (d, J = 2.0 Hz, 1H), 8.33 (s, 1H), 8.27 (s, 1H), 7.87 - 7.77 (m, 2H), 7.73 (dd, J = 8.6, 2.2 Hz, 1H), 6.81 (dd, J = 8.6, 5.7 Hz, 2H), 6.31 (s, 1H), 4.21 (t, J = 7.2 Hz, 2H), 3.99 (d, J = 4.7 Hz, 3H), 3.73 (s, 8H), 3.00 - 2.91 (m, 2H), 2.72 - 2.57 (m, 3H). LC-MS [M+H]⁺ 545.6.

### Example 15

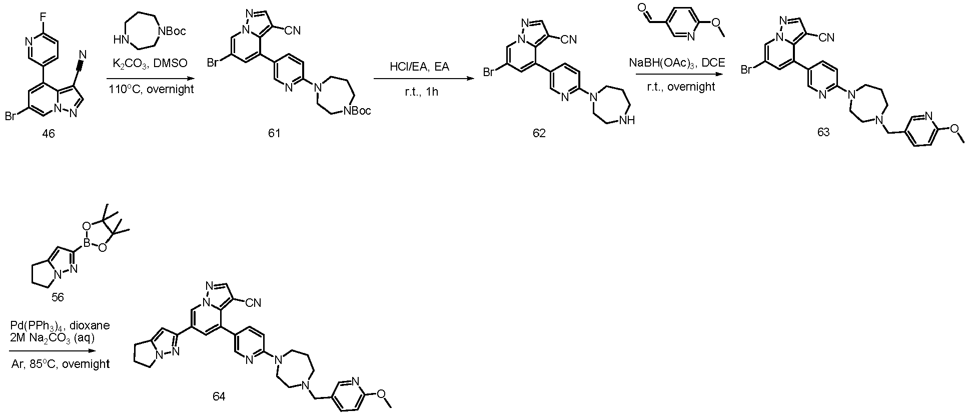

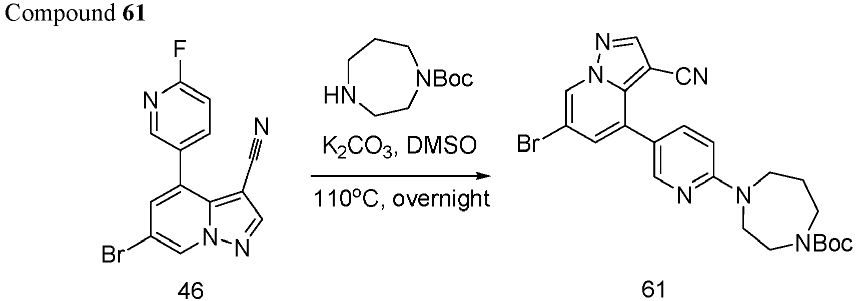

To a 45 mL sealed tube were added successively **46** (200 mg, 0.63 mmol), *tert*-butyl 1,4-diazepan-1-carboxylate (151.57 mg, 0.76 mmol), K₂CO₃ (173.88 mg, 1.26 mmol) and DMSO (10 mL). The mixture was stirred at 120 °C overnight, and TLC monitoring showed no starting material **46** remained. The mixture was cooled to room temperature, and 50 mL of water was added. The mixture was stirred for 10 min, and a yellow solid precipitated. The solid was collected by filtration and dried to give product **61** (250 mg, 80% yield).
LC-MS [M+H]⁺497.12.

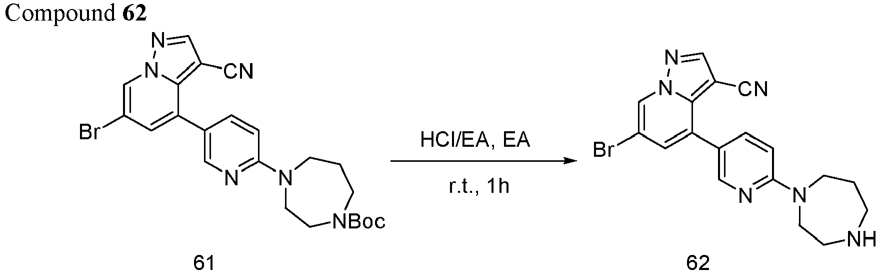

To a 25 mL single-neck flask was added a 3.5 M solution of HCl in EA (10 mL), and a solution of **61** (250 mg, 0.5 mmol) in EA was slowly added dropwise. After the addition was completed, the mixture was stirred at room temperature for another 1 h, concentrated to remove the solvent, neutralized by adding dropwise a solution of NH₃ in methanol, concentrated and purified by column chromatography to give product **62** as a brownish-yellow solid (188.7 mg, 95% yield).
LC-MS [M+H]⁺397.07.

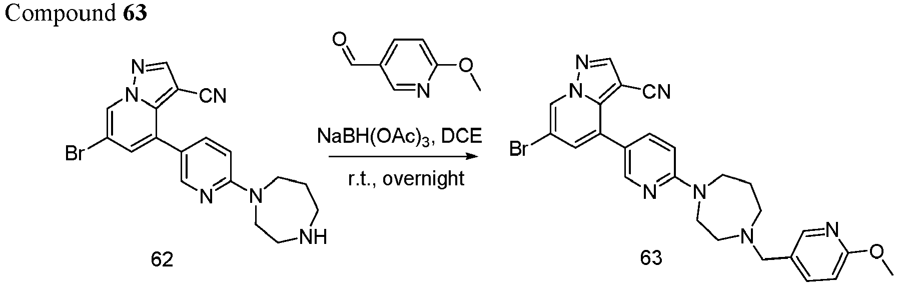

To a 50 mL single-neck flask were added successively **62** (188.7 mg, 0.47 mmol), 6-methoxy-3-pyridinecarboxaldehyde (78.2 mg, 0.57 mmol) and DCM (10 mL). After 10 min of stirring, NaBH(OAc)₃ (498.2 mg, 2.35 mmol) was added in batches at room temperature. The mixture was stirred at room temperature overnight, and TLC monitoring showed no starting material **62** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **63** as a yellow solid (158 mg, 65% yield).
LC-MS [M+H]⁺518.12.

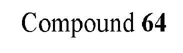

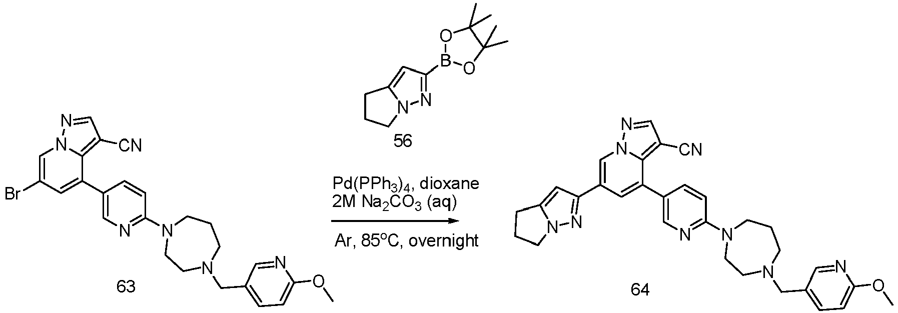

To a 25 mL sealed tube were added successively **63** (52 mg, 0.1 mmol), Pd(PPh₃)₄ (6.36 mg, 0.005 mmol), **56** (23.41 mg, 0.1 mmol), 2 M Na₂CO₃ (21.2 mg, 0.2 mmol) and 1,4-dioxane (5 mL). The mixture was stirred at 85 °C overnight under Ar, and TLC monitoring showed no starting material **63** remained. The mixture was cooled to room temperature, and 10 mL of water was added. The mixture was stirred for 10 min, and a yellow solid precipitated. The solid was collected by filtration, dried and purified by column chromatography to give product **64** (39.8 mg, 73% yield).
¹H NMR (400 MHz, CDCl₃) δ 8.88 (d, J = 1.3 Hz, 1H), 8.37 (d, J = 2.4 Hz, 1H), 8.26 (s, 1H), 8.05 (d, J = 2.0 Hz, 1H), 7.77 (d, J = 1.3 Hz, 1H), 7.72 (dd, J = 8.8, 2.6 Hz, 1H), 7.61 (d, J = 6.9 Hz, 1H), 6.72 (d, J = 8.5 Hz, 1H), 6.61 (d, J = 9.0 Hz, 1H), 6.31 (s, 1H), 4.25 - 4.17 (t, 2H), 3.93 (s, 3H), 3.86 (m, 2H), 3.72 (t, J = 6.1 Hz, 2H), 3.58 (s, 2H), 3.02 - 2.92 (m, 2H), 2.79 (m, 2H), 2.71, 2.63 (m, 4H), 1.99 (m, 2H). LC-MS [M+H]⁺ 545.6.

### Example 16

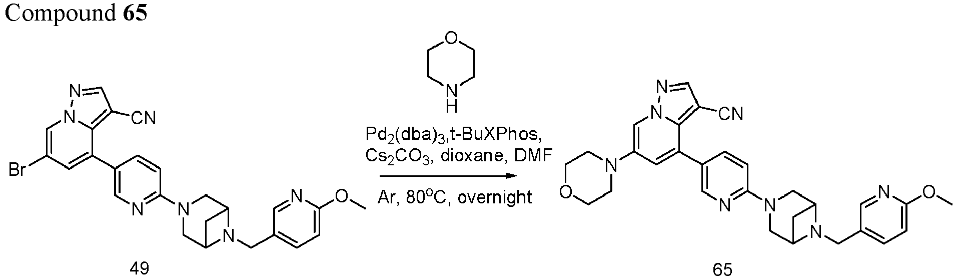

To a 25 mL sealed tube were added successively **49** (52 mg, 0.1 mmol), Pd₂(dba)₃ (5.5 mg, 0.006 mmol), t-BuXPhos (7.6 mg, 0.018 mmol), morpholine (26 mg, 0.3 mmol), Cs₂CO₃ (65.2 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **65** (47 mg, 90% yield).
¹H NMR (400 MHz, CDCl₃) δ 8.39 (d, J = 2.3 Hz, 1H), 8.20 (s, 1H), 8.10 (d, J = 2.0 Hz, 1H), 8.02 (d, J = 1.9 Hz, 1H), 7.79 (dd, J = 8.8, 2.5 Hz, 1H), 7.65 (dd, J = 8.5, 2.2 Hz, 1H), 7.17 (d, J = 1.9 Hz, 1H), 6.71 (dd, J = 13.3, 8.7 Hz, 2H), 3.91 (m, J = 8.4 Hz, 6H), 3.82 (m, J = 19.6, 8.9 Hz, 4H), 3.59 (m, 4H), 3.22 - 3.09 (m, 4H), 2.71 (m, J = 7.0 Hz, 1H), 1.66 (d, J = 8.7 Hz, 2H). LC-MS [M+H]⁺ 522.6.

### Example 17

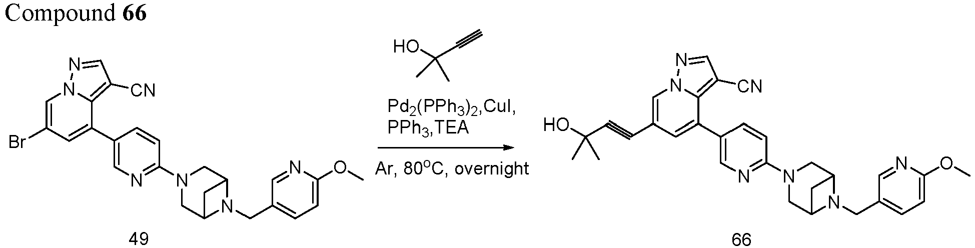

To a 25 mL sealed tube were added successively **49** (52 mg, 0.1 mmol), Pd₂(PPh₃)₂ (5.5 mg, 0.005 mmol), CuI (19 mg, 0.1 mmol), PPh₃ (52 mg, 0.2 mmol), 2-methylbut-3-yn-2-ol (25 mg, 0.3 mmol) and TEA (2 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **65** (46.7 mg, 90% yield).
¹H NMR (400 MHz, CDCl₃) δ 8.61 (d, J = 1.3 Hz, 1H), 8.40 (d, J = 2.3 Hz, 1H), 8.31 (s, 1H), 8.11 (d, J = 2.0 Hz, 1H), 7.76 (dd, J = 8.8, 2.5 Hz, 1H), 7.67 (d, J = 8.3 Hz, 1H), 7.30 (d, J = 1.3 Hz, 1H), 6.71 (dd, J = 13.8, 8.6 Hz, 2H), 3.92 (s, 3H), 3.89 - 3.74 (m, 4H), 3.61 (m, 4H), 2.73 (m, 1H), 2.22 (m, 1H), 1.66 (s, 6H). LC-MS [M+H]⁺519.6.

### Example 18

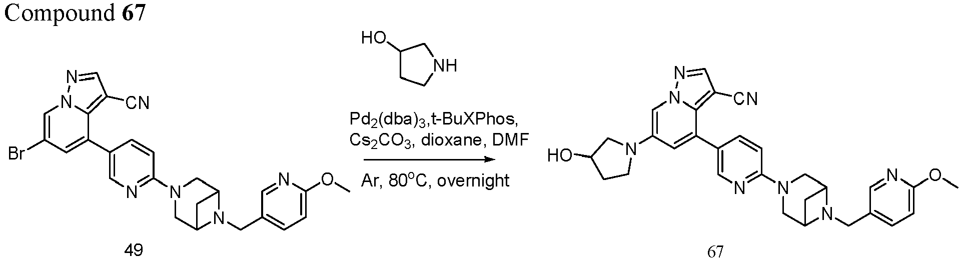

To a 25 mL sealed tube were added successively **49** (52 mg, 0.1 mmol), Pd₂(dba)₃ (5.5 mg, 0.006 mmol), t-BuXPhos (7.6 mg, 0.018 mmol), 3-hydroxypyrrolidine (26 mg, 0.3 mmol), Cs₂CO₃ (65.2 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **67** (47 mg, 90% yield).
¹H NMR (400 MHz, CDCl₃) δ 8.40 (d, J = 2.3 Hz, 1H), 8.14 (s, 1H), 8.10 (d, J = 1.9 Hz, 1H), 7.82 (d, J = 2.5 Hz, 1H), 7.79 (d, J = 1.8 Hz, 1H), 7.65 (dd, J = 8.5, 2.3 Hz, 1H), 6.93 (d, J = 2.0 Hz, 1H), 6.71 (t, J = 9.2 Hz, 2H), 4.69 (m, 1H), 3.92 (s, 3H), 3.88 - 3.75 (m, 4H), 3.67 - 3.50 (m, 6H), 3.40 (td, J = 8.7, 3.1 Hz, 1H), 3.31 (d, J = 10.0 Hz, 1H), 2.70 (m, 1H), 2.25 (m, 1H), 2.15 (m, 1H), 1.67 (d, J = 8.6 Hz, 2H). LC-MS [M+H]⁺ 522.6.

### Example 19

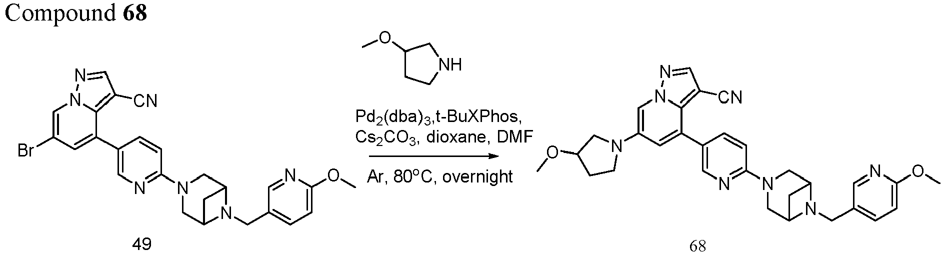

To a 25 mL sealed tube were added successively **49** (52 mg, 0.1 mmol), Pd₂(dba)₃ (5.5 mg, 0.006 mmol), *t*-BuXPhos (7.6 mg, 0.018 mmol), 3-methoxypyrrolidine (26 mg, 0.3 mmol), Cs₂CO₃ (65.2 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **68** (48.3 mg, 90% yield).
¹H NMR (400 MHz, CDCl₃) δ 8.40 (d, J = 2.3 Hz, 1H), 8.14 (s, 1H), 8.10 (d, J = 2.0 Hz, 1H), 7.82 (d, J = 2.5 Hz, 1H), 7.80 (t, J = 2.5 Hz, 1H), 7.66 (d, J = 8.3 Hz, 1H), 6.94 (d, J = 2.0 Hz, 1H), 6.76 - 6.65 (m, 2H), 4.20 - 4.10 (m, 1H), 3.92 (s, 3H), 3.83 (m, 4H), 3.65-3.60 (m, 4H), 3.55 - 3.42 (m, 2H), 3.39 (s, 3H), 3.37-3.34 (m, 1H), 2.74-2.68 (m, 1H), 2.29 - 2.09 (m, 2H), 1.67 (d, J = 8.6 Hz, 2H). LC-MS [M+H]⁺536.6.

### Example 20

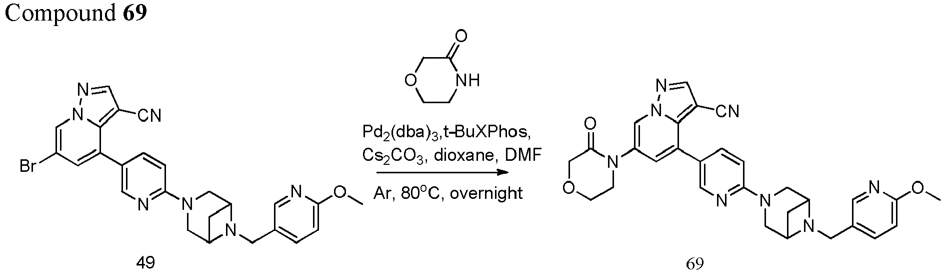

To a 25 mL sealed tube were added successively **49** (52 mg, 0.1 mmol), Pd₂(dba)₃ (5.5 mg, 0.006 mmol), *t*-BuXPhos (7.6 mg, 0.018 mmol), morpholin-3-one (30 mg, 0.3 mmol), Cs₂CO₃ (65.2 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **69** (48.3 mg, 90% yield).
¹H NMR (400 MHz, CDCl₃) δ 8.39 (d, J = 2.3 Hz, 1H), 8.20 (s, 1H), 8.10 (d, J = 2.0 Hz, 1H), 8.02 (d, J = 1.9 Hz, 1H), 7.79 (dd, J = 8.8, 2.5 Hz, 1H), 7.65 (dd, J = 8.5, 2.2 Hz, 1H), 7.17 (d, J = 1.9 Hz, 1H), 6.71 (dd, J = 13.3, 8.7 Hz, 2H), 3.91 (m, J = 8.4 Hz, 6H), 3.82 (m, J = 19.6, 8.9 Hz, 4H), 3.59 (m, 2H), 3.22 - 3.09 (m, 2H), 2.92 (s, 2H) 2.71 (m, J = 7.0 Hz, 1H), 1.66 (d, J = 8.7 Hz, 2H). LC-MS [M+H]⁺537.58

### Example 21

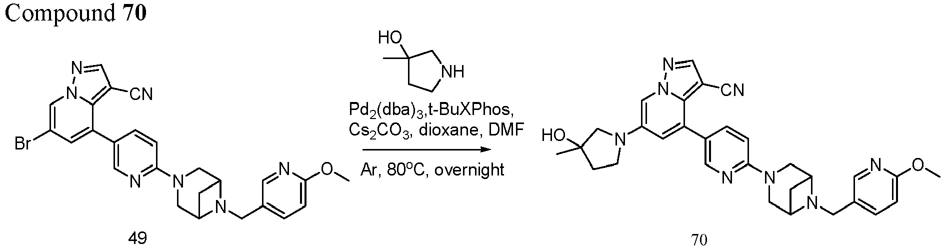

To a 25 mL sealed tube were added successively **49** (52 mg, 0.1 mmol), Pd₂(dba)₃ (5.5 mg, 0.006 mmol), *t*-BuXPhos (7.6 mg, 0.018 mmol), 3-hydroxy-3-methylpyrrole (30 mg, 0.3 mmol), Cs₂CO₃ (65.2 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **70** (48.3 mg, 90% yield).
¹H NMR (400 MHz, CDCl₃) δ 8.39 (d, J = 2.4 Hz, 1H), 8.14 (s, 1H), 8.11 (d, J = 2.2 Hz, 1H), 7.80 (dd, J = 8.8, 2.3 Hz, 1H), 7.76 (d, J = 1.3 Hz, 1H), 7.65 (dd, J = 8.5, 2.4 Hz, 1H), 6.90 (d, J = 1.7 Hz, 1H), 6.72 (dd, J = 11.9, 8.7 Hz, 2H), 3.93 (s, 3H), 3.85 (d, J = 11.7 Hz, 2H), 3.80 (d, J = 5.8 Hz, 2H), 3.65-3.60 (m, 2H), 3.59 (s, 3H), 3.42 (td, J = 8.5, 3.0 Hz, 1H), 3.38 - 3.30 (m, 2H), 2.71 (dd, J = 14.1, 6.3 Hz, 1H), 2.22 - 2.05 (m, 3H), 1.67 (d, J = 8.7 Hz, 0H), 1.56 (s, 1H). LC-MS [M+H]⁺ 536.6.

### Example 22

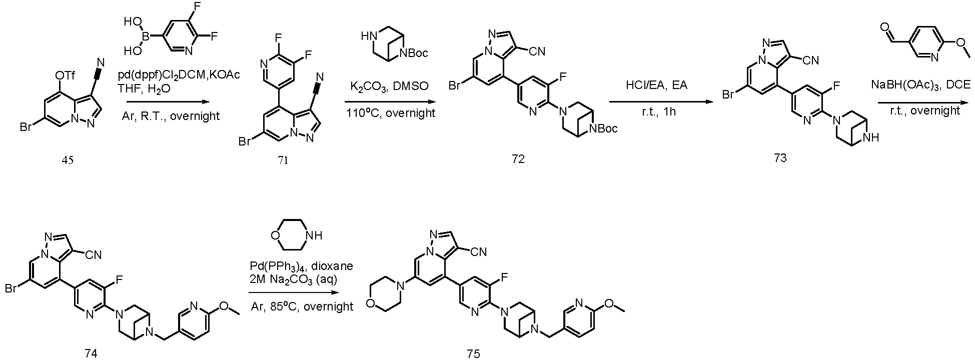

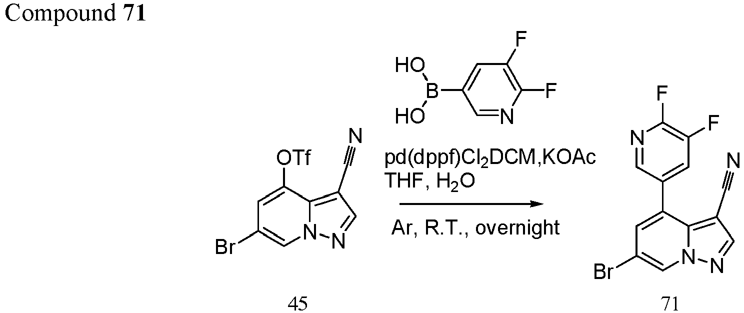

To a 12 mL sealed tube were added successively **45** (500 mg, 1.35 mmol), Pd(dppf)Cl₂ complexed with dichloromethane (57 mg, 0.07 mmol), 2,3-difluoropyridine-5-boronic acid (178.7 mg, 1.13 mmol), KOAc (265 mg, 2.7 mmol), THF (10 mL) and H₂O (2 mL). The mixture was stirred at 85 °C overnight under Ar, and TLC monitoring showed no starting material **45** remained. The mixture was cooled to room temperature, and 20 mL of water was added. The mixture was stirred for 10 min, and a brownish-yellow solid precipitated. The solid was collected by filtration and dried to give product **71** (93.8 mg, 80% yield).
LC-MS [M+H]⁺334.97.

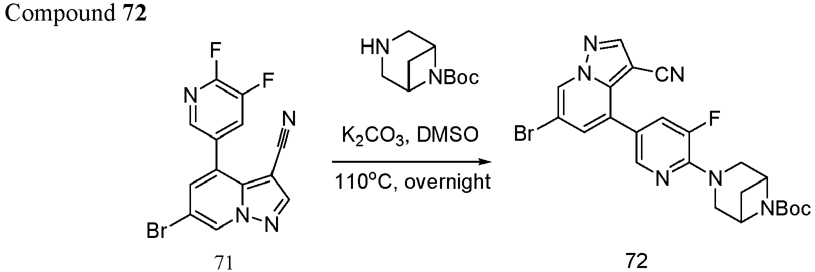

To a 45 mL sealed tube were added successively **71** (93.8 mg, 1.08 mmol), 6-(*tert-*butoxycarbonyl)-3,6-diazabicyclo[3.1.1]heptane (257 mg, 1.3 mmol), K₂CO₃ (298.5 mg, 2.16 mmol) and DMSO (2 mL). The mixture was stirred at 110 °C overnight, and TLC monitoring showed no starting material **71** remained. The mixture was cooled to room temperature, and 10 mL of water was added. The mixture was stirred for 10 min, and a yellow solid precipitated. The solid was collected by filtration and dried to give product 72 (382.5 mg, 69% yield).
LC-MS [M+H]⁺513.1.

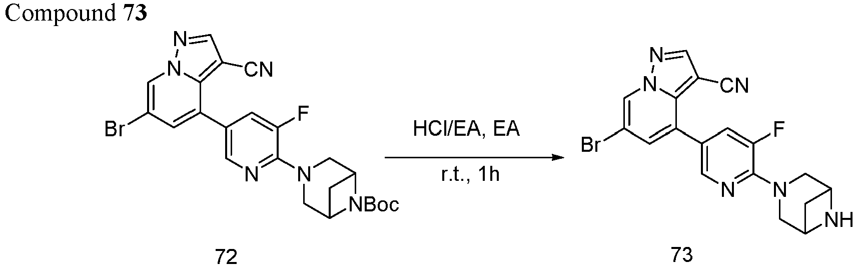

To a 25 mL single-neck flask was added a 3.5 M solution of HCl in EA (10 mL), and a solution **of 72** (382.5 mg, 0.74 mmol) in EA was slowly added dropwise. After the addition was completed, the mixture was stirred at room temperature for another 1 h, concentrated to remove the solvent, neutralized by adding dropwise a solution of NH₃ in methanol, concentrated and purified by column chromatography to give product **73** as a brownish-yellow solid (277 mg, 90% yield).
LC-MS [M+H]⁺413.04.

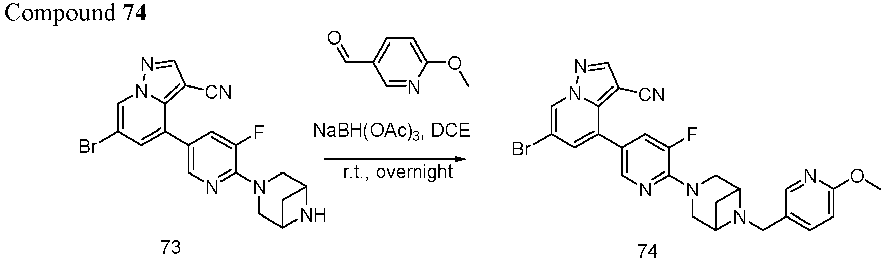

To a 50 mL single-neck flask were added successively 73 (100 mg, 0.24 mmol), 6-methoxy-3-pyridinecarboxaldehyde (39.8 mg, 0.29 mmol) and DCM (10 mL). After 10 min of stirring, NaBH(OAc)₃ (254.4 mg, 1.2 mmol) was added in batches at room temperature. The mixture was stirred at room temperature overnight, and TLC monitoring showed no starting material 73 remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **74** as a yellow solid (77 mg, 60% yield).
LC-MS [M+H]⁺534.1.

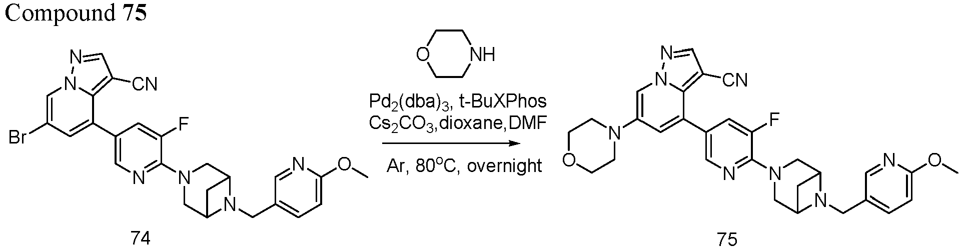

To a 25 mL sealed tube were added successively **74** (77 mg, 0.14 mmol), Pd₂(dba)₃ (7.9 mg, 0.009 mmol), *t*-BuXPhos (11.5 mg, 0.027 mmol), morpholine (36.6 mg, 0.42 mmol), Cs₂CO₃ (91.2 mg, 0.28 mmol), 1,4-dioxane (3 mL) and DMF (1 mL). The mixture was stirred at 80 °C overnight under Ar, and TLC monitoring showed no starting material **74** remained. The mixture was cooled to room temperature, and 10 mL of water was added. The mixture was stirred for 10 min, and a yellow solid precipitated. The solid was collected by filtration, dried and purified by column chromatography to give product **75** (68 mg, 90% yield).
¹H NMR (400 MHz, CDCl₃) δ 8.21 (s, 1H), 8.18 (s, 1H), 8.15 (s, 1H), 8.04 (d, J = 1.9 Hz, 1H), 7.50 (s, 1H), 7.46 (s, 1H), 7.19 (d, J = 1.9 Hz, 1H), 6.76 (d, J = 8.6 Hz, 1H), 3.95 - 3.88 (m, 10H), 3.78 (s, 2H), 3.23 - 3.11 (m, 5H), 2.39 - 2.18 (m, 2H), 2.01 (s, 2H). LC-MS [M+H]⁺ 540.6.

### Example 23

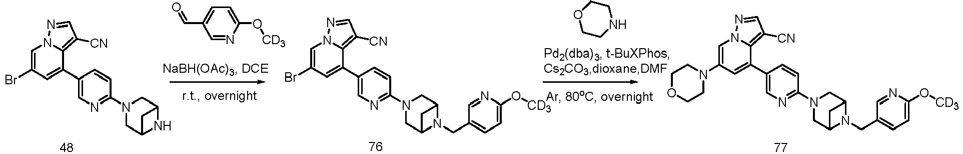

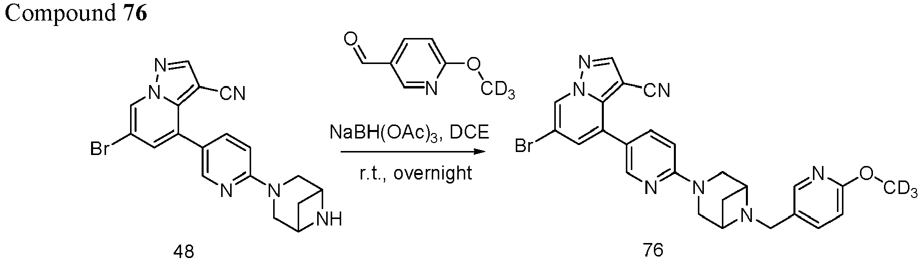

To a 50 mL single-neck flask were added successively **48** (60 mg, 0.15 mmol), 6-deuteromethoxy-3-pyridinecarboxaldehyde (25.5 mg, 0.18 mmol) and DCM (10 mL). After 10 min of stirring, NaBH(OAc)₃ (159 mg, 0.75 mmol) was added in batches at room temperature. The mixture was stirred at room temperature overnight, and TLC monitoring showed no starting material **48** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **76** as a yellow solid (56 mg, 71.8% yield).

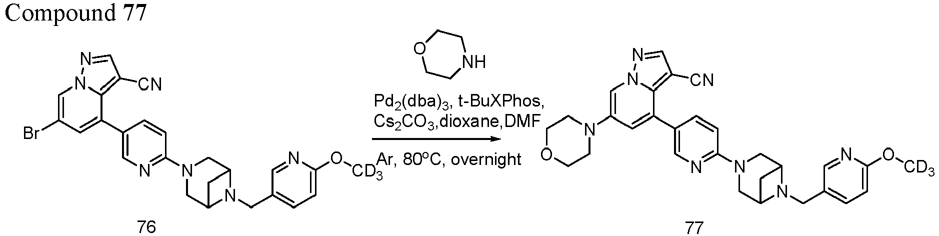

To a 25 mL sealed tube were added successively **76** (56 mg, 0.11 mmol), Pd₂(dba)₃ (6 mg, 0.007 mmol), t-BuXPhos (8.4 mg, 0.02 mmol), morpholine (26 mg, 0.33 mmol), Cs₂CO₃ (71.6 mg, 0.22 mmol), 1,4-dioxane (3 mL) and DMF (1 mL). The mixture was stirred at 80 °C overnight under Ar, and TLC monitoring showed no starting material **76** remained. The mixture was cooled to room temperature, and 10 mL of water was added. The mixture was stirred for 10 min, and a yellow solid precipitated. The solid was collected by filtration, dried and purified by column chromatography to give product **77** (53.7 mg, 93% yield).
¹H NMR (400 MHz, CDCl₃) δ 8.39 (s, 1H), 8.20 (s, 1H), 8.10 (s, 1H), 8.02 (s, 1H), 7.79 (d, J = 8.4 Hz, 1H), 7.17 (s, 1H), 6.99 (d, J = 8.9 Hz, 1H), 6.71 (dd, J = 15.3, 8.5 Hz, 2H), 3.91 (d, J = 4.7 Hz, 4H), 3.86-3.81 (m, 2H), 3.63 (s, 2H), 3.20 - 3.12 (m, 4H), 2.78-2.74 (m, 1H), 2.35 (m, 1H), 2.24 (dd, J = 20.5, 6.6 Hz, 2H), 2.04-2.00 (m, 2H). LC-MS [M+H]⁺ 525.6.

### Example 24

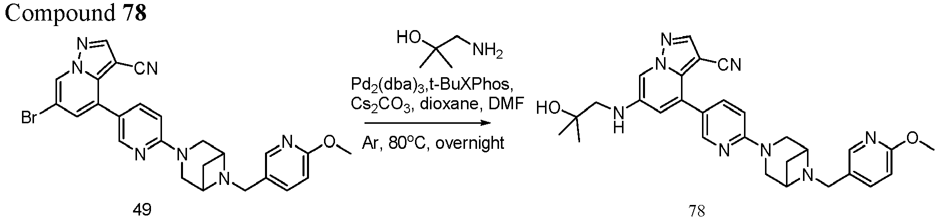

To a 25 mL sealed tube were added successively **49** (52 mg, 0.1 mmol), Pd₂(dba)₃ (5.5 mg, 0.006 mmol), t-BuXPhos (8.4 mg, 0.02 mmol), 1-amino-2-methyl-2-propanol (26.7 mg, 0.3 mmol), Cs₂CO₃ (65 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **78** (42.5 mg, 81% yield).
¹HNMR (400 MHz, CDCl₃) δ 8.40 (d, J = 2.3 Hz, 1H), 8.15 (s, 1H), 8.11 (s, 1H), 7.93 (d, J = 1.4 Hz, 1H), 7.82 (dd, J = 8.7, 2.3 Hz, 1H), 7.74 (d, J = 8.2 Hz, 1H), 7.08 (d, J = 1.5 Hz, 1H), 6.72 (dd, J = 16.0, 8.7 Hz, 2H), 3.97 (d, J = 21.1 Hz, 4H), 3.91 (s, 3H), 3.76-3.73 (m, 4H), 3.33 (dd, J = 43.0, 8.5 Hz, 2H), 2.92-2.86 (m, 2H), 1.76-1.72 (m, 1H), 1.47 (s, 3H), 1.27 (s, 3H). LC-MS [M+H]⁺526.6

### Example 25

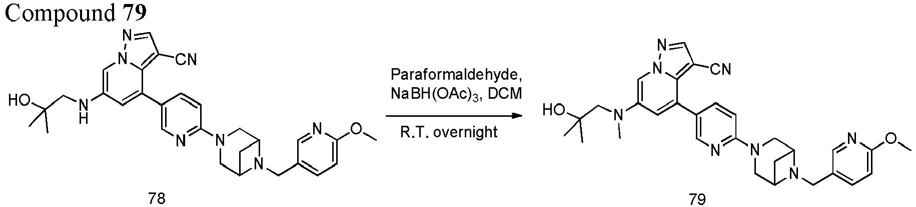

To a 25 mL single-neck flask were added successively **78** (50 mg, 0.09 mmol), paraformaldehyde (50 mg) and DCM (10 mL). After 10 min of stirring, NaBH(OAc)₃ (95.4 mg, 0.45 mmol) was added in batches at room temperature. The mixture was stirred at room temperature overnight, and TLC monitoring showed no starting material **78** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **79** as a yellow solid (33.9 mg, 70% yield).
¹HNMR (400 MHz, CDCl₃) δ 8.40 (d, J = 2.3 Hz, 1H), 8.15 (s, 1H), 8.11 (s, 1H), 7.93 (d, J = 1.4 Hz, 1H), 7.82 (dd, J = 8.7, 2.3 Hz, 1H), 7.74 (d, J = 8.2 Hz, 1H), 7.08 (d, J = 1.5 Hz, 1H), 6.72 (dd, J = 16.0, 8.7 Hz, 2H), 3.97 (d, J = 21.1 Hz, 4H), 3.91 (s, 3H), 3.84 (s, 3H), 3.76-3.73 (m, 4H), 3.33 (dd, J = 43.0, 8.5 Hz, 2H), 2.92-2.86 (m, 2H), 1.76-1.72 (m, 1H), 1.47 (s, 3H), 1.27 (s, 3H). LC-MS [M+H]⁺539.6

### Example 26

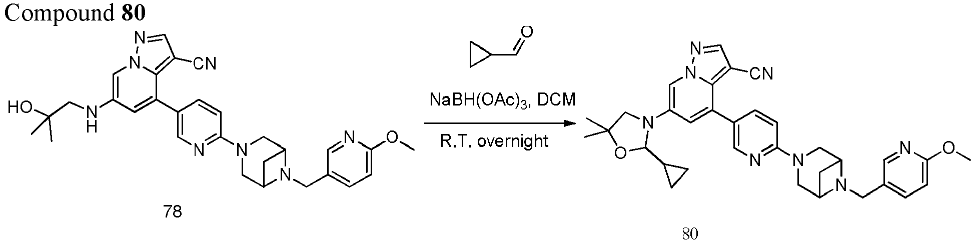

To a 25 mL single-neck flask were added successively **78** (50 mg, 0.09 mmol), cyclopropanecarboxaldehyde (0.5 mL) and DCM (10 mL). After 10 min of stirring, NaBH(OAc)₃ (95.4 mg, 0.45 mmol) was added in batches at room temperature. The mixture was stirred at room temperature overnight, and TLC monitoring showed no starting material **78** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **80** as a yellow solid (30 mg, 58% yield).
¹HNMR (400 MHz, CDCl₃ ) δ 8.40 (d, J = 2.3 Hz, 1H), 8.15 (s, 1H), 8.11 (s, 1H), 7.93 (d, J = 1.4 Hz, 1H), 7.82 (dd, J = 8.7, 2.3 Hz, 1H), 7.74 (d, J = 8.2 Hz, 1H), 7.08 (d, J = 1.5 Hz, 1H), 6.72 (dd, J = 16.0, 8.7 Hz, 2H), 4.84 (d, J = 5.7 Hz, 1H), 3.97 (d, J = 21.1 Hz, 4H), 3.91 (s, 3H), 3.76-3.73 (m, 4H), 3.33 (dd, J = 43.0, 8.5 Hz, 2H), 2.92-2.86 (m, 2H), 2.05 (s, 1H), 1.76-1.72 (m, 1H), 1.47 (s, 3H), 1.27 (d, J = 4.1 Hz, 3H), 1.24 (s, 3H), 1.18-1.11 (m, 1H), 0.7-0.50 (m, 5H). LC-MS [M+H]⁺576.7.

### Example 27

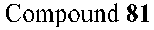

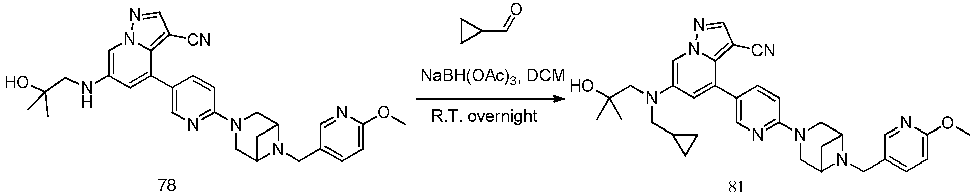

To a 25 mL single-neck flask were added successively **78** (50 mg, 0.09 mmol), cyclopropanecarboxaldehyde (0.5 mL) and DCM (10 mL). After 10 min of stirring, NaBH(OAc)₃ (95.4 mg, 0.45 mmol) was added in batches at room temperature. The mixture was stirred at room temperature overnight, and TLC monitoring showed no starting material **78** remained. The mixture was quenched by adding ammonia water. The aqueous phase was separated and extracted with DCM (5 mL × 3). The organic phases were combined, washed with saturated brine, dried over anhydrous sodium sulfate, concentrated and purified by column chromatography to give product **81** as a yellow solid (28 mg, 54% yield).
¹HNMR (400 MHz, CDCl₃ ) δ 8.40 (d, J = 2.3 Hz, 1H), 8.15 (s, 1H), 8.11 (s, 1H), 7.93 (d, J = 1.4 Hz, 1H), 7.82 (dd, J = 8.7, 2.3 Hz, 1H), 7.74 (d, J = 8.2 Hz, 1H), 7.08 (d, J = 1.5 Hz, 1H), 6.72 (dd, J= 16.0, 8.7 Hz, 2H), 3.97 (d, J = 21.1 Hz, 4H), 3.91 (s, 3H), 3.84 (d, J = 5.7 Hz, 2H), 3.76-3.73 (m, 4H), 3.33 (dd, J = 43.0, 8.5 Hz, 2H), 2.92-2.86 (m, 2H), 1.76-1.72 (m, 1H), 1.47 (s, 3H), 1.27 (s, 3H), 1.18-1.11 (m, 1H), 0.65-0.50 (m, 4H). LC-MS [M+H]⁺579.7

### Example 28

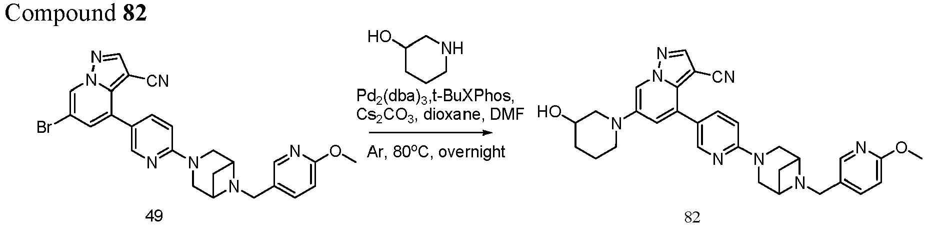

To a 25 mL sealed tube were added successively **49** (52 mg, 0.1 mmol), Pd₂(dba)₃ (5.5 mg, 0.006 mmol), t-BuXPhos (8.4 mg, 0.02 mmol), 3-hydroxypiperidine (30.3 mg, 0.3 mmol), Cs₂CO₃ (65 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **82** (40.2 mg, 75% yield).
¹H NMR (400 MHz, CDCl₃) δ 8.40 (s, 1H), 8.19 (s, 1H), 8.11 (s, 1H), 8.05 (s, 1H), 7.78 (d, J = 10.9 Hz, 1H), 7.29 (s, 1H), 7.19 (s, 1H), 6.71 (dd, J = 17.2, 8.7 Hz, 2H), 4.03-3.99 (m, 1H), 3.92 (s, 3H), 3.88-3.82 (m, 3H), 3.65-3.60 (m, 3H), 3.33 (d, J = 9.2 Hz, 1H), 3.16 - 3.01 (m, 3H), 2.83 - 2.70 (m, 2H), 1.99-1.94 (m, 4H). LC-MS [M+H]⁺536.6.

### Example 29

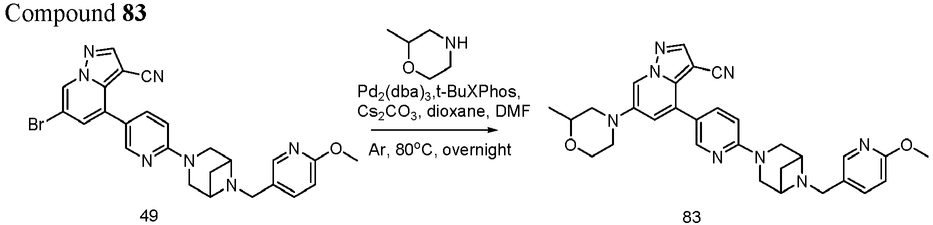

To a 25 mL sealed tube were added successively **49** (52 mg, 0.1 mmol), Pd₂(dba)₃ (5.5 mg, 0.006 mmol), *t*-BuXPhos (8.4 mg, 0.02 mmol), 2-methylmorpholine (30.3 mg, 0.3 mmol), Cs₂CO₃ (65 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **83** (38 mg, 70.8% yield).
¹H NMR (400 MHz, CDCl₃) δ 8.40 (s, 1H), 8.20 (s, 1H), 8.11 (s, 1H), 8.01 (s, 1H), 7.80 (d, J = 8.6 Hz, 1H), 7.69 (s, 1H), 7.18 (s, 1H), 6.75 - 6.63 (m, 2H), 4.08-4.05 (m, 1H), 3.92 (s,3H), 3.88-3.84 (m,5H), 3.64-3.58 (m, 4H), 3.38 (dd, J = 23.9, 11.6 Hz, 2H), 2.93-2.90(m, 1H), 2.76-2.72 (m, 1H), 2.59 - 2.51 (m, 1H), 1.41 (d, J = 19.1 Hz, 2H), 1.31 - 1.24 (m, 3H). LC-MS [M+H]⁺ 536.6.

### Example 30

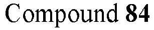

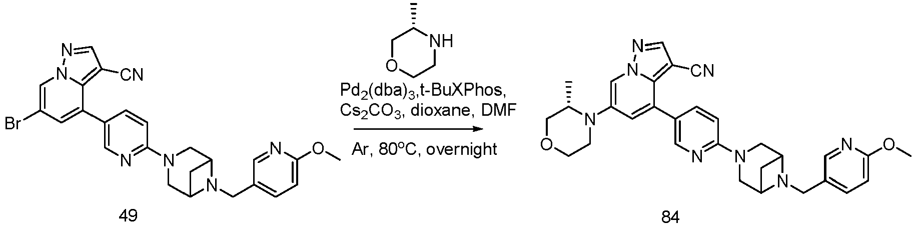

To a 25 mL sealed tube were added successively **49** (52 mg, 0.1 mmol), Pd₂(dba)₃ (5.5 mg, 0.006 mmol), *t*-BuXPhos (8.4 mg, 0.02 mmol), 3-(*S*)-3-methylmorpholine (30.3 mg, 0.3 mmol), Cs₂CO₃ (65 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **84** (30 mg, 56% yield).
¹H NMR (400 MHz, CDCl₃) δ 8.40 (d, J = 2.2 Hz, 1H), 8.20 (s, 1H), 8.11 (s, 1H), 8.02 (d, J = 1.8 Hz, 1H), 7.81 (dd, J = 8.8, 2.5 Hz, 1H), 7.70 (s, 1H), 7.18 (d, J = 1.9 Hz, 1H), 6.72 (dd, J = 14.1, 8.7 Hz, 2H), 4.02 (d, J = 11.2 Hz, 1H), 3.92 (s, 3H), 3.90-3.85 (m, 5H), 3.81-3.75 (m, 2H), 3.75-3.63 (m, 6H), 3.27 - 3.16 (m, 1H), 3.03 (d, J= 11.8 Hz, 1H), 2.79-2.75 (m, 1H), 1.14 (d, J = 6.5 Hz, 3H). LC-MS [M+H]⁺ 536.6.

### Example 31

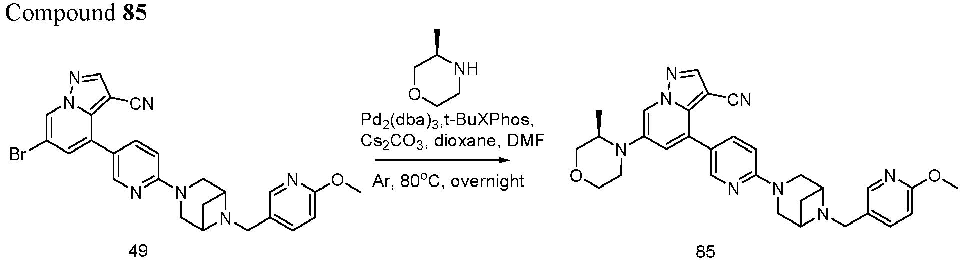

To a 25 mL sealed tube were added successively **49** (52 mg, 0.1 mmol), Pd₂(dba)₃ (5.5 mg, 0.006 mmol), *t*-BuXPhos (8.4 mg, 0.02 mmol), (*R*)-3-methylmorpholine (30.3 mg, 0.3 mmol), Cs₂CO₃ (65 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **85** (32 mg, 56% yield).
¹H NMR (400 MHz, CDCl₃) δ 8.41 (d, J = 2.1 Hz, 1H), 8.20 (s, 1H), 8.13 (s, 1H), 8.03 (d, J = 1.9 Hz, 1H), 7.82 (dd, J = 8.7, 2.2 Hz, 1H), 7.34 (d, J = 7.1 Hz, 1H), 7.18 (d, J = 1.8 Hz, 1H), 6.74 (dd, J = 21.1, 8.7 Hz, 2H), 4.02 (d, J= 11.8 Hz, 2H), 3.93 (s, 3H), 3.92-3.89 (m, 2H), 3.85 - 3.56 (m, 6H), 3.38 - 2.81 (m, 4H), 2.50-2.39(m,3H),1.14 (d, J = 6.5 Hz, 3H). LC-MS [M+H]⁺ 536.6.

### Example 32

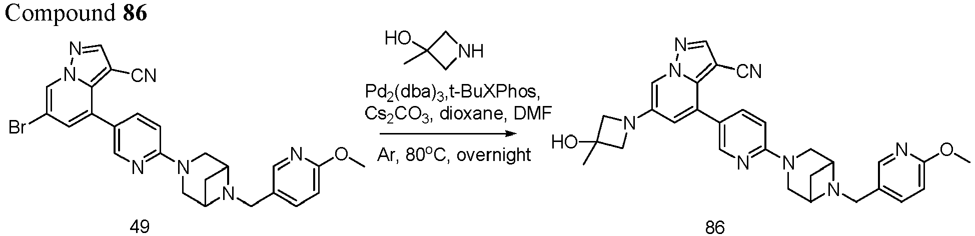

To a 25 mL sealed tube were added successively **49** (52 mg, 0.1 mmol), Pd₂(dba)₃ (5.5 mg, 0.006 mmol), *t*-BuXPhos (8.4 mg, 0.02 mmol), 3-methyl-3-azetidinol (26 mg, 0.3 mmol), Cs₂CO₃ (65 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **86** (34 mg, 65% yield).
¹H NMR (400 MHz, CDCl₃) δ 8.37 (d, J = 2.3 Hz, 1H), 8.15 (s, 1H), 8.10 (d, J = 2.0 Hz, 1H), 7.78 (dd, J = 8.8, 2.5 Hz, 1H), 7.72 (d, J = 1.9 Hz, 1H), 7.65 (dd, J = 8.5, 2.2 Hz, 1H), 6.77 - 6.66 (m, 3H), 4.12 - 3.98 (m, 1H), 3.92 (s, 3H), 3.91 (s, 2H), 3.87 - 3.74 (m, 6H), 3.62-3.58 (m, 4H), 2.73-2.67 (m, 1H), 1.67 (s, 3H). LC-MS [M+H]⁺ 522.6.

### Example 33

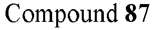

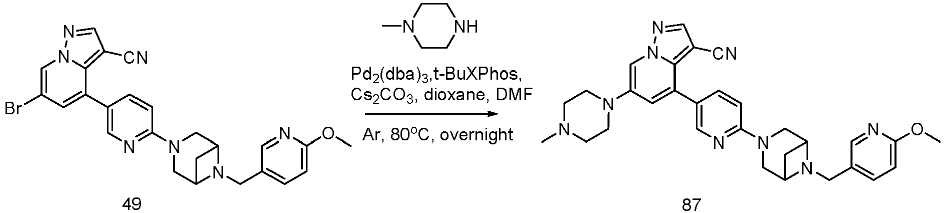

To a 25 mL sealed tube were added successively **49** (52 mg, 0.1 mmol), Pd₂(dba)₃ (5.5 mg, 0.006 mmol), *t*-BuXPhos (8.4 mg, 0.02 mmol), *N*-methylpiperazine (30 mg, 0.3 mmol), Cs₂CO₃ (65 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **87** (34 mg, 63.5% yield).
¹H NMR (400 MHz, CDCl₃) δ 8.40 (d, J = 2.2 Hz, 1H), 8.21 (s, 1H), 8.12 (d, J = 2.0 Hz, 1H), 8.04 (d, J = 2.0 Hz, 1H), 7.80 (dd, J = 8.8, 2.5 Hz, 1H), 7.66 (dd, J = 8.5, 2.4 Hz, 1H), 7.20 (d, J = 2.0 Hz, 1H), 6.72 (dd, J = 14.9, 8.6 Hz, 2H), 3.94 (s, 3H), 3.85 (d, J = 11.9 Hz, 2H), 3.81 (d, J = 5.8 Hz, 2H), 3.65-3.61 (m, 2H), 3.60 (s, 2H), 3.29 - 3.16 (m, 4H), 2.77 - 2.68 (m, 1H), 2.68 - 2.63 (m, 4H), 2.41 (s, 3H), 1.68 (d, J = 8.7 Hz, 1H). LC-MS [M+H]⁺535.6.

### Example 34

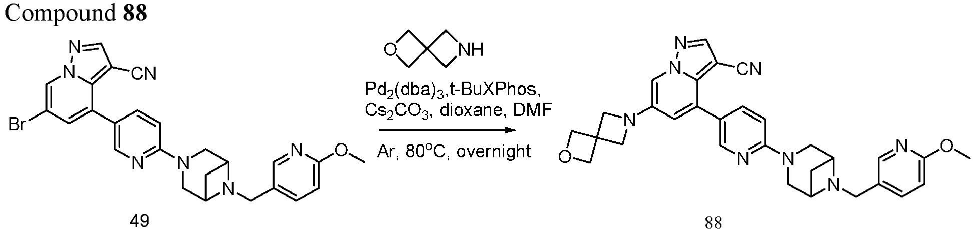

To a 25 mL sealed tube were added successively **49** (52 mg, 0.1 mmol), Pd₂(dba)₃ (5.5 mg, 0.006 mmol), *t*-BuXPhos (8.4 mg, 0.02 mmol), 2-oxa-6-aza-spiro[3,3]heptane (29.7 mg, 0.3 mmol), Cs₂CO₃ (65 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **88** (35.3 mg, 66% yield).
¹H NMR (400 MHz, CDCl₃) δ 8.37 (d, J = 2.2 Hz, 1H), 8.16 (s, 1H), 8.10 (d, J = 1.9 Hz, 1H), 7.78 (dd, J = 8.8, 2.5 Hz, 1H), 7.71 (d, J = 1.9 Hz, 1H), 7.66 (d, J = 6.9 Hz, 1H), 6.76 - 6.66 (m, 3H), 4.88 (s, 4H), 4.10 (s, 4H), 3.92 (s, 3H), 3.83 - 3.80 (m, 4H), 3.69 - 3.60 (m, 2H), 3.59 (s, 2H), 2.76-2.72 (m, 1H), 1.96-1.89 (m, 1H). LC-MS [M+H]⁺534.6.

### Example 35

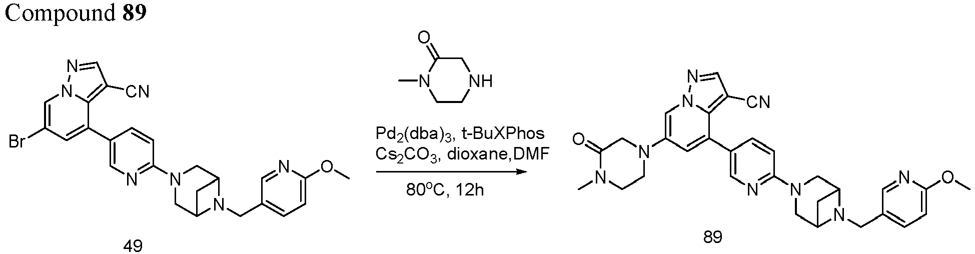

To a 25 mL sealed tube were added successively **49** (52 mg, 0.1 mmol), Pd₂(dba)₃ (5.5 mg, 0.006 mmol), *t*-BuXPhos (8.4 mg, 0.02 mmol), 1-methylpiperazin-2-one (34.2 mg, 0.3 mmol), Cs₂CO₃ (65 mg, 0.2 mmol), 1,4-dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **89** (50 mg, 90% yield).
¹H NMR (400 MHz, CDCl₃) δ 8.39 (d, J = 2.3 Hz, 1H), 8.21 (s, 1H), 8.10 (d, J = 2.0 Hz, 1H), 8.03 (d, J = 2.0 Hz, 1H), 7.78 (dd, J = 8.8, 2.5 Hz, 1H), 7.66 (dd, J = 8.5, 2.2 Hz, 1H), 7.14 (d, J = 2.0 Hz, 1H), 6.71 (dd, J = 11.8, 8.7 Hz, 2H), 3.92 (s, 3H), 3.87 (s, 2H), 3.85 - 3.75 (m, 4H), 3.75-3.60 (m, 4H), 3.57 - 3.47 (m, 4H), 3.07 (s, 3H), 2.72 (d, J = 7.1 Hz, 1H), 2.07-2.01 (m, 1H). LC-MS [M+H]⁺ 549.6.

### Example 36

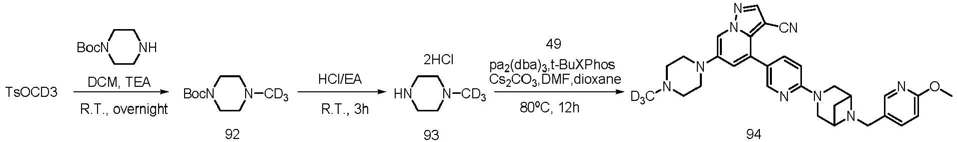

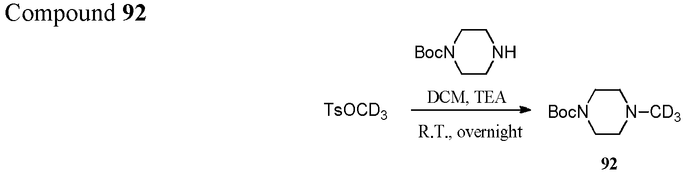

To a 25 mL single-neck eggplant-shaped flask were added successively methyl D₃-*p-*benzenesulfonate (500 mg, 2.64 mmol), 1-*tert*-butoxycarbonylpiperazine (327.8 mg, 1.76 mmol), triethylamine (534 mg, 5.28 mmol) and 1,4-dioxane (10 mL). The mixture was stirred at room temperature overnight, and TLC monitoring showed no starting material remained. The mixture was cooled to room temperature, and 10 mL of water was added. The mixture was stirred for 10 min, and the aqueous phase was separated and extracted with DCM (5 mL × 3). The organic phases were combined, dried over anhydrous sodium sulfate and concentrated to give a crude product as a colorless oil, which was purified by column chromatography to give product **92** as a colorless liquid (340 mg, 95% yield).
LC-MS [M+H]⁺204.17.

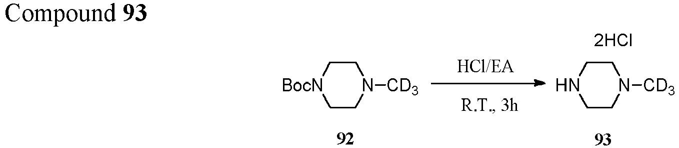

To a 25 mL single-neck eggplant-shaped flask was added a solution of hydrochloric acid in ethyl acetate (50 mL), and **92** (340 mg, 1.68 mmol) was slowly added dropwise. The mixture was stirred at room temperature for 3 h, and TLC monitoring showed no starting material remained. A white solid precipitated. The solid was collected by filtration and dried to give target product **93** as a white solid (296 mg, 100% yield).

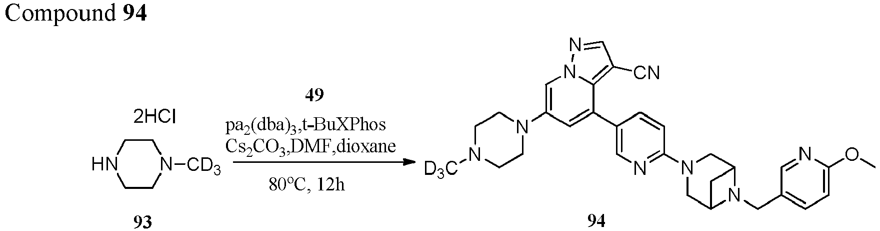

To a 25 mL sealed tube were added successively **49** (52 mg, 0.1 mmol), Pd₂(dba)₃ (5.5 mg, 0.006 mmol), t-BuXPhos (8.4 mg, 0.02 mmol), **93** (52.8 mg, 0.3 mmol), Cs₂CO₃ (65 mg, 0.2 mmol), dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **94** (42 mg, 78% yield).
¹H NMR (400 MHz, CDCl₃) δ 8.40 (d, J = 2.2 Hz, 1H), 8.21 (s, 1H), 8.12 (d, J = 2.0 Hz, 1H), 8.04 (d, J = 2.0 Hz, 1H), 7.80 (dd, J = 8.8, 2.5 Hz, 1H), 7.66 (dd, J = 8.5, 2.4 Hz, 1H), 7.20 (d, J = 2.0 Hz, 1H), 6.72 (dd, J = 14.9, 8.6 Hz, 2H), 3.94 (s, 3H), 3.85 (d, J = 11.9 Hz, 2H), 3.81 (d, J = 5.8 Hz, 2H), 3.65-3.61 (m, 2H), 3.60 (s, 2H), 3.29 - 3.16 (m, 4H), 2.77 - 2.68 (m, 1H), 2.68 - 2.63 (m, 4H), 1.68 (d, J = 8.7 Hz, 1H). LC-MS [M+H]⁺ 539.6.

### Example 37

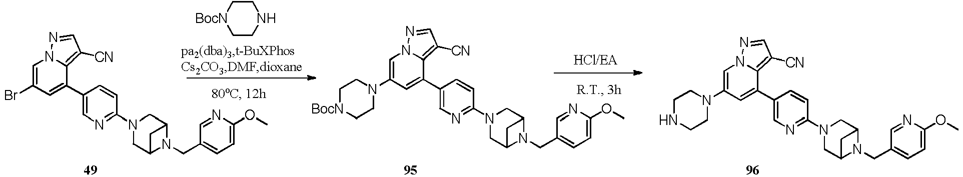

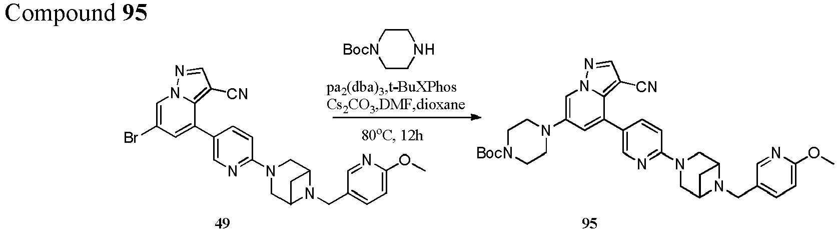

To a 25 mL sealed tube were added successively **49** (52 mg, 0.1 mmol), Pd₂(dba)₃ (5.5 mg, 0.006 mmol), *t*-BuXPhos (8.4 mg, 0.02 mmol), 1-*tert*-butoxycarbonylpiperazine (55.9 mg, 0.3 mmol), Cs₂CO₃ (65 mg, 0.2 mmol), dioxane (3 mL) and DMF (1 mL). The mixture was stirred at 80 °C overnight under Ar, and TLC monitoring showed no starting material **49** remained. The mixture was cooled to room temperature, and 10 mL of water was added. The mixture was stirred for 10 min, and a yellow solid precipitated. The solid was collected by filtration, dried and purified by column chromatography to give product **95** (53 mg, 88% yield).
LC-MS [M+H]⁺622.32.

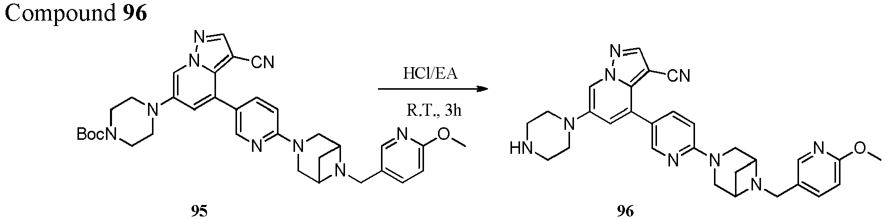

To a 25 mL single-neck eggplant-shaped flask was added a solution of hydrochloric acid in ethyl acetate (10 mL), and **95** (53 mg, 0.09 mmol) was slowly added dropwise. The mixture was stirred at room temperature for 3 h, and TLC monitoring showed no starting material remained. The mixture was concentrated, adjusted to pH 9 with ammonia water and extracted with DCM (3 mL × 3). The organic phases were combined, concentrated and purified by column chromatography to give the target product **96** as a yellow solid (36 mg, 78% yield).
¹H NMR (400 MHz, CDCl₃) δ 8.40 (d, J = 2.2 Hz, 1H), 8.21 (s, 1H), 8.12 (d, J = 2.0 Hz, 1H), 8.04 (d, J = 2.0 Hz, 1H), 7.80 (dd, J = 8.8, 2.5 Hz, 1H), 7.66 (dd, J = 8.5, 2.4 Hz, 1H), 7.20 (d, J = 2.0 Hz, 1H), 6.72 (dd, J = 14.9, 8.6 Hz, 2H), 3.94 (s, 3H), 3.85 (d, J = 11.9 Hz, 2H), 3.81 (d, J = 5.8 Hz, 2H), 3.65-3.61 (m, 2H), 3.60 (s, 2H), 3.29 - 3.16 (m, 4H), 2.77 - 2.68 (m, 1H), 2.68 - 2.63 (m, 4H), 1.68 (d, J = 8.7 Hz, 1H). LC-MS [M+H]⁺ 522.6.

### Example 38

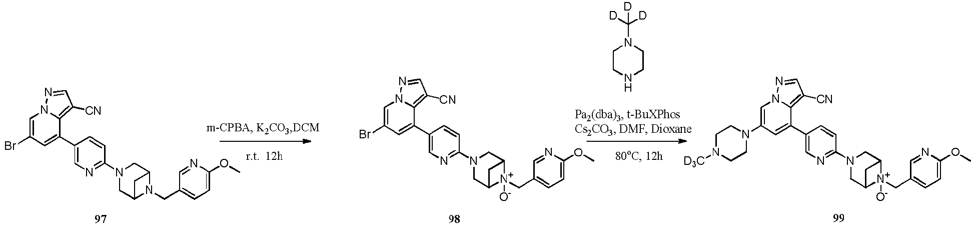

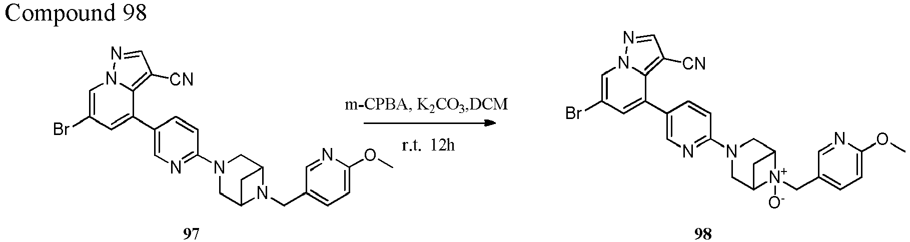

To a 25 mL single-neck flask were added successively **97** (500 mg, 0.97 mmol), K₂CO₃ (402 mg, 2.91 mmol) and DCM (50 mL). *m*-CPBA (200 mg, 1.16 mmol) was added in batches at 0 °C. The mixture was warmed to room temperature and stirred overnight, and TLC monitoring showed no starting material **97** remained. 50 mL of water was added, and the mixture was stirred for 10 min. The organic phase was separated, washed successively with H₂O and saturated NaCl solution, dried over anhydrous Na₂SO₄, concentrated and purified by column chromatography to give product **98** as a pale yellow solid (439 mg, 85% yield).

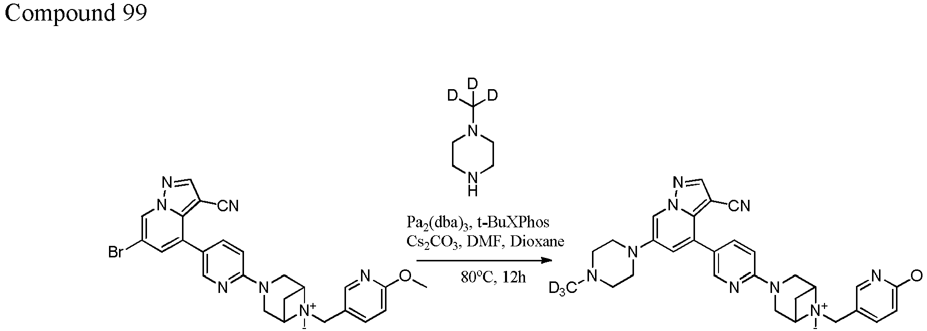

To a 25 mL sealed tube were added successively **98** (100 mg, 0.19 mmol), Pd₂(dba)₃ (10.4 mg, 0.01 mmol), *t*-BuXPhos (12.7 mg, 0.03 mmol), deuterated methylpiperazine (23.7 mg, 0.23 mmol), Cs₂CO₃ (187 mg, 0.57 mmol), dioxane (3 mL) and DMF (1 mL). The mixture was stirred at 80 °C overnight under Ar, and TLC monitoring showed no starting material **98** remained. The mixture was cooled to room temperature, and 10 mL of water was added. The mixture was stirred for 10 min, and a yellow solid precipitated. The solid was collected by filtration, dried and purified by column chromatography to give product **99** (79 mg, 75% yield). 1H NMR (400 MHz, CDCl3) δ 8.34 - 8.28 (m, 1H), 8.17 (d, J = 2.0 Hz, 1H), 8.12 (d, J = 2.1 Hz, 1H), 8.01 (s, 1H), 7.72 - 7.63 (m, 2H), 7.16 (d, J = 1.5 Hz, 1H), 6.76 (dd, J = 8.5, 3.2 Hz, 1H), 6.56 (dd, J = 41.0, 8.8 Hz, 1H), 4.07 - 3.88 (m, 6H), 3.84 - 3.57 (m, 5H), 3.49 (d, J = 4.3 Hz, 1H), 3.23 (t, 4H), 2.66 (t, 4H), 2.49 (d, J = 7.0 Hz, 1H).

### Example 39

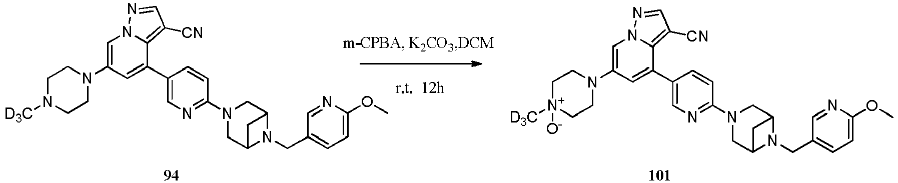

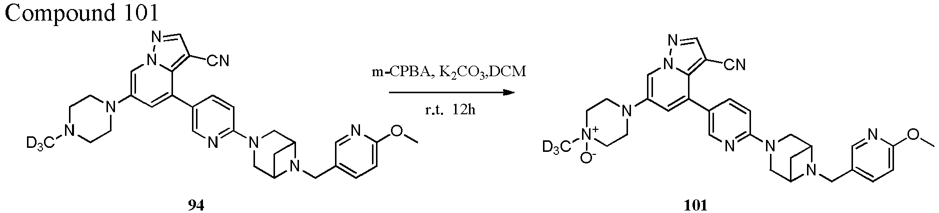

To a 25 mL single-neck flask were added successively **94** (100 mg, 0.18 mmol), K₂CO₃ (51.2 mg, 0.36 mmol) and DCM (20 mL). *m*-CPBA (37 mg, 0.22 mmol) was added in batches at 0 °C. The mixture was warmed to room temperature and stirred overnight, and TLC monitoring showed no starting material **94** remained. 20 mL of water was added, and the mixture was stirred for 10 min. The organic phase was separated, washed successively with H₂O and saturated NaCl solution, dried over anhydrous Na₂SO₄, concentrated and purified by column chromatography to give product **101** as a pale yellow solid (78 mg, 78% yield).
¹H NMR (400 MHz, DMSO) δ 8.55 (s, 1H), 8.49 - 8.34 (m, 2H), 8.07 (s, 1H), 7.85 (dd, J = 8.7, 2.2 Hz, 1H), 7.68 (dd, J = 8.5, 2.1 Hz, 1H), 7.55 (s, 1H), 6.78 (t, J = 8.2 Hz, 2H), 3.82 (s, 3H), 3.73 (d, J = 11.9 Hz, 2H), 3.68 (d, J = 5.6 Hz, 2H), 3.64 - 3.51 (m, 7H), 3.50 (s, 2H), 3.00 (s, 2H), 2.51 (s, 2H), 1.59 (d, J = 8.3 Hz, 1H).

### Activity Experiment 1: Inhibition of RET Family Kinases' Activity by Prepared Compounds

The compounds were assayed for activity IC₅₀ against kinases RET wild type (RET WT), RET (V804M), RET (M918T) point mutant CCDC6-RET fusion mutations and KDR (VEGFR2). The above kinases were purchased from Thermo Fisher Scientific and ProQinase GmbH.

The activity assay method for the above kinases was established by homogeneous time-resolved fluorescence (HTRF), and the inhibitory activity of the compounds was determined. An 8 µL reaction solution was prepared, comprising 1× enzymatic buffer (Cisbio, HTRF KinEASE^{™}-TK), 5 mM MgCl₂, 1 mM MnCl₂, 1 mM DTT, 1 µM TK substrate-biotin (Cisbio, HTRF KinEASETM-TK), 10 µM ATP (1 µM for RET WT, CCDC6-RET; 20 µM for KDR), gradient concentrations of compounds and the following concentrations of related kinases: 0.03 ng/µL RET WT, 0.2 ng/µL RET (V804M), 0.04 ng/µL RET (M918T), 0.12 ng/µL CCDC6-RET and 0.02 ng/µL KDR. The kinases and compounds were pre-incubated for 5 min, and then ATP and substrate were added to start the reaction. All the enzyme-catalyzed reactions were carried out at 25 °C for 60 min. After the enzyme-catalyzed reactions were completed, 4 µL of TK antibody-cryptate and 4 µL of streptavidin-XL665 (the reaction concentration was 62.5 nM) were added to the reaction mixtures, and the mixtures were incubated at 25 °C for another 60 min. After the incubation was completed, the HTRF fluorescence values were determined on CLARIOstar (BMG LABTECH), and IC₅₀ was calculated using the GraphPad Prism 5.0 software.

**Table 1. In Vitro Enzymatic Activity Assay Data (IC₅₀, nM)**

| **Compound ID** | **RET WT** | **RET(V804M)** | **RET(M918T)** | **CCDC6-RET** | **KDR** |
|---|---|---|---|---|---|
| **16** | 8.40 | 4.78 | 2.42 | 3.65 | 405.70 |
| **17** | 5.50 | 3.82 | 1.70 | 2.61 | 551.30 |
| **20** | 4.10 | 3.23 | 1.45 | 2.14 | 339.70 |
| **17-a** | 27.93 | 127.80 | 48.40 | 17.16 | 122.20 |
| **21** | 45.00 | 188.00 | 80.00 | 60.45 | 108.90 |
| **32** | 2.11 | 4.80 | 1.73 | 3.13 | 39.11 |
| **34** | 35.79 | 209.60 | 97.76 | 71.57 | 311.00 |
| **39** | 98.29 | 860.50 | 325.80 | N.D. | N.D. |
| **43** | 30.89 | 241.90 | 53.15 | N.D. | N.D. |
| **51** | 4.61 | 7.16 | 3.90 | 2.15 | 51.99 |
| **55** | 12.35 | 39.66 | 15.32 | 7.45 | 222.90 |
| **57** | 2.24 | 4.33 | 4.06 | 4.44 | 7.40 |
| **58** | 4.98 | 19.35 | 9.52 | 8.07 | 134.10 |
| **60** | 12.56 | 26.34 | 10.73 | 6.01 | 185.6 |
| **64** | 26.55 | 110.80 | 42.80 | 30.74 | 386.60 |
| **65** | 1.09 | 3.37 | 0.92 | 0.52 | 92.84 |
| **66** | 1.66 | 7.69 | 2.05 | 1.13 | 123.50 |
| **67** | 0.79 | 1.09 | 0.75 | 0.34 | 24.63 |
| **68** | 1.30 | 5.37 | 8.47 | 2.54 | 16.60 |
| **69** | 18.60 | 38.54 | 22.30 | 11.99 | 754.50 |
| **70** | 1.17 | 1.01 | 1.62 | 1.02 | 23.88 |
| **78** | 1.28 | 8.23 | 5.47 | 4.60 | 51.91 |
| **79** | 11.81 | 45.20 | 4.77 | N.D. | N.D. |
| **80** | 21.60 | N.D. | N.D. | N.D. | 565.96 |
| **81** | 193.10 | N.D. | N.D. | N.D. | 612.50 |
| **82** | 10.73 | N.D. | N.D. | N.D. | 280.40 |
| **83** | 15.81 | N.D. | N.D. | N.D. | N.D. |
| **84** | 24.81 | N.D. | N.D. | N.D. | N.D. |
| **85** | 19.04 | N.D. | N.D. | N.D. | N.D. |
| **86** | 4.56 | N.D. | N.D. | N.D. | 71.26 |
| **87** | 2.26 | 7.40 | 2.78 | 3.00 | 154.20 |
| **88** | 7.22 | N.D. | N.D. | N.D. | 96.93 |
| **89** | 0.674 | 4.719 | 1.471 | 3.045 | 156.90 |
| **94** | 4.625 | N.D. | N.D. | N.D. | 169.10 |
| **96** | 5.238 | N.D. | N.D. | N.D. | 239.60 |
| **99** | 56.6 | N.D. | N.D. | N.D. | N.D. |
| **101** | 2.5 | N.D. | N.D. | N.D. | 215.2 |
| Selpercatinib | 1.98 | 5.09 | 2.17 | 2.07 | 172.00 |

| | | | | | |
|---|---|---|---|---|---|
| N.D. - not detected | | | | | |

### Activity Experiment 2: Research Report on Metabolic Stability of Prepared Compounds in Human Liver Microsomes

The rate and extent of metabolism of compounds in liver microsomes under the action of NADPH-reducing coenzymes were investigated by liquid chromatography-mass spectrometry (LC-MS/MS). 445 µL of 0.562 mg/mL human liver microsome working solution was added to a corresponding 96-well plate and pre-incubated in a 37 °C water bath for 10.0 min. A reaction was then started by adding 5.00 µL of 100 µM compound and 50.0 µL of 10.0 mM NADPH. After 0 min, 2 min, 5 min, 10 min, 20 min, 30 min and 60 min, 50.0 µL of reaction mixture was added to 400 µL of glacial acetonitrile (I02) containing 50.0 ng/mL internal standard (tolbutamide) to terminate the reaction. For a no-co-factor sample (NCF), 445 µL of working solution of microsome of each species was well mixed with 50.0 µL of 10 mM MgCl₂, and finally 5.00 µL of test sample working solution was added. The mixture was well mixed and incubated at 37 °C for 10 min. After time is up, 50.0 µL of reaction mixture was added to 400 µL of ice cold (I02) to terminate the reaction. The mixture was well mixed by vortexing and centrifuged at 1700× g at 4 °C for 15 min. 150 µL of supernatant was collected, diluted by adding 150 µL of ultrapure water and subjected to LC-MS/MS analysis. The results are shown in Table 2 and Table 3.

**Table 2. The half-lives and intrinsic clearances of the compounds in human liver microsomes**

| **Compound ID** | **Human Liver Microsomes** | |
|---|---|---|
| | **T_{1/2} (min)** | **CLᵢₙₜ (µL/min/mg)** |
| **16** | 157.5 | 8.8 |
| **17** | 38.1 | 36.4 |
| **20** | 231.0 | 6.0 |
| Selpercatinib | 21.7 | 63.8 |

**Table 3. The half-lives and intrinsic clearance of the compounds in human liver microsomes**

| **Compound ID** | **Human Liver Microsomes** | |
|---|---|---|
| | **T_{1/2} (min)** | **CLᵢₙₜ (µL/min/mg)** |
| **57** | 19.9 | 69.8 |
| **65** | 30.9 | 44.8 |
| **70** | 27.4 | 50.6 |
| **87** | 38.7 | 35.8 |
| **94** | 33.6 | 41.2 |
| Selpercatinib | 22.9 | 60.4 |

### Activity Experiment 3: Inhibition of Ba/F3 KIF5B-RET Cells by Prepared Compounds

Ba/F3 KIF5B-RET, Ba/F3KIF5B-RET-V804M, Ba/F3 RET-M918T and Ba/F3 KIF5B-RET-G810R cells growing in log phase were harvested and counted using a platelet counter. The cell viability was determined by the trypan blue exclusion method, and the cell viability was kept over 90%. The cell concentration was adjusted, and 90 µL of the cell suspension was added to a 96-well plate. The cells in the 96-well plate were incubated overnight at 37 °C with 5% CO₂ and 95% humidity. Corresponding gradient concentrations of drug solutions (the maximum concentration was 1000 nM) were added to the 96-well plate inoculated with cells at 10 µL/well. Triplicate wells were set per drug concentration, and the final concentration of DMSO was 0.1%. The drug-treated cells in the 96-well plate were incubated for another 72 h at 37 °C with 5% CO₂ and 95% humidity. After the drug treatment was completed, 100 µL of CellTiter-Glo reagent was added to each well. The cell plate was shaken on an orbital shaker for 5 min to lyse the cells and then let stand at room temperature for 20 min to stabilize the luminescence signals, and then the luminescence values were read. The data were analyzed using the GraphPad Prism 5.0 software. Dose-response curves were fit to the data using nonlinear S-curve regression, and IC₅₀ was calculated from the curves. The results are shown in Table 4.

**Table 4. In vitro cell level activity assay data (IC₅₀, nM)**

| **Compound ID** | **Ba/F3 KIF5B-RET** | **Ba/F3 KIF5B-RET-V804M** | **Ba/F3 RET-M918T** | **Ba/F3 KIF5BRET-G810R** |
|---|---|---|---|---|
| **65** | 8.06 | 58.64 | 23.85 | N.D. |
| **66** | 9.17 | N.D. | N.D. | N.D. |
| **67** | 3.58 | N.D. | N.D. | N.D. |
| **68** | 2.00 | N.D. | N.D. | N.D. |
| **70** | 0.99 | 11.91 | 1.15 | 194.40 |
| **78** | 13.60 | N.D. | N.D. | N.D. |
| **82** | 17.10 | N.D. | N.D. | N.D. |
| **86** | 3.66 | N.D. | N.D. | N.D. |
| **87** | 11.78 | 115.87 | 13.45 | 431.10 |
| **89** | 24.00 | 95.30 | 11.60 | 958.70 |
| **90** | 2.39 | 10.57 | 1.00 | 162.40 |
| **91** | 2.43 | 8.90 | 0.90 | 275.30 |
| **96** | 41.80 | 195.50 | 45.10 | N.D. |
| **94** | 19.80 | 99.00 | 17.10 | 463.90 |
| BLU-667 | 11.35 | 11.05 | 12.93 | 480.90 |
| Selpercatinib | 8.34 | 38.42 | 20.35 | >1000 |

The structural formula of BLU-667 is shown below:

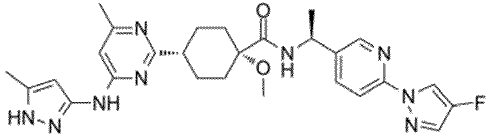

The structural formula of selpercatinib (Loxo-292) is shown below:

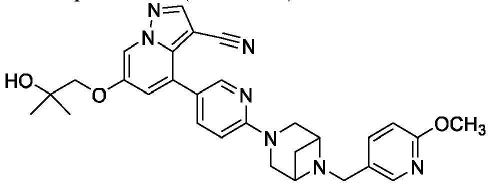

### Activity Experiment 4: Inhibition of hERG Potassium Channel by Prepared Compounds

The final concentrations of test compounds were all prepared on the day of experiment and then dissolved in an extracellular fluid. The extracellular fluid (mM) was: NaCl, 137; KCl, 4; CaCl₂, 1.8; MgCl₂, 1; HEPES, 10; glucose 10; pH 7.4 (NaOH titration). All the test and control compound solutions contained 0.3% DMSO. HEK293 cells stably expressing the hERG ion channel were transferred to a perfusion chamber and perfusion was carried out using the extracellular fluid. A intracellular fluid was stored in small batches in a -80 °C freezer and thawed the day of experiment. The intracellular fluid (mM) was: K Aspartate, 130; MgCl₂, 5; EGTA 5; HEPES, 10; Tris-ATP 4; pH 7.2 (KOH titration). Electrodes were produced by pulling with PC-10 (Narishige, Japan). Whole-cell patch-clamp recording was carried out. Noise was filtered at one fifth of the sampling frequency.

The cells were clamped at -80 mV, then depolarized to 40 mV with a 4 second lasting square wave, and hyperpolarized to -40 mV with a 2 second lasting square wave to give a hERG tail current. This procedure was repeated every 20 seconds. The hERG tail current was a pure hERG current. The maximum current induced by the second square wave was detected, and after it was stable, perfusion was carried out using the test compounds. After the reaction was stable, the blocking intensity was calculated. The IC₅₀ values for the inhibition of the hERG channel by the compounds were calculated from the blocking intensity. The results are shown in Table 5.

**Table 5. IC₅₀ (µM) for inhibition of hERG potassium channel by compounds at cellular level**

| **Compound ID** | **IC₅₀** |
|---|---|
| **16** | 2.06 |
| **17** | 1.31 |
| **20** | 22.2 |
| **65** | 2.72 |
| **70** | 4.74 |
| **87** | 2.13 |
| **89** | 7.17 |
| Selpercatinib | 2.75 |

### Activity Experiment 5: Pharmacokinetic Assay in Rats

SD male rats (weighing 220 ± 20 g) were administered **compound 94** and Loxo-292 at a dose of 1.0 mg/kg by tail vein injection and at a dose of 5.0 mg/kg orally. Each group consisted of 3 animals. The solvent for administration was a solution of 5% DMSO + 5% polyethoxylated castor oil (Cremophor EL) in normal saline. The rats were fasted for about 12 h before administration and were given *ad libitum* access to food and water 4 h after administration. Blood was collected from the orbit at about 0.2 mL before administration and 5 min, 15 min, 30 min, 1 h, 2 h, 4 h, 6 h, 8 h, 10 h and 24 h after administration, placed in EDTA-K₂ anticoagulant EP tubes in an ice bath, and centrifuged at 4 °C at 8000 rpm for 5 min to isolate plasma, which was stored at -20 °C before analysis. The concentration of compound in the plasma was quantified by liquid chromatography-tandem mass spectrometry (LC-MS/MS). Pharmacokinetic parameters were calculated from the analysis results of the samples using WinNonlin5.2.

**Table 6. Pharmacokinetic parameters**

| **Parameter** | **Unit** | **Compound 94** | | **Loxo-292** | |
|---|---|---|---|---|---|
| | | i.v. 1 mg/kg | p.o. 5 mg/kg | i.v. 1 mg/kg | p.o. 5 mg/kg |
| T_{1/2} | h | 13.26 | 15.76 | 3.62 | 3.11 |
| Tₘₐₓ | h | 0.08 | 4.00 | 0.083 | 2.67 |
| Cₘₐₓ | ng/mL | 42.30 | 63.93 | 1257 | 1039 |
| AUC₀₋ₜ | hr^{∗}ng/mL | 240.05 | 1022.96 | 3314 | 10922 |
| AUC_{0-inf} | hr^{∗}ng/mL | 335.44 | 1644.08 | 3333 | 11010 |
| Vz | mL/kg | 57620.00 | 68492.14 | 1613 | 2039 |
| CL | mL/hr/kg | 3071.40 | 3567.58 | 306 | 456 |
| F | % | 98.02 | | 66.07 | |

As can be seen from the data in Table 6, after oral administration, **compound 94** took longer to be cleared in rats and had higher bioavailability than Loxo-292.

### Activity Experiment 6: Tissue Distribution Assay in Mice

Male NVSG mice (weighing 20-25 g) were orally administered **compound 94** and Loxo-292 at a dose of 30 mg/kg. The plasma and tissue samples of the liver, the brain and the lungs and the like of the animals are collected 4 h after administration. Blood plasma collection: The whole blood was collected at about 80-100 µL in EDTA-containing EP tubes and centrifuged at 4000 g for 5 min, and then the upper plasma was collected and stored at -80 °C. Tissue collection: After the animals were euthanized, the tissues were collected and snap-frozen in liquid nitrogen, 5 mL of homogenization solution (50% acetonitrile) was added per gram of tissue. The tissues were processed into homogenates in a tissue homogenizer and stored at - 80 °C. The concentration of compound in the plasma and tissue samples was quantified by liquid chromatography-tandem mass spectrometry (LC-MS/MS).

**Table 7. Concentration distribution of compounds in mouse plasma and organ tissues**

| Tissue | Concentration of compound in tissue/plasma (ng/g or ng/mL) | |
|---|---|---|
| | **Compound 94** | **Loxo-292** |
| Liver | 70199.89 | 12859.19 |
| Lung | 87220.49 | 7186.38 |
| Brain | 2883.99 | 88.26 |
| Plasma | 3174.97 | 14631.15 |

As can be seen from the data in Table 7, after oral administration, compound 94 had a higher concentration distribution in the tissues of the target organs of mice, which is more favorable for the exertion of drug effects in the target organs.

### Activity Experiment 7: Mouse Subcutaneous BA/F3 KIF5B-RET Graft Tumor Model Assay

BA/F3 KIF5B-RET cells were cultured in RPMI-1640 medium supplemented with 10% FBS in a 5% CO₂, 37 °C incubator. NVSG mice were each inoculated subcutaneously with about 1 × 10⁶ cells in a volume of 100 µL on the right shoulder by subcutaneous injection. When the volume of graft tumors reached about 100 mm³, mice with properly sized graft tumors were selected and randomly grouped according to body weight and size of graft tumor. The drugs were administered by intragastric administration in a volume of 10 µL/g body weight. Loxo-292 was administered at a dose of 30 mg/kg twice daily (b.i.d.); compound **94** was administered at a dose of 30 mg/kg twice daily (b.i.d.) and at doses of 30 mg/kg and 60 mg/kg once daily (q.d.); a solvent control group was administered a solution of 2% DMSO + 2% polyethoxylated castor oil in normal saline. The drugs were administered for 15 consecutive days, and the volume of graft tumors was measured twice a week. Day 0 refers to the first day of grouping and administration.

**Table 8. Inhibitory effects of compounds on BA/F3 KIF5B-RET mouse graft tumor model**

| Group | Volume of graft tumor (mm³, mean measurement ± SD) | | |
|---|---|---|---|
| | Day0 | Day 11 | Day 15 |
| Solvent control group | 96.31±7.77 | 1101.69±367.87 | N.A.* |
| **Compound 94** 30 mg/kg b.i.d. | 96.85±2.24 | 12.19±27.25 | 8.00±17.89 |
| **Compound 94** 30 mg/kg q.d. | 96.95±1.48 | 14.57±23.42 | 9.73±16.31 |
| **Compound 94** 60 mg/kg q.d. | 96.76±2.34 | 6.11±14.98 | 3.75±9.19 |
| Loxo-292 30mg/kg b.i.d. | 96.89±2.07 | 19.68±17.97 | 13.59±12.43 |

| | | | |
|---|---|---|---|
| *On day 15, all the solvent control animals died, and thus there were no measurements. | | | |

As can be seen from the data in Table 8, after 15 days of administration, the dose groups of compound **94** all had smaller mouse graft tumors than the Loxo-292 groups, showing superior inhibitory activity against tumors; in addition, compound **94** demonstrated good inhibitory activity against tumors even when administered once daily.

### Activity Experiment 8: Inhibition of FGFR Family Kinases' Activity by Prepared Compounds

The compounds were assayed for activity IC₅₀ against the FGFR family kinases. Related kinases were purchased from Carna and Signalchem.

The activity assay method for the related kinases was established by homogeneous time-resolved fluorescence (HTRF), and the inhibitory activity of the compounds was determined. A 5 µL reaction mixture was prepared, comprising 1× enzymatic buffer (Cisbio, HTRF KinEASE^{™}-TK), 5 mM MgCl₂, 1 mM DTT, 1 µM TK substrate-biotin (Cisbio, HTRF KinEASE^{™}-TK), corresponding concentrations of ATP and related kinases, and gradient concentrations of compounds. The concentrations of the related kinases and the corresponding ATP concentrations are shown in Table 9 below:

**Table 9. Kinase concentrations and corresponding ATP concentrations**

| Name of kinase | Reaction concentration of kinase | Reaction concentration of ATP |
|---|---|---|
| FGFR1 | 0.02ng/ul | 50 |
| FGFR1 V561M | 0.04ng/ul | 5 |
| FGFR2 | 0.005ng/ul | 50 |
| FGFR2 V564F | 0.02ng/ul | 10 |
| FGFR2 N549H | 0.02ng/ul | 5 |
| FGFR2 V564I | 0.04ng/ul | 5 |
| FGFR2 K641R | 0.02ng/ul | 1 |
| FGFR3 | 0.02ng/ul | 50 |
| FGFR3 V555M | 0.02ng/ul | 20 |
| FGFR3 K650E | 0.04ng/ul | 50 |
| FGFR4 | 5nM | 50 |

The kinases and compounds were pre-incubated for 10 min, and then ATP and substrate were added to start the reaction. All the enzyme-catalyzed reactions were carried out at 25 °C for 40 min. After the enzyme-catalyzed reactions were completed, 5 µL of TK antibody-cryptate and streptavidin-XL665 (the reaction concentration of streptavidin-XL665 was 62.5 nM) were added to the reaction mixtures, and the mixtures were incubated at 25 °C for another 60 min. After the incubation was completed, the fluorescence signals at 615 nm (cryptate) and 665 nm (XI,665) were read on Biotek, and IC₅₀ was calculated using the GraphPad Prism 5.0 software.

**Table 10. Inhibition of the compound on activity of FGFR family kinases (IC₅₀, nM)**

| | Compound 94 | Loxo-292 |
|---|---|---|
| FGFR1 | 300.6 | 303.9 |
| FGFR1 V561M | 1397 | 1954 |
| FGFR2 | 26.49 | 20.6 |
| FGFR2 V564F | 3.576 | 12.94 |
| FGFR2 N549H | 2.188 | 9.262 |
| FGFR2 V564I | 45.63 | 97.56 |
| FGFR2 K641R | 7.393 | 11.25 |
| FGFR3 | 308.6 | 348.8 |
| FGFR3 V555M | 74.29 | 188.3 |
| FGFR3 K650E | 36.47 | 45.89 |
| FGFR4 | 595.4 | 474.9 |

The data in Table 10 above indicate that compound 94 had an inhibitory effect on the activity of the FGFR family kinases, and that compound 94 had better inhibitory activity against kinases FGFR2 V564F, FGFR2 N549H, FGFR2 V564I and FGFR3 V555M than LOXO-292 (IC₅₀ was a factor of 2 or more smaller).

## Claims

1. A compound of formula (I) or a pharmaceutically acceptable salt, ester, stereoisomer, solvate, oxide or prodrug thereof, 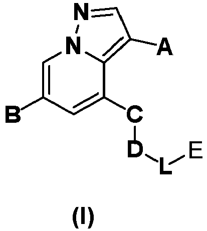 wherein,
A is selected from H, -CN, halogen, -C=ONH₂, -C=C-CN and -C≡CH;
B is selected from the following groups unsubstituted or substituted with one or more identical or different substituents: C1-C6 alkyl, C1-C6 alkylamino, C2-C6 alkynyl, C2-C6 alkenyl, HetAr¹ and HetCyc¹; the substituents are independently selected from halogen, hydroxy, -CN, =O (carbonyl), C1-C6 alkyl, deuterated C1-C6 alkyl, C1-C6 alkoxy, hydroxy C1-C6 alkyl, halo C1-C6 alkyl, cyano C1-C6 alkyl, (C1-C6 alkoxy) C1-C6 alkyl, C3-C6 cycloalkyl and (C1-C6 alkoxy SO₂) C1-C6 alkyl;
HetAr¹ is a 5- to 6-membered heteroaromatic ring having 1-3 heteroatoms independently selected from N, S and O;
HetCyc¹ is a 4- to 8-membered heterocycle having 1-3 heteroatoms selected from N and O, an 8- to 10-membered spiro ring having 1-3 heteroatoms selected from N and O or a 7- to 11-membered fused heterocycle having 1-3 heteroatoms selected from N and O;
C is a 5- to 6-membered heteroaromatic ring having 1-3 heteroatoms independently selected from N, S and O, wherein the heteroaromatic ring is unsubstituted or is optionally substituted with one or more identical or different substituents independently selected from halogen, hydroxy, -CN, nitro, C1-C3 alkyl and halo C1-C3 alkyl;
D is a C1-C6 alkyl having 1-3 heteroatoms selected from N and O, a 4- to 8-membered heterocycle having 1-3 heteroatoms selected from N and O, a 7- to 8-membered bridged ring having 1-3 heteroatoms selected from N and O, a 7- to 11-membered spiro ring having 1-3 heteroatoms selected from N and O, a 7- to 10-membered fused heterocycle having 1-3 heteroatoms selected from N and O, 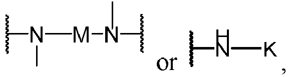 wherein M is selected from C1-C3 alkyl and C3-C8 cycloalkyl; K is a 4- to 8-membered heterocycle having 1-3 heteroatoms selected from N and O;
L is 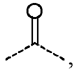 -C(=O)-C1-C3 alkyl or C1-C3 alkyl;
E is HetAr² unsubstituted or substituted with one or more identical or different substituents; the substituents are independently selected from halogen, C1-C6 alkyl, deuterated C1-C6 alkyl, C1-C6 alkoxy, deuterated C1-C6 alkoxy, hydroxy C1-C6 alkyl, C1-C6 haloalkyl, cyano C1-C6 alkyl, (C1-C6 alkoxy) C1-C6 alkyl, C3-C6 cycloalkyl and (C1-C6 alkoxy SO₂) C1-C6 alkyl;
HetAr² is a 5- to 6-membered heteroaromatic ring having 1-3 ring heteroatoms independently selected from N, S and O.

2. The compound of formula (I) or the pharmaceutically acceptable salt, ester, stereoisomer, solvate, oxide or prodrug thereof according to claim 1, wherein A is selected from -CN, -C=C-CN and -C≡CH; preferably, A is -CN.

3. The compound of formula (I) or the pharmaceutically acceptable salt, ester, stereoisomer, solvate, oxide or prodrug thereof according to claim 1, wherein B is selected from the following groups unsubstituted or substituted with one or two identical or different substituents: 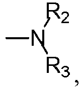 R₁-C≡CH and HetCyc¹; R₁ is selected from H, C1-C6 alkyl, deuterated C1-C6 alkyl and C1-C6 hydroxyalkyl; R₂ or R₃ is independently selected from H, C1-C6 alkyl, deuterated C1-C6 alkyl and C1-C6 hydroxyalkyl; the substituents are independently selected from halogen, hydroxy, -CN, carbonyl, C1-C3 alkyl, deuterated C1-C3 alkyl, C1-C3 alkoxy, hydroxy C1-C3 alkyl, C1-C3 fluoroalkyl, cyano C1-C3 alkyl, (C1-C3 alkoxy) C1-C3 alkyl, C3-C6 cycloalkyl and (C1-C3 alkoxy SO₂) C1-C3 alkyl;
HetCyc¹ is a 4- to 8-membered heterocycle having 1-2 heteroatoms selected from N and O, a 7- to 11-membered spiro ring having 1-2 heteroatoms selected from N and O or an 8- to 10-membered fused heterocycle having 1-2 heteroatoms selected from N and O;
preferably, B is selected from the following groups unsubstituted or substituted with one or two identical or different substituents: R₁-C≡CH, 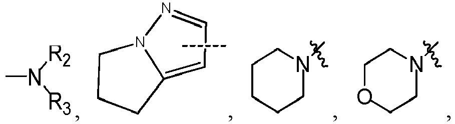 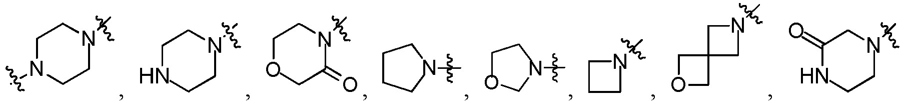 and 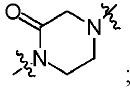 R₁ is selected from C1-C4 alkyl and C1-C4 hydroxyalkyl; R₂ or R₃ is independently selected from H, C1-C4 alkyl, deuterated C1-C4 alkyl and C1-C4 hydroxyalkyl; the substituents are independently selected from hydroxy, cyano, halogen, C1-C3 alkyl, deuterated C1-C3 alkyl, C1-C3 alkoxy and C3-C6 cycloalkyl.

4. The compound of formula (I) or the pharmaceutically acceptable salt, ester, stereoisomer, solvate, oxide or prodrug thereof according to claim 1, wherein B is 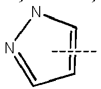 unsubstituted or substituted with one or two identical or different substituents; the substituents are independently selected from halogen, hydroxy, -CN, carbonyl, C1-C3 alkyl, deuterated C1-C3 alkyl, C1-C3 alkoxy, hydroxy C1-C3 alkyl, C1-C3 fluoroalkyl, cyano C1-C3 alkyl, (C1-C3 alkoxy) C1-C3 alkyl, C3-C6 cycloalkyl and (C1-C3 alkoxy SO₂) C1-C3 alkyl C1-C3 alkyl; C is a 5- to 6-membered heteroaromatic ring having 1-2 ring heteroatoms selected from N and S; D is 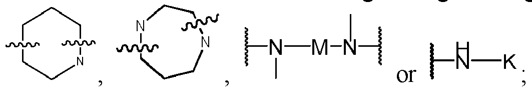 wherein M is selected from C3-C6 cycloalkyl; K is a 4- to 8-membered heterocycle having 1-3 heteroatoms selected from N and O; L is -CH₂-; E is

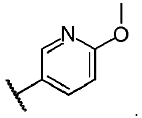

5. The compound of formula (I) or the pharmaceutically acceptable salt, ester, stereoisomer, solvate, oxide or prodrug thereof according to claim 1, wherein C is the following group unsubstituted or substituted with one or two identical or different substituents: 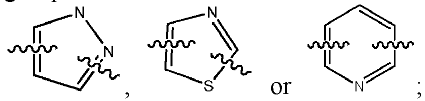 the substituents are independently selected from fluorine, chlorine and bromine.

6. The compound of formula (I) or the pharmaceutically acceptable salt, ester, stereoisomer, solvate, oxide or prodrug thereof according to claim 1, wherein D is 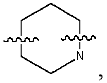 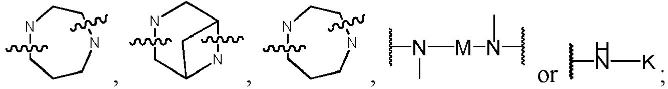 wherein M is selected from C1-C3 alkane and C3-C6 cycloalkyl; K is a 4- to 8-membered heterocycle having 1-3 heteroatoms selected from N and O; preferably, D is -N(CH₃)CH₂CH₂N(CH₃)-,

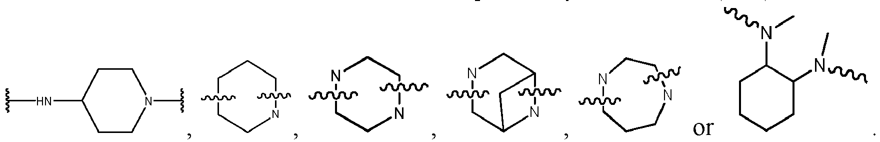

7. The compound of formula (I) or the pharmaceutically acceptable salt, ester, stereoisomer, solvate, oxide or prodrug thereof according to claim 1, wherein L is 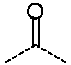 or - CH₂-.

8. The compound of formula (I) or the pharmaceutically acceptable salt, ester, stereoisomer, solvate, oxide or prodrug thereof according to claim 1, wherein E is 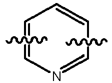 unsubstituted or substituted with one or two identical or different substituents; the substituents are independently selected from C1-C3 alkoxy and deuterated C1-C3 alkoxy.

9. A compound of formula (II) or a pharmaceutically acceptable salt, ester, stereoisomer, solvate, oxide or prodrug thereof, wherein 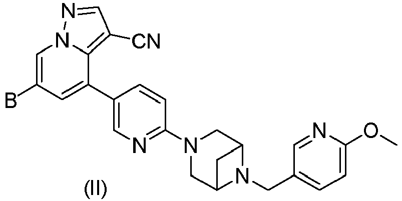
B is selected from HetCyc¹ unsubstituted or substituted with one or two identical or different substituents; HetCyc¹ is a 4- to 5-membered heterocycle having 1 N atom or 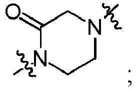 the substituents are independently selected from H, halogen, hydroxy, -CN, carbonyl, C1-C3 alkyl, deuterated C1-C3 alkyl, C1-C3 alkoxy, hydroxy C1-C3 alkyl, C1-C3 fluoroalkyl and cyano C1-C3 alkyl; preferably, the substituents are independently selected from halogen, hydroxy, -CN, carbonyl, methyl, ethyl, deuterated methyl and methoxy;
or, B is selected from HetCyc¹ unsubstituted or substituted with one or two identical or different substituents; HetCyc¹ is a 6-membered heterocycle having 1-2 N atoms; the substituents are independently selected from H, C1-C3 alkyl, deuterated C1-C3 alkyl and C1-C3 fluoroalkyl;
preferably, HetCyc¹ is 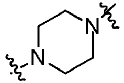 unsubstituted or substituted with substituents; the substituents of B are independently selected from halogen, methyl, ethyl, deuterated methyl and -CF₃;
or, B is substituted or unsubstituted HetCyc², or B is a substituted or unsubstituted 7- to 8-membered bridged ring having 1-3 heteroatoms selected from N, S and O; HetCyc² is a 4- to 6-membered heterocycle having a P atom or a 4- to 6-membered heterocycle having a P atom and a N atom; the substituents are independently selected from H, halogen, hydroxy, -CN, carbonyl, C1-C3 alkyl, deuterated C1-C3 alkyl, C1-C3 alkoxy, hydroxy C1-C3 alkyl, C1-C3 fluoroalkyl and cyano C1-C3 alkyl; preferably, B is substituted or unsubstituted HetCyc², or B is a substituted or unsubstituted 7- to 8-membered bridged ring having 1-2 heteroatoms including N; HetCyc² is a 4- to 6-membered heterocycle having a P atom and a N atom; preferably, B is substituted or unsubstituted 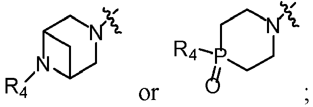 wherein R₄ is selected from H, halogen, hydroxy, -CN, carbonyl, C1-C3 alkyl, deuterated C1-C3 alkyl, C1-C3 alkoxy, hydroxy C1-C3 alkyl, C1-C3 fluoroalkyl and cyano C1-C3 alkyl; preferably, R₄ is selected from H, halogen, hydroxy, -CN, carbonyl, methyl, ethyl, deuterated methyl, methoxy or trifluoromethyl; preferably, the substituents are independently selected from halogen, hydroxy, -CN, carbonyl, methyl, ethyl, deuterated methyl, methoxy and trifluoromethyl.

10. The compound or the pharmaceutically acceptable salt, ester, stereoisomer, solvate, oxide or prodrug thereof according to any one of claims 1-9, wherein the oxide is formed by oxidation at a N atom of a 4- to 8-membered heterocycle or bridged ring having a N heteroatom, preferably at a nitrogen atom of

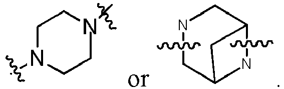

11. A compound or a pharmaceutically acceptable salt, ester, stereoisomer, solvate or prodrug thereof, selected from:

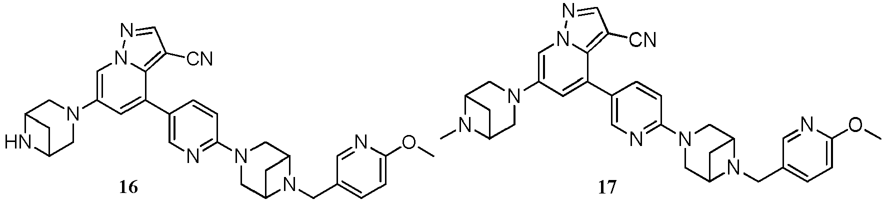

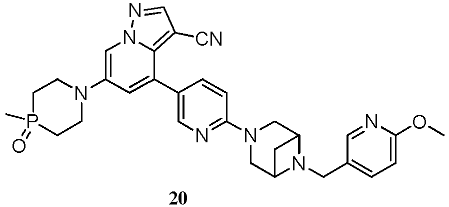

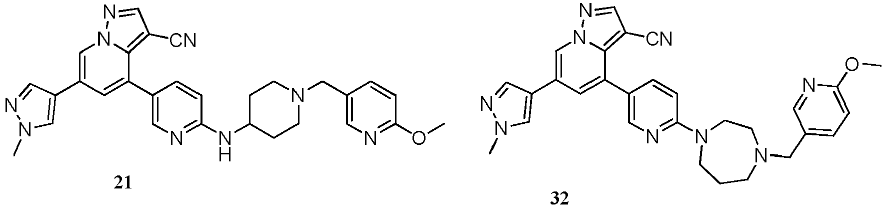

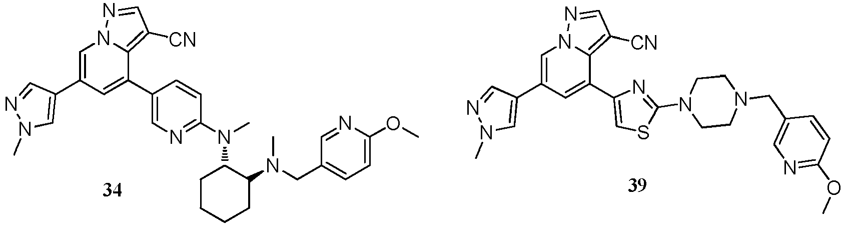

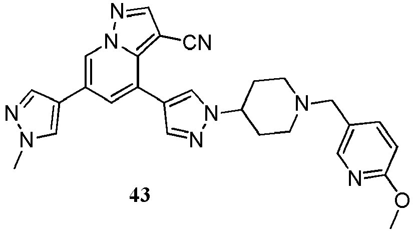

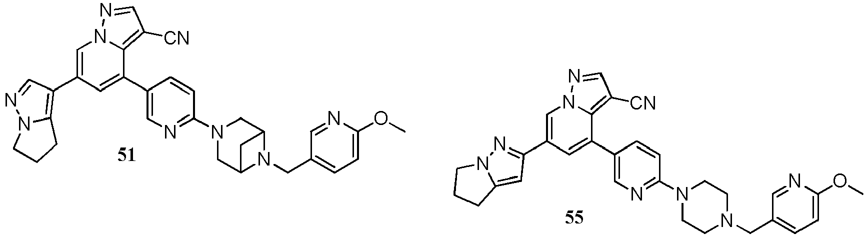

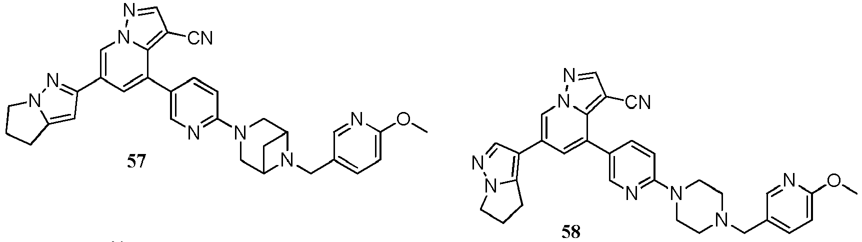

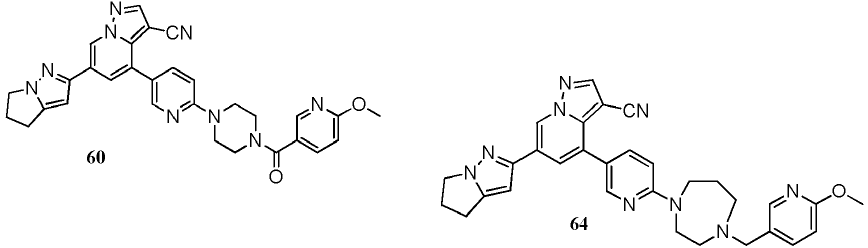

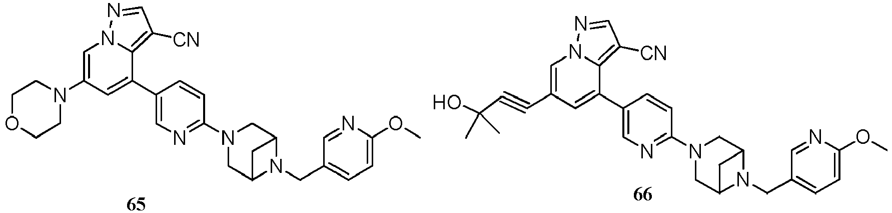

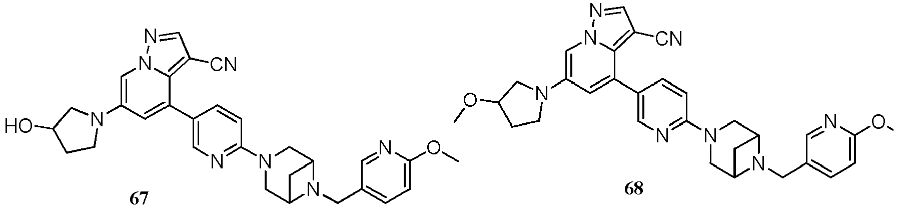

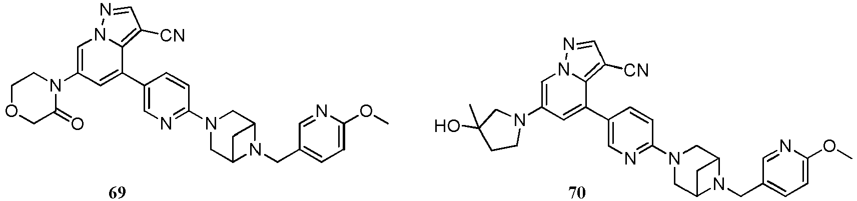

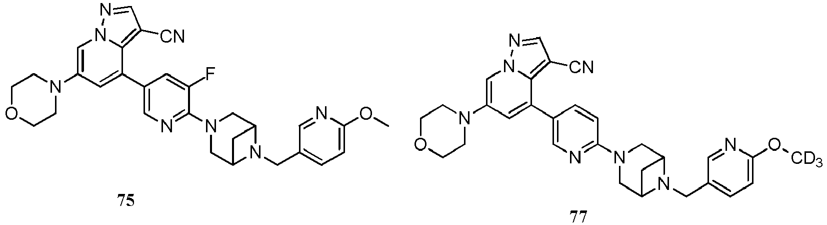

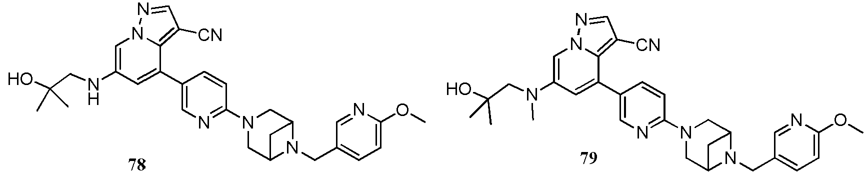

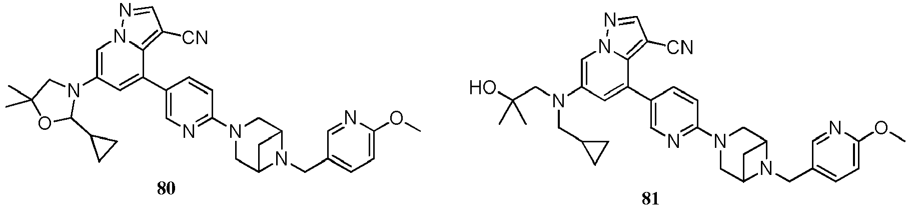

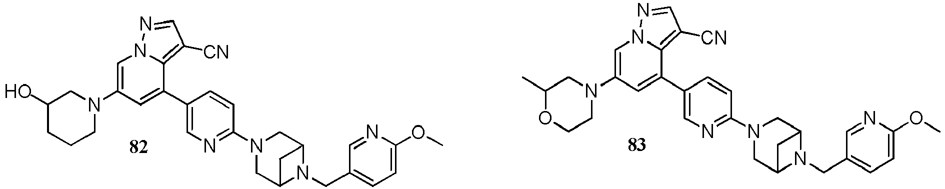

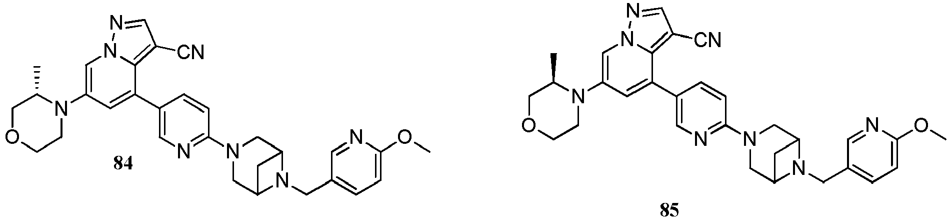

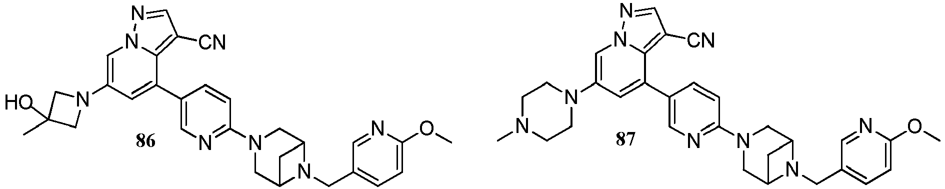

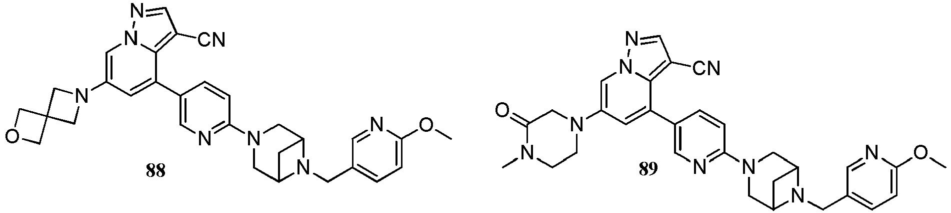

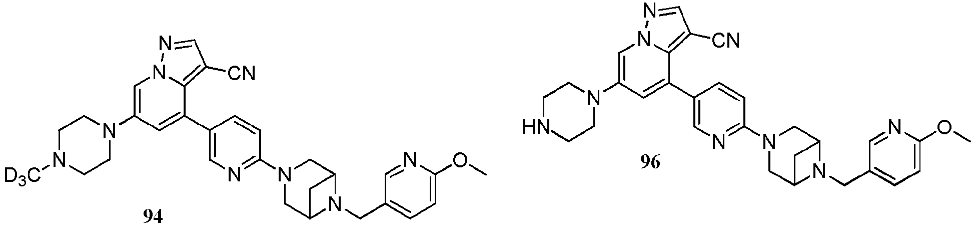

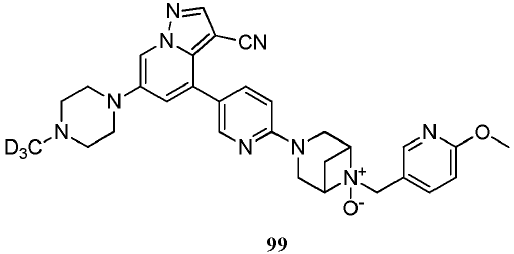

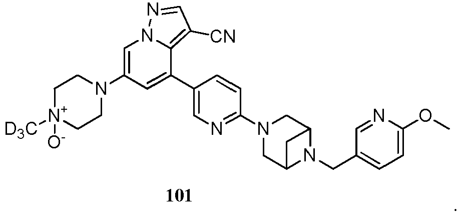

12. A method for preparing a compound of formula (I) or a pharmaceutically acceptable salt, ester, stereoisomer, solvate or prodrug thereof: 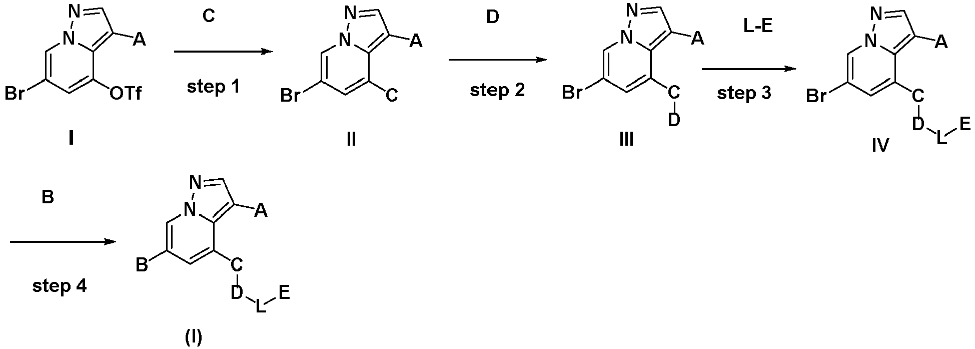 wherein each group is as defined in claim 1.

13. The method according to claim 11, comprising the following steps:
step 1: carrying out a coupling reaction of compound **I** with a boric acid reagent C in solvent dioxane to give **II**;
step 2: carrying out a nucleophilic substitution reaction of intermediate II with an amine compound to give **III**;
step 3: carrying out a reductive amination reaction of or an acylation reaction of intermediate **III** with **L-E** in solvent 1,2-dichloroethane to give intermediate **IV;**
step 4: carrying out a C-N coupling reaction in solvents dioxane and *N*,*N*-dimethylformamide to give the final product (I).

14. A method for preparing a compound of formula (II) or a pharmaceutically acceptable salt, ester, stereoisomer, solvate or prodrug thereof: 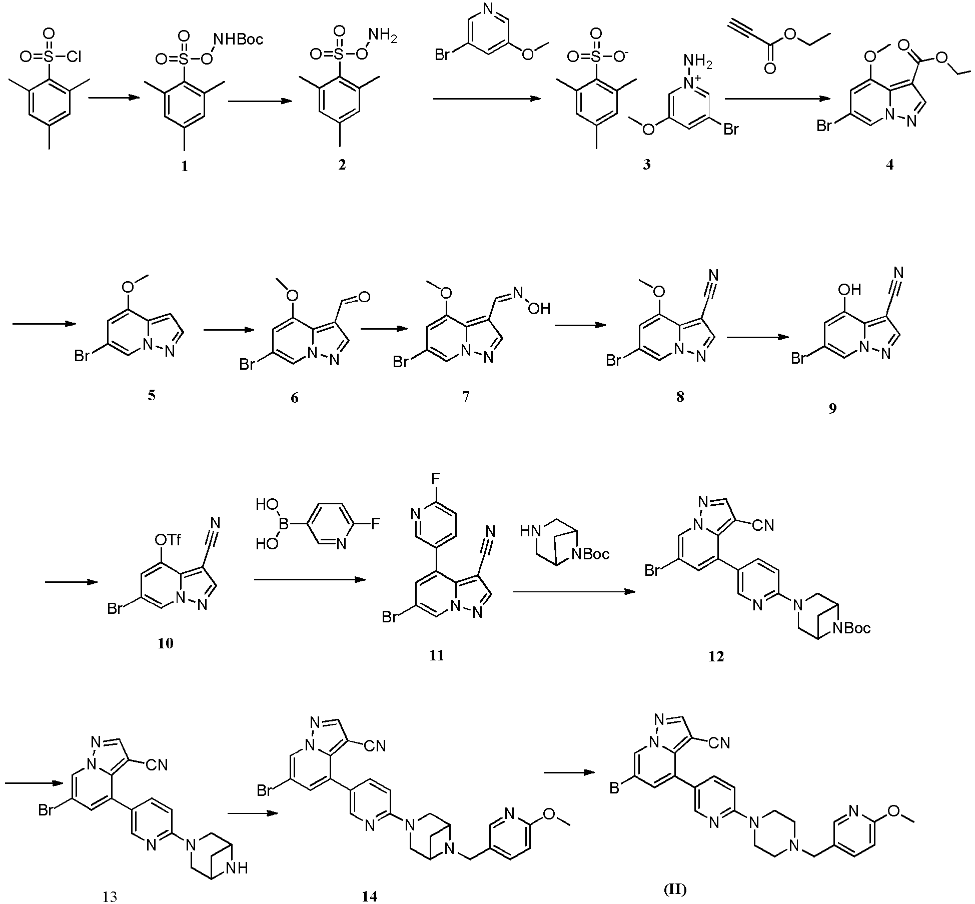 wherein B is as defined in claim 9.

15. A pharmaceutical composition comprising the compound or the pharmaceutically acceptable salt, ester, stereoisomer, solvate or prodrug thereof according to any one of claims 1-11 and a pharmaceutically acceptable carrier.

16. Use of the compound or the pharmaceutically acceptable salt, ester, stereoisomer, solvate or prodrug thereof according to any one of claims 1-11 as a RET kinase inhibitor.

17. Use of the compound or the pharmaceutically acceptable salt, ester, stereoisomer, solvate or prodrug thereof according to any one of claims 1-11 in the manufacture of a medicament for the treatment of a RET-related disease, wherein preferably, the RET-related disease is cancer; more preferably, the cancer is selected from lung cancer, thyroid cancer, breast cancer and colorectal cancer.

18. Use of the compound or the pharmaceutically acceptable salt, ester, stereoisomer, solvate or prodrug thereof according to any one of claims 1-11 in the manufacture of an FGFR family kinase inhibitor medicament, wherein preferably, the FGFR family includes FGFR1, FGFR1 V561M, FGFR2, FGFR2 V564F, FGFR2 N549H, FGFR2 V564I, FGFR2 K641R, FGFR3, FGFR3 V555M, FGFR3 K650E and FGFR4; more preferably, the FGFR family includes FGFR2 V564F, FGFR2 N549H, FGFR2 V564I and FGFR3 V555M.
